(19) Europäisches Patentamt
European Patent Office
Office européen des brevets

(11) EP 2 364 699 A1

(12) **EUROPEAN PATENT APPLICATION**

(43) Date of publication:
**14.09.2011 Bulletin 2011/37**

(21) Application number: **11169343.8**

(22) Date of filing: **13.09.2005**

(51) Int Cl.:
***A61K 31/18*** *(2006.01)* ***A61K 31/381*** *(2006.01)*
***A61K 31/404*** *(2006.01)* ***A61K 31/4439*** *(2006.01)*
***A61K 31/47*** *(2006.01)* ***A61K 31/4709*** *(2006.01)*
***A61K 31/498*** *(2006.01)* ***A61K 31/635*** *(2006.01)*
***A61K 31/64*** *(2006.01)* ***A61K 39/395*** *(2006.01)*
***A61P 9/00*** *(2006.01)* ***A61P 35/00*** *(2006.01)*
***A61K 45/06*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **13.09.2004 US 609452 P**
**28.02.2005 JP 2005054150**
**28.02.2005 JP 2005054475**

(62) Document number(s) of the earlier application(s) in accordance with Art. 76 EPC:
**05785820.1 / 1 797 877**

(71) Applicant: **Eisai R&D Management Co., Ltd.**
**Bunkyo-ku**
**Tokyo 112-8088 (JP)**

(72) Inventors:
- **Owa, Takashi**
  **Ibaraki, 300-2635 (JP)**
- **Ozawa, Yoichi**
  **Ibaraki, 300-2635 (JP)**
- **Semba, Taro**
  **Ibaraki, 300-2635 (JP)**

(74) Representative: **Woods, Geoffrey Corlett**
**J.A. Kemp & Co.**
**14 South Square**
**Gray's Inn**
**London**
**WC1R 5JJ (GB)**

Remarks:
This application was filed on 09-06-2011 as a divisional application to the application mentioned under INID code 62.

(54) **Joint use of sulfonamide based compound with angiogenesis inhibitor**

(57) The present invention relates to a pharmaceutical composition comprising a sulfonamide-including compound in combination with a VEGF receptor kinase inhibitor as an angiogenesis, inhibitor, wherein the sulfonamide-including compound is N-(3-cyano-4-methyl-1 H-indol-7-yl)-3-cyanobenzenesulfonaxnide (E7820), or a pharmacologically acceptable salt thereof or a solvate thereof.

FIG. 8

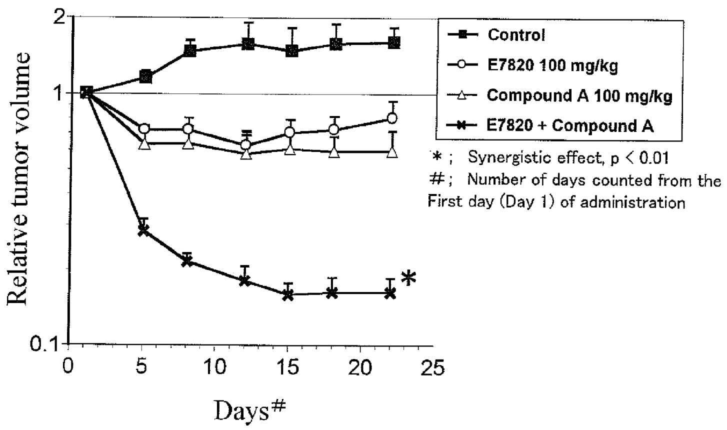


EP 2 364 699 A1

Printed by Jouve, 75001 PARIS (FR)

## Description

FIELD OF THE INVENTION

The present invention relates to novel pharmaceutical compositions and kits, which are characterized by comprising a sulfonamide-including compound in combination with an angiogenesis inhibitor, more specifically with a VEGF inhibitor or a FGF inhibitor.

BACKGROUND ART

Cancer chemotherapeutics conventionally used include alkylating agents such as cyclophosphamide, antimetabolites such as methotrexate and fluorouracil, antibiotics such as adriamycin, mitomycin and bleomycin, plant-derived agents such as taxol, vincristine and etoposide, as well as metal complexes such as cisplatin. However, none of these substances can be regarded as having a sufficient anti-tumor effect; there has been a strong demand for the development of new anti-tumor agents.

In recent years, sulfonamide-including compounds have been reported as useful anti-tumor agents(4-1). Among them, N-(3-chloro-IH-indol-7-yl)-4-sulfamoylbenzenesulfonamide (hereinafter also referred to as E7070), N-(3-cyano-4-methyl-1H-indol-7-yl)-3-cyanobenzenesulfonamide (hereinafter also referred to as E7820), N-[[(4-chlorophenyl)-amino]carbonyl]-2,3-dihydro-1H-indene-5-sulfonamide (hereinafter also referred to as LY186641), N-[[(3,4-dichlorophenyl) amino]carbonyl]-2,3-dihydrobenzofuran-5-sulfonamide (hereinafter also referred to as LY295501), N-(2,4-dichlorobenzoyl)-4-chlorophenyl-sulfonamide (hereinafter also referred to as LY-ASAP), N-(2,4-dichlorobenzoyl)-5-broznothiophene-2-sulfonamide (hereinafter also referred to as LY573636), 2-sulfanylamido-5-chloroquinoxaline (hereinafter also referred to as CQS) and the like have been found to be active against various types of tumors and hence very useful.

Previous studies have reported that the combined use of E7820 which is one of the sulfonamide-including compounds and an angiogenesis inhibitor produces excellent angiogenesis inhibitory activity and anti-tumor activity[(5)].

In recent years, methods have been established for simultaneously detecting the expression levels of many genes using various DNA microarrays, which are applied to a wide range of purposes[(6-7)] Likewise, several reports have been made on studies in which DNA microarrays (In part, there is macroarray which uses membrane filters) are used to detect changes in gene expression caused when tumor cells are treated with anti-cancer agents[(8-10)]. These reports indicate that the analysis of gene expression changes is very useful in comprehensively studying, at the molecular level, property comparison of a plurality of cell populations and biological changes in cells caused by treatment with drugs, etc.

In addition, there are other reports in which a panel of 60 cancer cell lines from the US National Cancer Institute is reclassified and examined for their properties based on the analysis of their gene expression profiled [(11)] and in which the panel of 60 cancer cell lines is further studied for the relationship between their gene expression profiles and the susceptibility of each cell line to various anti-cancer agents[(12)].

References

(1) JP 7-165708 A
(2) International Publication No. WO00/50395
(3) European Patent Publication No. 0222475
(4) International Publication No. WO02/098848
(5) International Publication No, WO03/074045
(6) Schena M, Shalon D, Davis RW, Brown PO. Science, 1995, 270, 467-70.
(7) Lockhart, D.J., Dong, H., Byrne, M.C., Follettie, M.T, Gallo, M.V, Chee, M.S., Mittmann, M., Wang C., Kobayashi, M., Horton, H. Brown, E.L., Nature Biotechnology, 1996, 14, 1675-1680.
(8) Rhee CH, Ruan S, Chen S, Chenchik A, Levin VA, Yung AW, Fuller GN, Zhang W, Oncol Rep, 1999,6,393-401.
(9) Zimmermann J, Erdmann D, Lalande I, Grossenbacher R, Noorani M, Furst P, Oncogene, 2000, 19,2913-20.
(10) Kudoh K, Ramanna M, Ravatn R, Elkahloun AG, Bittner ML, Meltzer PS, Trent JM, Dalton WS, Chin KV, Cancer Res, 2000, 4161-6.
(11) Ross DT, Scherf U, Eisen MB, Perou CM, Rees C, Spellman P, Iyer V, Jeffrey SS, Van de Rijn M, Waltham M, Pergamenschikov A, Lee JC, Lashkari D, Shalon D, Myers TG, Weinstein JN, Botstein D, Brown PO, Nat Genet, 2000, 24, 227-3 5.
(12) Scherf U, Ross DT, Waltham M, Smith LH, Lee JK, Tanabe L, Kohn KW, Reinhold WC, Myers TG, Andrews DT, Scudiero DA, Eisen MB, Sausville EA, Pommier Y, Botstein D, Brown PO, Weinstein JN, Nat Genet, 2000, 24, 236-44.

SUMMARY OF THE INVENTION

The present invention was made under such circumstances, and the problem to be solved by the invention is to find out pharmaceutical compositions and kits having excellent angiogenesis inhibitory activity and/or anti-tumor activity.

As a result of extensive and intensive efforts made to solve the problem stated above, the inventors of the present invention have found that there is a high correlation between the pattern of gene expression changes and cell growth inhibitory activity provided by E7820, E7070, LY186641, LY295501, LY573636 or CQS or combinations thereof in experiments using DNA microarrays and cancer cell line panels. Likewise, in cell growth inhibition assays, the inventors have also found that cancer cell lines resistant to E7070 have cross-resistance to E7820, LY186641, LY295501, LY-ASAP, LY573636 and CQS. Based on these results, the inventors of the present invention have obtained a finding that E7070, E7820, LY186641, LY295501, LY-ASAP, LY573636 or CQS or combinations thereof have the same or similar mechanism of action and produce the same or similar genetic changes and effects.

On the other hand, E7820 has been reported to show excellent angiogenesis inhibitory activity and anti-tumor activity when used in combination with an angiogenesis inhibitor (WO03/074045). Thus, based on the above finding, E7070, LY186641, LY295501, LY-ASAP, LY573636 or CQS or combinations thereof are also believed to show excellent angiogenesis inhibitory activity and anti-tumor activity when used in combination with an angiogenesis inhibitor; sulfonamide-including compounds, preferably E7070, LY186641, LY295501, LYASAP, LY573636 or CQS or combinations thereof, have now been found to provide useful pharmaceutical compositions and kits when combined with angiogenesis inhibitors.

Namely, the present invention is directed to the following.

(1) A pharmaceutical composition comprising a sulfonamide-including compound in combination with an angiogenesis inhibitor.

(2) A kit comprising:

(a) at least one selected from the group consisting of a packaging container, an instruction manual and an package insert, each of which describes the combined use of a sulfonamide-including compound and an angiogenesis inhibitor; and

(b) a pharmaceutical composition comprising the sulfonamide-including compound.

(3) A kit comprising a set of a formulation comprising a sulfonamide-including compound and a formulation comprising an angiogenesis inhibitor.

(4) A method for preventing or treating cancer and/or a method for inhibiting angiogenesis, which comprises administering a sulfonamide-including compound and an angiogenesis inhibitor to a patient.

(5) Use of a sulfonamide-including compound for the manufacture of a pharmaceutical composition in combination with an angiogenesis inhibitor.

(6) A pharmaceutical composition comprising a sulfonamide-including compound, which is administered together with an angiogenesis inhibitor to a patient.

The above sulfonamide-including compound includes at least one compound selected from the group consisting of:

a compound of Formula (I):

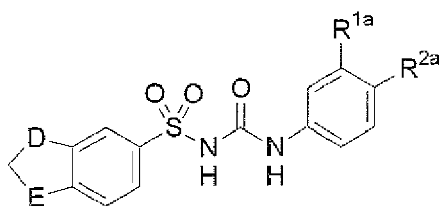

(I)

[wherein E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom, and $R^{2a}$ represents a halogen atom or a trifluoromethyl group],

a compound of Formula (II):

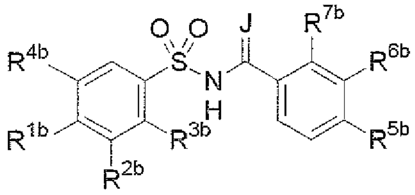

(II)

[wherein J represents -O- or -NH-, $R^{1b}$ represents a hydrogen atom, a halogen atom, an optionally substituted $C_1$-$_6$ alkyl group, an optionally substituted $C_{1-4}$ alkoxy group, an optionally substituted $C_1$-$_4$ alkylthio group, an optionally substituted $C_2$-$_5$ alkoxycarbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, an optionally substituted $C_{1-6}$ alkyl group, an optionally substituted $C_2$-$_5$ alkoxycarbonyl group, an optionally substituted $C_{1-4}$ alkoxy group, an optionally substituted phenyl group or an optionally substituted quinolinyl group, $R^{3b}$ represents a hydrogen atom or an optionally substituted $C_{1-4}$ alkoxy group, $R^{4b}$ represents a hydrogen atom or an optionally substituted $C_1$-$_6$ alkyl group (provided that at least one of $R^{3b}$ and $R^{4b}$ is a hydrogen atom), $R^{5b}$ represents a hydrogen atom, a halogen atom, an optionally substituted $C_{1-6}$ alkyl group or a nitro group, $R^{6b}$ represents a hydrogen atom, a halogen atom or an optionally substituted $C_{1-6}$ alkyl group (provided that when $R^{6b}$ is an optionally substituted $C_{1-6}$ alkyl group, $R^{5b}$ is a hydrogen atom and $R^{7b}$ is a halogen atom), and $R^{7b}$ represents a halogen atom or an optionally substituted $C_1$-$_6$ alkyl group (provided that when either $R^{5b}$ or $R^{7b}$ is an optionally substituted $C_{1-6}$ alkyl group or when $R^{7b}$ is a halogen atom or an optionally substituted $C_{1-6}$ alkyl group, either $R^{5b}$ or $R^{6b}$ is a hydrogen atom)],
a compound of Formula (III):

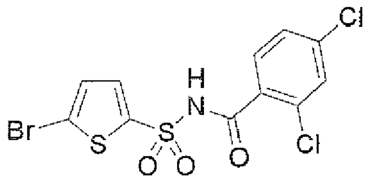

(III)

and
a compound of Formula (IV):

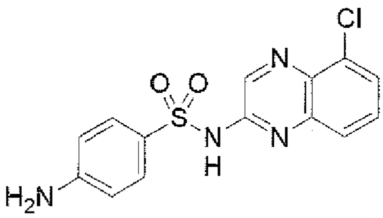

(IV)

or a pharmacologically acceptable salt thereof or a solvate thereof

Alternatively, the above sulfonamide-including compound may be a compound of Formula (IX):

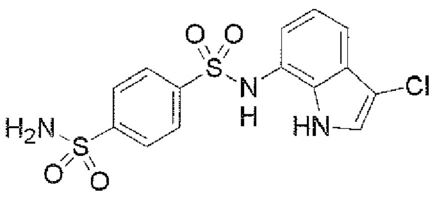

(IX)

or a pharmacologically acceptable salt thereof or a solvate thereof.

The present invention is also directed to the following.

(7) A pharmaceutical composition comprising a sulfonamide-including compound in combination with a VEGF receptor kinase inhibitor

(8) A kit comprising:

(a) at least one selected from the group consisting of a packaging container, an instruction manual and an package insert, each of which describes the combined use of a sulfonamide-including compound and a VEGF receptor kinase inhibitor; and

(b) a pharmaceutical composition comprising the sulfonamide-including compound.

(9) A kit comprising a set of a formulation comprising a sulfonamide-including compound and a formulation comprising a VEGF receptor kinase inhibitor.

(10) A method for preventing or treating cancer and/or a method for inhibiting angiogenesis, which comprises administering a sulfonamide-including compound and a VEGF receptor kinase inhibitor to a patient.

(11) Use of a sulfonamide-including compound for the manufacture of a pharmaceutical composition in combination with a VEGF receptor kinase inhibitor.

(12) A pharmaceutical composition comprising a sulfonamide-including compound, which is administered together with a VEGF receptor kinase inhibitor.

The above sulfonamide-including compound includes a compound of Formula (XIV):

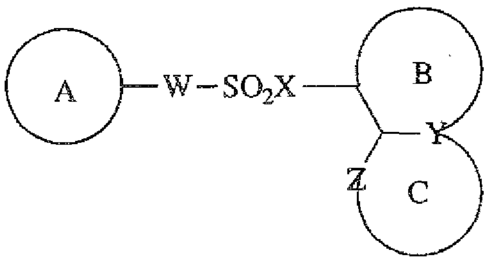

(XIV)

[wherein
the ring A represents an optionally substituted monocyclic or bicyclic aromatic ring,
the ring B represents an optionally substituted 6-membered cyclic unsaturated hydrocarbon or an optionally substituted unsaturated 6-membered heterocyclic ring containing one nitrogen atom as a heteroatom,
the ring C represents an optionally substituted 5-membered heterocyclic ring containing one or two nitrogen atoms,
W represents a single bond or -CH=CH-,
X represents $-N(R^1)-$ or an oxygen atom,
Y represents:

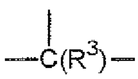 or 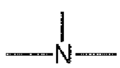

and
Z represents $-N(R^2)-$, wherein $R^1$, $R^2$ and $R^3$, which may be the same or different, each independently represent a hydrogen atom or a lower alkyl group] or a pharmacologically acceptable salt thereof or a solvate thereof

The above VEGF receptor kinase inhibitor may include at least one compound selected from the group consisting of

(10) N-{2-chloro-4-[(6,7-dimethoxy-4-quinolyl)oxy]phenyl} -N'-(5-methyl-3-isoxazolyl)urea,

(11) 4-[(4-fluoro-2-methylindol-5-yl)oxy]-6-methoxy-7-[3-(pyrrolidin-1-yl)propoxy]quinazoline,

(12) 6-[2-(methylcarbamoyl)phenylsulfanyl]-3-E-[2-(pyridin-2-yl)ethenyl]indazole,
(13) 5-((Z)-(5-fluoro-2-oxo-1,2-dihydro-3H-indol-3-ylidene)methyl)-N-((2S)-2-hydroxy-3-morpholin-4-ylpropyl)-2,4-dimethyl-1H-pyrrole-3-carboxarnide,
(14) 3-((quinolin-4-ylmethyl)amino)-N-(4-(trifluoromethoxy)phenyl)thiophene-2-carboxamide,
(15) 6-(2,6-dichlorophenyl)-8-methyl-2-phenylamino-8H-pyrido[2,3-d]pyrimidin-7 -one,
(16) 2-((1,6-dihydro-6-oxo-pyridin-3-ylmethyl)amino)-N-(3-(trifluoromethyl)phenyl)-3-pyridine-carboxamide,
(17) 4-(4-(4-chloro-phenylamino)-furo[2,3-d]pyridazin-7-yloxymethyl)-pyridine-2-carboxylic acid methylamide,
(18) N-(3-trifluoromethyl-4-chlorophenyl)-N'-(4-(2-methylcarbamoylpyridin-4-yl_)oxyphenyl)urea,
(19) 4-amino-5-fluoro-3-(6-(4-methyl-piperazin-1-yl)-1H-benzimidazol-2-yl)-1H-quinolin-2-one,
(20) 4-(4-(1-amino-1-methyl-ethyl)-phenyl)-2-( 4-(2-morpholin-4-yl-ethyl)-phenylamino)-pyrimidine-5-carbonitrile,
(21) [6-[4-[(4-ethylpiperazin-1-yl)methyl]phenyl]-7H-pyrrolo[2,3-d]pyrimidin-4-yl]-((R)-1-phenylethyl)amine,
(22) 9-(1-methylethoxy)methyl-12-(3-hydroxypropyl)-6H,7H, 13H-indeno[2, 1-a]pyrrolo[3,4-c]carbazol-5-one,
(23) N-(2,4-diftuorophenyl)-N'-{4-[(6,7-dimethoxy-4-quimolyl)-oxy]-2-fluorophenyl}urea,
(24) 5-[N-methyl-N-(4-octadecyloxyphenyl)acetyl]amino-2-methylthiobenzoic acid,
(25) N-[4-(3-amino-1H-indazol-4-yl)phenyl]-N'-(2-fluoro-5-methylphenyl)urea,
(26) 2-methyl-6-[2-(1-methyl-1H-imidazol-2-yl)-thieno[3,2-b]pyridin-7-yloxy]-benzo[b]thiophene-3-carboxylic acid methylamide,
(27) (R)-1-(4-(4-fluoro-2-methyl-1H-indol-5-yloxy)-5-methylpyrrolo[1,2-f][1,2,4]triazin-6-yloxy)propan-2-ol, and
(28) (S)-((R)-1-(4-(4-fluoro-2-methyl-1H-indol-5-yloxy)-5-methylpyrrolo[1,2-f] [1,2,4]triazin-6-yloxy)propan-2-ol) 2-aminopropanonate

or a pharmacologically acceptable salt thereof or a solvate thereof.

Alternatively, the above VEGF receptor kinase inhibitor may be 4-(3-chloro-4-(cyclopropylaminocarbonyl)aminophenoxy)-7 -methoxy-6-quinolinecarboxamide, or a pharmacologically acceptable salt thereof or a solvate thereof

The present invention provides pharmaceutical compositions and kits showing excellent angiogenesis inhibitory activity and/or anti-tumor activity.

More specifically, as a result of combining sulfonamide-including compounds, preferably E7070, LY186641, LY295501, LYASAP, LY573636 or CQS or combinations thereof, with angiogenesis inhibitors, the present invention provides pharmaceutical compositions and kits showing excellent angiogenesis inhibitory activity and/or anti-tumor activity and enables them to be used for cancer treatment or angiogenesis inhibition. Likewise, when combining sulfonamide-including compounds, preferably E7820, with VEGF receptor kinase inhibitors, the present invention provides pharmaceutical compositions and kits showing excellent angiogenesis inhibitory activity and/or anti-tumor activity and enables them to be used for cancer treatment or angiogenesis inhibition.

BRIEF DESCRIPTION OF THE DRAWING

Figure 1 shows the results of hierarchical clustering analysis in DNA microarrays in Example I.
Figure 2 shows correlation coefficients in DNA microarrays in Example 2.
Figure 3 shows the results of hierarchical clustering analysis in DNA microarrays in Example 2.
Figure 4 shows correlation coefficients in DNA microarrays in Example 2.
Figure 5 shows the results of hierarchical clustering analysis in DNA microarrays in Example 2.
Figure 6 shows the antiproliferative effect of E7070, E7820, CQS, LY186641, LY295501 and LY-ASAP on HCT116-C9, HCTI 16-C9-C and HCT116-C9-C4, as measured by cell growth inhibition assay.
Figure 7 shows the antiproliferative effect of E7070 and LY573636 on HCT116-C9, HCT116-C9-C1 and HCT116-C9-C4, as measured by cell growth inhibition assay.
Figure 8 shows the combined effect of E7820 and 4-(3-chloro-4-(cyclopropylaminocarbonyl)aminophenoxy)-7-methoxy-6-quinolinccarboxamide on tumor growth in a subcutaneous transplantation model *(in vivo)* of human renal cancer cell line 786-O. In Figure 8, Compound A denotes 4-(3-chloro-4-(cyclopropylaminocarbonyl)aminophenoxy)-7-methoxy-6-quinolinecarboxarnide.

DETAILED DESCRIPTION OF THE INVENTION

Embodiments of the present invention will be explained below. The following embodiments are provided for illustrative purposes only, and are not intended to limit the scope of the invention. The present invention can be embodied in various forms without departing from the spirit of the invention.

All documents cited herein, including journal articles, patent gazettes and other patent documents, are incorporated herein by reference.

As used herein, the term "halogen atom" refers to a fluorine atom, a chlorine atom, a bromine atom or an iodine atom.

As used herein, the term "$C_{l-6}$ alkyl group" refers to a linear or branched alkyl group containing 1 to 6 carbon atoms. Specific examples include a methyl group, an ethyl group, a 1-propyl group (i.e., a n-propyl group), a 2-propyl group (i.e., an i(iso)-propyl group or an isopropyl group), a 2-methyl-1-propyl group (i.e., an i-butyl group or an isobutyl group), a 2-methyl-2-propyl group (i.e., a t(tert)-butyl group), a 1-butyl group (i.e., a n-butyl group), a 2-butyl group (i.e., a s(sec)-butyl group), a 1-pentyl group (i.e., a n-pentyl group or an amyl group), a 2-pentyl group (i.e., a 1-methylbutyl group), a 3-pentyl group (i.e., a 1-ethylpropyl group), a 2-methyl-1-butyl group (i.e., a 2-methylbutyl group), a 3 -methyl-l -butyl group (i.e., an isopentyl group), a 2-methyl-2-butyl group (a.e., a t(tert)-pentyl group), a 3-methyl-2-butyl group (i.e., a 1,2-dimethylpropyl group), a 2,2-dimethyl-1-propyl group (i.e., a neopentyl group), a 1-hexyl group (i.e., a n-hexyl group), a 2-hexyl group (i.e., a 1-methylpentyl group), a 3-hexyl group (i.e., a 1-ethylbutyl group), a 2-methyl-1-pentyl group (i.e., a 2-methylpentyl group), a 3-methyl-1-pentyl group (i.e., a 3-methylpentyl group), a 4-methyl-l-pentyl group (i.e., an isohexyl group), a 2-methyl-2-pentyl group (i.e., a 1,1-dimethylbutyl group), a 3-methyl-2-pentyl group (i.e., a 1,2-dimethylbutyl group), a 4-methyl-2-pentyl group (i.e., a 1,3-dimethylbutyl group), a 2-methyl-3-pentyl group (i.e., a 1-ethyl-2-methylpropyl group), a 3-methyl-3-pentyl group (i.e., a 1-ethyl-1-methylpropyl group), a 2,3-dimethyl-1-butyl group (i.e., a 2,3-dimethylbutyl group), a 3,3-dimethyl-l-butyt group (i.e., a 3,3-dimethylbutyl group), a 2,2-dimethyl-1-butyl group (i.e., a 2,2-dimethylbutyl group), a 2-ethyl-l-butyl group (i.e., a 2-ethylbutyl group), a 3,3-dimethyl-2-butyl group (i.e., a 1,2,2-trimethylpropyl group), and a 2,3-dimethyl-2-butyl group (i.e., a 1,1,2-trimethylpropyl group).

Preferred examples of the "$C_{1-6}$ alkyl group" may include a methyl group, an ethyl group, a 1-propyl group, a 2-propyl group, a 2-methyl-1-propyl group, a 2-methyl-2-propyl group, a 1-butyl group, and a 2-butyl group.

As used herein, the term "$C_{l-6}$ alkylene group" refers to a divalent group derived by removing any one hydrogen atom from the "$C_{1-6}$ alkyl group" defined above. Specific examples include a methylene group, a 1,2-ethylene group, a 1,1-ethylene group, a 1,3-propylene group, a tetramethylene group, a pentamethylene group, and a hexamethylene group.

As used herein, the term "$C_{2-6}$ alkenyl group" refers to a linear or branched alkenyl group having one double bond and containing 2 to 6 carbon atoms. Specific examples include an ethenyl group (i.e., a vinyl group), a 1-propenyl group, a 2-propenyl group (i.e., an allyl group), a 1-butenyl group, a 2-butenyl group, a 3-butenyl group, a pentenyl group, and a hexenyl group.

As used herein, the term "$C_{2-6}$ alkynyl group" refers to a linear or branched alkynyl group having one triple bond and containing 2 to 6 carbon atoms. Specific examples include an ethynyl group, a 1-propynyl group, a 2-propynyl group, a 1-butynyl group, a 2-butynyl group, a 3-butynyl group, a pentynyl group, and a hexynyl group.

As used herein, the term "$C_{3-8}$ cycloalkyl group" refers to a monocyclic or bicyclic saturated aliphatic hydrocarbon group containing 3 to 8 carbon atoms. Specific examples include a cyclopropyl group, a cyclobutyl group, a cyclopentyl group, a cyclohexyl group, a cycloheptyl group, a cyclooctyl group, a bicyclo[2.1.0]pentyl group, a bicyclo[3.1.0]hexyl group, a bicyclo[2.1.l]hexyl group, a bicyclo[4.1.0]heptyl group, a bicyclo[2.2.1]heptyl group (i.e., a norbornyl group), a bicyclo[3.3.0]octyt group, a bicyclo[3.2.1]octyl group, and abicyclo[2.2.2]octyl group.

Preferred examples of the "$C_{3-8}$ cycloalkyl group" may include a cyclopropyl group, a cyclobutyl group, and a cyclopentyl group.

As used herein, the term "$C_{6-10}$ aryl group" refers to an aromatic cyclic hydrocarbon group containing 6 to 10 carbon atoms. Specific examples include a phenyl group, a 1-naphthyl group, a 2-naphthyl group, an indenyl group, and an azulenyl group.

Preferred examples of the "$C_{6-10}$ aryl group" may include a phenyl group.

As used herein, the term "heteroatom" refers to a nitrogen atom, an oxygen atom or a sulfur atom.

As used herein, the term "5- to 10-membered heteroaryl group" refers to an aromatic cyclic group having 5 to 10 ring member atoms, 1 to 5 of which are heteroatoms. Specific examples include a furyl group, a thienyl group, a pyrrolyl group, an imidazolyl group, a triazolyl group, a tetrazolyl group, a thiazolyl group, a pyrazolyl group, an oxazolyl group, an isoxazolyl group, an isothiazolyl group, a furazanyl group, a thiadiazolyl group, an oxadiazolyl group, a pyridyl group, a pyrazinyl group, a pyridazinyl group, a pyrimidinyl group, a triazinyl group, a purinyl group, a pteridinyl group, a quinolyl group, an isoquinolyl group, a naphthylizinyl group, a quinoxalinyl group, a cinnolinyl group, a quinazolinyl group, a phthalazinyl group, an imidazopyridyl group, an imidazothiazolyl group, an imidazooxazolyl group, a benzothiazolyl group, a benzoxazolyl group, a benzimidazolyl group, an indolyl group, an isoindolyl group, an indazolyl group, a pyrrolopyridyl group, a thienopyridyl group, a furopyridyl group, a benzothiadiazolyl group, a benzooxadiazolyl group, a pyridopyrimidinyl group, a benzofuryl group, a benzothienyl group, and a thienofuryl group.

Preferred examples of the "5- to 10-membered heteroaryl! group" may include a furyl group, a thienyl group, a pyrrolyl group, an imidazolyl group, a thiazolyl group, a pyrazolyl group, an oxazolyl group, an isoxazolyl group, an isothiazolyl group, a pyridyl group, and a pyrimidinyl group.

As used herein, the term "3- to 10-membered non-aromatic heterocyclic group" refers to a non-aromatic cyclic

group characterized by:

(1) having 3 to 10 ring member atoms;
(2) containing 1 or 2 heteroatoms as the ring member atoms;
(3) optionally containing 1 or 2 double bonds in the ring;
(4) optionally containing 1 to 3 carbonyl groups, sulfinyl groups or sulfonyl groups in the ring; and
(5) being monocyclic or bicyclic. In the case of containing a nitrogen atom as a ring member, the nitrogen atom may have a binding hand. Specific examples include an aziridinyl group, an azetidinyl group, a pyrrolidinyl group, a piperidinyl group, an azepanyl group, an azocanyl group, a piperazinyl group, a diazepanyl group, a diazocanyl group, a diazabicylo[2.2.1]heptyl group, a morpholinyl group, a thiomorpholinyl group, a 1,1-dioxothiomorpholinyl group, an oxilanyl group, an oxetanyl group, a tetrahydrofuryl group, a dioxolanyl group, a tetrahydropyranyl group, a dioxanyl group, a tetrahydrothienyl group, a tetrahydrothiopyranyl group, an oxazolidinyl group, and a thiazolidinyl group.

Preferred examples of the "3- to 10-membered non-aromatic heterocyclic group" may include an aziridinyl group, an azetidinyl group, a pyrrolidinyl group, a piperidinyl group, an azepanyl group, a piperazinyl group, a diazepanyl group, a morpholinyl group, a thiomorpholinyl group, a 1,1-dioxothiomorpholinyl group, a tetrahydrofuryl group, and a tetrahydropyranyl group.

As used herein, the term "$C_{l-6}$ alkoxy group" refers to a group having an oxygen atom attached to the end of the "$C_{1-6}$ alkyl group" defined above. Specific examples include a methoxy group, an ethoxy group, a 1-propoxy group (i.e., a n-propoxy group), a 2-propoxy group (i.e., an i-propoxy group), a 2-methyl-1-propoxy group (i.e., an i-butoxy group), a 2-methyl-2-propoxy group (i.e., a t-butoxy group), a 1-butoxy group (i.e., a n-butoxy group), a 2-butoxy group (i.e., a s-butoxy group), a l-pentyloxy group, a 2-pentyloxy group, a 3-pentyloxy group, a 2-methyl-l-butoxy group, a 3-methyl-l-butoxy group, a 2-methyl-2-butoxy group, a 3-methyl-2-butoxy group, a 2,2-dimethyl-1-propoxy group, a 1-hexyloxy group, a 2-hexyloxy group, a 3-hexyloxy group, a 2-methyl-1-pentyloxy group, a 3-methyl-l-pentyloxy group, a 4-methyl-1-pentyloxy group, a 2-methyl-2-pentyloxy group, a 3-methyl-2-pentyloxy group, a 4-methyl-2-pentyloxy group, a 2-methyl-3-pentyloxy group, a 3-methyl-3-pentyloxy group, a 2,3-dimethyl-1-butoxy group, a 3,3-dimethyl-1-butoxy group, a 2,2-dimethyl-1-butoxy group, a 2-ethyl-1-butoxy group, a 3,3-dimethyl-2-butoxy group, and a 2,3-dimethyl-2-butoxy group.

Preferred examples of the "$C_{1-6}$ alkoxy group" may include "$C_{1-4}$ alkoxy groups," as exemplified by a methoxy group, an ethoxy group, a 1-propoxy group, a 2-propoxy group, a 2-methyl-1-propoxy group, a 2-methyl-2-propoxy group, a 1-butoxy group, and a 2-butoxy group.

As used herein, the term "$C_{1-6}$ alkylthio group" refers to a group having a sulfur atom attached to the end of the "$C_{1-6}$ alkyl group" defined above. Specific examples include a methylthio group, an ethylthio group, a 1-propylthio group (i.e., a n-propylthio group), a 2-propylthio group (i.e., an i-propylthio group or an isopropylthio group), a 2-methyl-1-propylthio group (i.e., an i-butylthio group or an isobutylthio group), a 2-methyl-2-propylthio group (i.e., a t(tert)-butylthio group), a 1-butylthio group (i.e., a n-butylthio group), a 2-butylthio group (i.e., a s(sec)-butylthio group), a l-pentylthio group, a 2-pentylthio group, a 3-pentylthio group, a 2-methyl-1-butylthio group, a 3-methyl-1-butylthio group, a 2-methyl-2-butylthio group, a 3-methyl-2-butylthio group, a 2,2-dimethyl-1-propylthio group, a 1-hexylthio group, a 2-hexylthio group, a 3-hexylthio group, a 2-methyl-1-pentylthio group, a 3-methyl-I-pentylthio group, a 4-methyl-1-pentylthio group, a 2-methyl-2-pentylthio group, a 3-methyl-2-pentylthio group, a 4-methyl-2-pentylthio group, a 2-methyl-3-pentylthio group, a 3-methyl-3-pentylthio group, a 2,3-dimethyl-l-butylthio group, a 3,3-dimethyl-l-butylthio group, a 2,2-dimethyl-1-butylthio group, a 2-ethyl-1-butylthio group, a 3,3-dimethyl-2-butylthio group, and a 2,3-dimethyl-2-butylthio group.

Preferred examples of the "$C_{1-6}$ alkylthio group" may include "$C_{1-4}$ alkylthio groups," as exemplified by a methylthio group, an ethylthio group, a 1-propylthio group, a 2-propylthio group, a 2-methyl-1-propylthio group, a 2-methyl-2-propylthio group, a 1-butylthio group, and a 2-butylthio group.

As used herein, the term "$C_{3-R}$ cycloalkoxy group" refers to a group having an oxygen atom attached to the end of the "$C_{3-9}$ cycloalkyl group" defined above. Specific examples include a cyclopropoxy group, a cyclobutoxy group, a cyclopentyloxy group, a cyclohexyloxy group, a cycloheptyloxy group, a cyclooctyloxy group, a bicyclo[2.1.0]pentyloxy group, a bicyclo[3.1.0]hexyloxy group, a bicyclo[2.1.1]hexyloxy group, a bicyclo[4.1.0]heptyioxy group, a bicyclo[2.2.1]heptyloxy group (i.e., a norbornyloxy group), a bicyclo[3.3.0]octyloxy group, a bicyclo[3.2.1]octyloxy group, and a bicyclo[2.2.2]octyloxy group.

Preferred examples of the "$C_{3-8}$ cycloalkoxy group" may include a cyclopropoxy group, a cyclobutoxy group, and a cyclopentyloxy group.

As used herein, the term "mono-$C_{1-6}$ alkylamino group" refers to an amino group whose one hydrogen atom is replaced by the "$C_{1-6}$ alkyl group" defined above. Specific examples include a methylamino group, an ethyl amino group, a l-propylamino group (i.e., a n-propylamino group), a 2-propylamino group (i.e., an i-propylamino group), a 2-methyl-i-propylamino group (i.e., an i-butylamino group), a 2-methyl-2-propylamino group (i.e., a t-butylamino group), a

1-butylamino group (i.e., a n-butylamino group), a 2-butylamino group (i.e., a s-butylamino group), a l-pentylamino group, a 2-pentylamino group, a 3-pentylamino group, a 2-methyl-1-butylamino group, a 3-methyl-1-butylamino group, a 2-methyl-2-butylamino group, a 3-methyl-2-butylamino group, a 2,2-dimethyl-1-propylamino group, a 1-hexylamino group, a 2-hexylamino group, a 3-hexylamino group, a 2-methyl-1-pentylamino group, a 3-methyl-1-pentylamino group, a 4-methyl-l-pentylamino group, a 2-methyl-2-pentylamino group, a 3-methyl-2-pentylamino group, a 4-methyl-2-pentylamino group, a 2-methyl-3-pentylamina group, a 3-methyl-3-pentylamino group, a 2,3-dimethyl-1-butylamino group, a 3,3-dimethyl-1-butylamino group, a 2,2-dimethyl-l-butylamino group, a 2-ethyl-1-butylamino group, a 3,3-dimethyl-2-butylamino group, and a 2,3-dimethyl-2-butylamino group.

As used herein, the term "di-$C_{1-6}$ alkylamino group" refers to an amino group whose two hydrogen atoms are replaced by the same or different "$C_{l-6}$ alkyl groups" defined above. Specific examples include an N,N-dimethylamino group, an N,N-diethylamino group, an N,N-di-n-propylamino group, an N,N-di-i-propylamino group, an N,N-di-n-butylamino group, an N,N-di-I-butylamino group, an N,N-di-s-butylamino group, an N,N-di-t-butylamino group, an N-ethyl-N-methylamino group, an N-n-propyl-N-methylamino group, an N-i-propyl-N-methylamino group, an N-n-butyl-N-methylamino group, an N-i-butyi-N-methylamino group, an N-s-butyl-N-methylamino group, and an N-t-butyl-N-inethylamino group.

As used herein, the term "$C_{2-7}$ acyl group" refers to a carbonyl group, to which the "$C_{i-6}$ alkyl group" defined above is attached. Specific examples include, for example, an acetyl group, a propionyl group, an isopropionyl group, a butyryl group, an isobutyryl group, a valeryl group, an isovaleryl group, and a pivaloyl group.

As used herein, the term "$C_{2-7}$ alkoxycarbonyl group" refers to a carbonyl group, to which the "$C_{l-6}$ alkoxy group" defined above is attached. Specific examples include, for example, a methoxycarbonyl group, an ethoxycarbonyl group, a 1-propyloxycarbonyl group, a 2-propyloxycarbonyl group, and a 2-methyl-2-propoxy group (i.e., a t-butoxycarbonyl group).

As used herein, the term "$C_{2-5}$ alkoxycarbonyl group" refers to a carbonyl group, to which the "$C_{1-4}$ alkoxy group" defined above is attached. Specific examples include, for example, a methoxycarbonyl group, an ethoxycarbonyl group, a 1-propyloxycarbonyl group, a 2-propyloxycarbonyl group, and a 2-methyl-2-propoxy group.

As used herein, the phrase "optionally substituted" or "substituted" means "optionally having one or more substituents in any combination at a substitutable site(s)" or "having one or more substituents in any combination at a substitutable site(s)." As used herein, specific examples of substituents include, for example, a halogen atom, a hydroxyl group, a thiol group, a nitro group, a cyano group, a formyl group, a carboxyl group, an amino group, a silyl group, a $C_{l-6}$ alkyl group, a $C_{2-6}$ alkenyl group, a $C_{2-6}$ alkynyl group, a $C_{3-8}$ cycloalkyl group, a $C_{6-10}$ aryl group, a 5- to 10-membered heteroaryl group, a 3- to 10-membered non-aromatic heterocyclic group, a $C_{1-6}$ alkoxy group, a $C_{1-6}$ alkylthio group, a $C_{3-8}$ cycloalkoxy group, a mono-$C_{1-6}$ alkylamino group, a di-$C_{1-6}$ alkylamino group, a $C_{2-7}$ acyl group or a $C_{2-7}$ alkoxycarbonyl group (provided that the amino group, the $C_{1-6}$ alkyl group, the $C_{2-6}$ alkenyl group, the $C_{2-6}$ alkynyl group, the $C_{3-8}$ cycloalkyl group, the $C_{6-10}$ aryl group, the 5- to 10-membered heteroaryl group, the 3- to 10-membered non-aromatic heterocyclic group, the $C_{1-6}$ alkoxy group, the $Ci_{-6}$ alkylthio group, the $C_{3-8}$ cycloalkoxy group, the mono-$C_{1-6}$ alkylamino group, the di-$C_{1-6}$ alkylamino group, the $C_{2-7}$ acyl group and the $C_{2-7}$ alkoxycarbonyl group may each independently have 1 to 3 groups selected from the group consisting of the following substituent group). In the present invention, substituents other than those listed above may be intended in some cases.

substituent group>

A halogen atom, a hydroxyl group, a thiol group, a nitro group, a cyano group, a silyl group, a $C_{1-6}$ alkyl group, a $C_{3-8}$ cycloalkyl group, a $C_{2-6}$ alkenyl group, a $C_{2-6}$ alkynyl group, a $C_{6-10}$ aryl group, a 5- to 10-membered heteroaryl group, a 3- to 10-membered non-aromatic heterocyclic group, a $Ci_{-6}$ alkoxy group and a $C_{1-6}$ alkylthio group.

I. Sulfonamide-including compounds

In the present invention, the sulfonamide-including compound includes compounds of the following Formula (I):

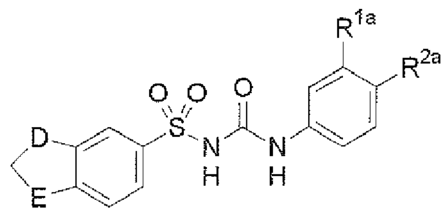

(I)

In the above Formula (I), E represents -0-, $-N(CH_3)-$, $-CH_2-$, $-CH_2CH_2-$ or -CHzO-, D represents $-CH_2-$ or -O-, $R^{la}$ represents a hydrogen atom or a halogen atom, and $R^{2a}$ represents a halogen atom or a trifluoromethyl group.

The compounds of Formula (I) according to the present invention can be prepared in a known manner, for example as described in European Patent Publication No. 0222475A1.

In Formula (I), a preferred compound is LY 186641 or LY295501.

LY186641 refers to N-[[(4-chlorophenyl)amino]carbonyl]-2,3-dihydro-1H-indene-5-sulfonamide and its structural formula is shown in the following Formula (Vfl) :

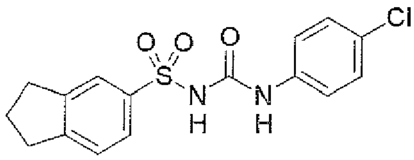

(VII)

LY186641

LY186641 can be prepared in a known manner, for example as described in European Patent Publication No. 0222475A1.

In the present invention, LY295501 refers to N-[[(3,4-dichlorophenyl)amino]-carbonyl]-2,3-dihydrobenzofuran-5-sulfonamide and its structural formula is shown in the following Formula (VIll):

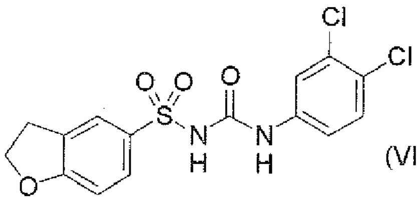

(VIII)

LY295501

LY295501 can be prepared in a known manner, for example as described in European Patent Publication No. 0222475A1 and/or European Patent Publication No. 0555036A2.

In the present invention, the sulfonamide-including compound also includes compounds of the following Formula (II);

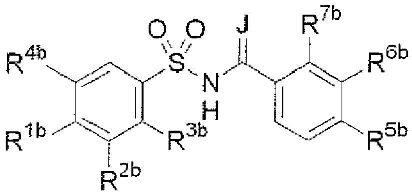

(II)

In Formula (It), J represents -O- or -NH-, $R^{1b}$ represents a hydrogen atom, a halogen atom, an optionally substituted $C_{i}$-$_{6}$ alkyl group, an optionally substituted $C_{1-4}$ alkoxy group, an optionally substituted $C_{l}$-$_{4}$ alkylthio group, an optionally substituted $C_{2-5}$ alkoxycarbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, an optionally substituted $C_{1}$-$_{6}$ alkyl group, an optionally substituted $C_{2-5}$ alkoxycarbonyl group, an optionally substituted $C_{1}$-$_{4}$ alkoxy group, an optionally substituted phenyl group or an optionally substituted quinolinyl group, $R^{3b}$ represents a hydrogen atom or an optionally substituted $C_{1-4}$ alkoxy group, $R^{4b}$ represents a hydrogen atom or an optionally substituted $C_{1-6}$ alkyl group (provided that at least one of $R^{3b}$ and $R^{4b}$ is a hydrogen atom), $R^{5b}$ represents a hydrogen atom, a halogen atom, an optionally substituted $C_{l-6}$ alkyl group or a nitro group, $R^{6b}$ represents a hydrogen atom, a halogen atom or an optionally substituted $C_{l}$-$_{6}$ alkyl group (provided that when $R^{6b}$ is an optionally substituted $C_{1-6}$ alkyl group, $R^{5b}$ is a hydrogen atom and $R^{7b}$

is a halogen atom), and R 7b represents a halogen atom or an optionally substituted $C_{1-6}$ alkyl group (provided that when either $R^{5b}$ or $R^{7b}$ is an optionally substituted $C_{1-6}$ alkyl group or when $R^{7b}$ is a halogen atom or an optionally substituted $C_{1-6}$ alkyl group, either $R^{5b}$ or $R^{6b}$ is a hydrogen atom).

In Formula (II), the "$C_{l-6}$ alkyl group" may preferably include a methyl group, an ethyl group, a 1-propyl group, a 2-propyl group, a 2-methyl-1-propyl group, a 2-methyl-2-propyl group, a 1-butyl group, and a 2-butyl group. Among them, most preferred are a methyl group, an ethyl group, a 1-propyl group, and a 2-propyl group.

Likewise, in Formula (II), the optionally substituted $C_{i-6}$ alkyl group may be, for example, a trifluoromethyl group.

In Formula (II), the "$C_{1-4}$ alkoxy group" may preferably include a methoxy group, an ethoxy group, a 1-propoxy group, a 2-propoxy group, and a 1-butoxy group.

Likewise, in Formula (II), the optionally substituted $C_{l-4}$ alkoxy group may be, for example, $-OCF_3$.

In Formula (II), the "$C_{1-4}$ alkylthio group" may preferably include a methylthio group, an ethylthio group, a 1-propylthio group, a 2-propylthio group, a 2-methyl-l-propylthio group, a 2-methyl-2-propyl group, a 1-butylthio group, and a 2-butylthio group.

Likewise, in Formula (II), the optionally substituted $C_{1-4}$ alkylthio group may be, for example, $-SCF_3$.

In Formula (II), the "$C_{2-5}$ alkoxycarbonyl group" may preferably include a methoxycarbonyl group, an ethoxycarbonyl group, a 1-propyloxycarbonyl group, a 2-propyloxycarbonyl group, and a t-butoxycarbonyl group.

In Formula (II), examples of substituents to be introduced may include those exemplified by a $C_{1-6}$ alkyl group, a $C_{1-4}$ alkoxy group, an amino group, a hydroxyl group, a halogen atom or a silyl group.

The compounds of Formula (II) according to the present invention can be prepared in a known manner, for example as described in International Publication No. W0021038848.

In Formula (II), a preferred compound is LY-ASAP

LY-ASAP refers to N-(2,4-dichlorobenzoyl)-4-cfAorophenylsulfonamide and its structural formula is shown in the following Formula (XI):

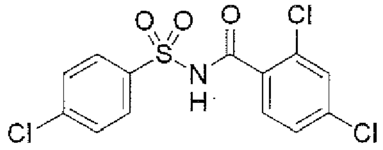
(XI)

LY-ASAP

LY-ASAP can be prepared in a known manner, for example as described in International Publication No. WO02/098848.

In the present invention, the sulfonamide-including compound may also include LY573636. In the present invention, LY573636 refers to N-(2,4-dichlorobenzoyl)-5-bromothiophene-2-sulfonamide and its structural formula is shown in the following Formula

(III):

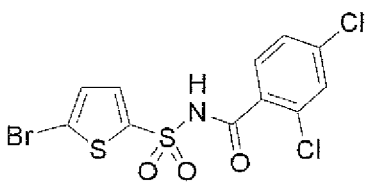
(III)

LY573636

LY573636 is preferably in sodium salt form.

LY573636 can be prepared in a known manner, for example, in the same manner as described in International Publication No. WO02/098848, starting with commercially available 5-bromothiophene-2-sulfonyl chloride and 2,4-dichlorobenzoic acid.

In the present invention, the sulfonamide-including compound may also include CQS. In the present invention,

CQS refers to 2-sulfanylamido-5-chlorocluinoxaline and its structural formula is shown in the following Formula (IV):

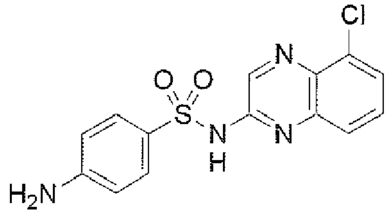

(IV)

CQS

CQS can be prepared in a known manner, for example as described in J. Am. Chem. Soc., 1947, 71, 6-10.

Moreover, in the present invention, the sulfonamide-including compound may also include E7070. E7070 refers to N-(3-chloro-1H-indol-7-yl)-4-sulfamoylbenzenesulfonamide and its structural formula is shown in the following Formula (IX)

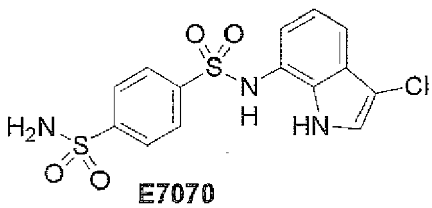

(IX)

E7070

E7070 can be prepared in a known manner, for example as described in International Publication No. WO95/07276 or in Example 19 of JP 7-165708 A.

In the present invention, the sulfonamide-including compound also includes compounds of the following Formula (XIV)

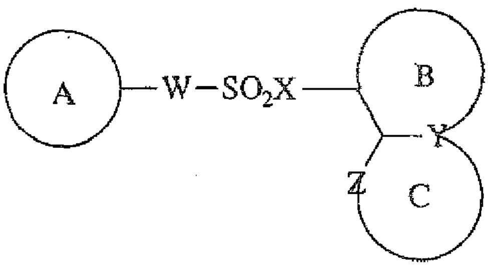

(XIV)

In the above Formula (XIV),

the ring A represents an optionally substituted monocyclic or bicyclic aromatic ring,

the ring B represents an optionally substituted 6-membered cyclic unsaturated hydrocarbon or an optionally substituted unsaturated 6-membered heterocyclic ring containing one nitrogen atom as a heteroatom,

the ring C represents an optionally substituted 5-membered heterocyclic ring containing one or two nitrogen atoms,

W represents a single bond or -CH=CH-,

X represents -N($R^1$)- or an oxygen atom,

Y represents:

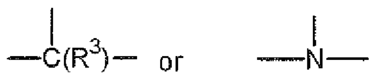

and

Z represents $-N(R^2)-$.

In these formulae, $R^1$, $R^2$ and $R^3$, which may be the same or different, each independently represents a hydrogen atom or a lower alkyl group.

The "optionally substituted monocyclic or bicyclic aromatic ring" defined for the ring A refers to an aromatic hydrocarbon or an aromatic heterocyclic ring containing at least one of a nitrogen atom, an oxygen atom and a sulfur atom. Such a ring may have 1 to 3 substituents. Examples of major aromatic rings encompassed by the ring A may include pyrrole, pyrazole, imidazole, thiophene, furan, thiazole, oxazole, benzene, pyridine, pyrimidine, pyrazine, pyridazine, naphthalene, quinoline, isoquinoline, phthalazine, naphthylizine, quinoxaline, quinazoline, cinnoline, indole, isoindole, indolizine, indazole, benzofuran, benzothiophene, benzoxazole, benzimidazole, benzopyrazole, and benzothiazole. These aromatic rings may have 1 to 3 substituents, which may be the same or different. Examples of substituents may include an optionally lower alkyl- or lower cycloalkyl-substituted amino group, a lower alkyl group, a lower alkoxy group, a hydroxyl group, a nitro group, a mercapto group, a cyano group, a lower alkylthio group, a halogen atom, a group represented by the formula -a-b [wherein a represents a single bond, $-(CH_2)_k-$, $-O-(CH_2)_k-$, $-S-(CH_2)_k-$ or $-N(R^3)$ $_(CH_z)_k-$, k represents an integer of 1 to 5, $R^3$ represents a hydrogen atom or a lower alkyl group, and b represents $-CH_2-d$ (wherein d represents an optionally lower alkyl-substituted amino group, a halogen atom, a hydroxyl group, a lower alkylthio group, a cyano group or a lower alkoxy group)], a group represented by the formula -a-e-f [wherein a is as defined above, e represents -S(O)- or $-S(O)_2-$, and f represents an optionally lower alkyl- or lower alkoxy-substituted amino group, a lower allcyl group, a trifluoromethyl group, $-(CH_z)_m-b$ or $-N(R^4)-(CH_2)_m-b$ (wherein b is as defined above, $R^4$ represents a hydrogen atom or a lower alkyl group, and m represents an integer of 1 to 5)], a group represented by the formula -a-g-h [wherein a is as defined above, g represents - C(O)- or -C(S)-, and h represents an optionally lower alkyl-substituted amino group, a hydroxyl group, a lower alkyl group, a lower alkoxy group, $-(CH_{2)n}-b$ or $-N(R^5)-(CH_2)_n-b$ (wherein b is as defined above, $R^5$ represents a hydrogen atom or a lower alkyl group, and n represents an integer of I to 5)], a group represented by the formula $-a-N(R^6)-g-i$ [wherein a and g are as defined above, $R^6$ represents a hydrogen atom or a lower alkyl group, and i represents a hydrogen atom, a lower alkoxy group or f (wherein f is as defined above)], a group represented by the formula $-a-N(R^7)-e-f$ (wherein a, e and f are as defined above, and $R^7$ represents a hydrogen atom or a lower alkyl group), or a group represented by the formula - $(CH_2)_p-j-(CH_2)_q-b$ (wherein j represents an oxygen atom or a sulfur atom, b is as defined above, and p and q, which may be the same or different, each represent an integer of 1 to 5).

In the above examples of substituents, in the case of an amino group substituted with two alkyl groups, these alkyl groups may together form a 5- or 6-membered ring. Likewise, in a case where the ring A is a nitrogen-containing heterocyclic ring having a hydroxyl group or a mercapto group, these groups may form an oxo group or a thioxo group by taking the resonance structure.

In Formula (XTV), the "optionally substituted 6-membered cyclic unsaturated hydrocarbon" or the "optionally substituted unsaturated 6-membered heterocyclic ring containing one nitrogen atom as a heteroatom" defined for the ring B refers to, for example, benzene or pyridine whose unsaturated bonds may be partially hydrogenated. Such a ring may have one or more substituents, which may be the same or different.

The "optionally substituted 5-membered heterocyclic ring containing one or two nitrogen atoms" defined for the ring C refers to pyrrole, pyrazole or imidazole whose unsaturated bonds may be partially hydrogenated. Such a ring may have one or two substituents, which may be the same or different.

In Formula (XIV), Z represents $-N(R^2)-$. $R^2$ may be the same as or different from $R^1$ described later and represents a hydrogen atom or a lower alkyl group.

Examples of substituents which may be on the rings B and C include a halogen atom, a cyano group, a lower alkyl group, a lower alkoxy group, a hydroxyl group, an oxo group, a group represented by the formula -C(O)-r (wherein r represents a hydrogen atom, an optionally lower alkyl-substituted amino group, a lower alkyl group, a lower alkoxy group or a hydroxyl group), an optionally lower alkyl-substituted amino group, and a trifluoromethyl group.

In Formula (XIV), Y represents:

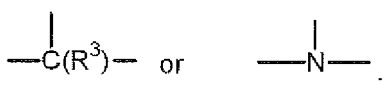

In the above formula, $R^3$ represents a hydrogen atom or a lower alkyl group.

In Formula (XIV), W represents a single bond or -CH=CH-, and X represents -N($R^1$)-or an oxygen atom. $R^1$ may be the same as or different from $R^2$ and represents a hydrogen atom or a lower alkyl group.

In the above Formula (XIV), the "lower alkyl (group)" found in the definition of substituents which may be on $R^1$, $R^2$ and $R^3$ as well as the rings A, B and C refers to a linear or branched alkyl group containing 1 to 6 carbon atoms and has the same meaning as the "$C_{1-6}$ alkyl group" mentioned above.

The "lower cycloalkyl (group)" found in the definition of substituents which may be on the ring A refers to a cycloalkyl group containing 3 to 8 carbon atoms and has the same meaning as the "$C_3$-$_8$ cycloalkyl group" mentioned above. Examples of such a lower cycloalkyl group include a cyclopropyl group, a cyclopentyl group, and a cyclohexyl group. Likewise, the "lower alkylthio (group)" refers to an alkylthio group derived from the above lower alkyl group and has the same meaning as the "$C_l$-$_6$ alkylthio group" mentioned above.

The "lower alkoxy (group)" found in the definition of substituents which may be on the rings A, B and C refers to, for example, a lower alkoxy group derived from the above lower alkyl group, as exemplified by a methoxy group, an ethoxy group, a n-propoxy group, an isopropoxy group, a n-butoxy group, an isobutoxy group, and a tert-butoxy group. Among them, most preferred are a methoxy group and an ethoxy group. Likewise, examples of the "halogen atom" include a fluorine atom, a chlorine atom, a bromine atom, and an iodine atom.

The compounds of Formula (XIV) according to the present invention can be prepared in a known manner, for example as described in International Publication No. WO95/07276 and/or JP 7-165708 A.

In Formula (I), a preferred compound is the E7070 mentioned above or E7820.

E7820 refers to N-(3-cyano-4- methyl-l H - indol- 7 - yl)- 3-cyanobenzenesulfonamide and its structural formula is shown in the following Formula (X):

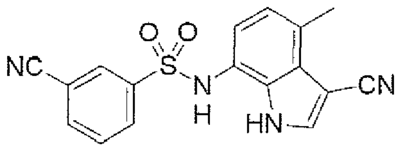

(X)

E7820

E7820 can be prepared in a known manner, for example as described in International Publication No. WO00/50395.

These sulfonamide-including compounds may form pharmacologically acceptable salts with acids or bases. The compounds according to the present invention also encompass these pharmacologically acceptable salts. Examples of salts with acids include inorganic acid salts (e.g., hydrochloride salt, hydrobromide salt, sulfate salt, phosphate salt), as well as salts with organic acids such as formic acid, acetic acid, lactic acid, succinic acid, fumaric acid, maleic acid, citric acid, tartaric acid, benzoic acid, methanesulfonic acid, benzenesulfonic acid, p-toluenesulfonic acid and trifluoroacetic acid. Likewise, examples of salts with bases include alkali metal salts (e.g., sodium salt, potassium salt), alkaline earth metal salts (e.g., calcium salt, magnesium salt), salts with organic bases such as trimethylamine, triethylamine, pyridine, picoline, dicyclohexylamine, N,N'-dibenzylethylenediamine, arginine and lysine (organic amine salts), as well as ammonium salts.

Alternatively, these sulfonamide-including compounds may be in anhydride form or may form solvates such as hydrates. Solvates may be either hydrates or anhydrates, preferably hydrates. Solvents available for use include water, alcohols (e.g., methanol, ethanol, n-propanol) and dimethylformamide.

In a case where these compounds are present in solvate form and/or have optical isomers, the compounds according to the present invention also encompass their solvates and/or optical isomers. The compounds according to the present invention further encompass sulfonamide-including compounds that are metabolized *in* vivo by oxidation, reduction, hydrolysis, conjugation, etc. Furthermore, the compounds according to the present invention encompass compounds that produce sulfonamide-including compounds when metabolized *in vivo* by oxidation, reduction, hydrolysis, etc.

2. Angiogenesis inhibitors

As a result of combining a sulfonamide-including compound with an angiogenesis inhibitor, the present invention provides pharmaceutical compositions and kits showing excellent angiogenesis inhibitory activity and/or anti-tumor activity.

For convenience purposes, the angiogenesis inhibitor is also referred to as an angiogenesis inhibitory agent

in the following explanation.

In the present invention, the angiogenesis inhibitor is not limited in any way as long as it has an inhibitory activity on angiogenesis. Examples of such an angiogenesis inhibitor include VEGF (vascular endothelial growth factor) inhibitors (e.g., VEGF receptor kinase inhibitors, anti-VEGF antibodies (Cancer Research., 55, 529b-5301, 1995)), FGF (fibroblast growth factor) inhibitors (e.g., FGF receptor kinase inhibitors, anti-FGF antibodies (Cancer Research., 51, 6180-4, 1991)), PDGF (platelet-derived growth factor) inhibitors (e.g., PDGF receptor kinase inhibitors (J. Clinical Investigation., 111, 1287-95)), integrin inhibitors (e,g., αvβ3 integrin inhibitors, αvβ5 integrin inhibitors (Clinical Cancer Research., 6, 3056-61, 2000)), endogenous inhibitors (e.g., IL-12, Trombospondin-1, Endostatin, Angiostatin (International J. Cancer., 78, 361-5, 1998), COX-2 inhibitors (Annuals ofIV.Y Acad. Science., 84-6, 1999)), matrix metalloprotein inhibitors (International J. Pancreatol., 21, 1-12, 1997), as well as other inhibitors (e.g., farnesyltransferase inhibitors, nitric oxide inhibitors, angiotensin converting enzyme inhibitors, HMG-CoA reductase inhibitors, Vascular Target inhibitors, methionine aminopeptidase inhibitors (Science., 282, 1324-1327, 1998)). Preferred examples are VEGF receptor kinase inhibitors, anti-VEGF antibodies, FGF receptor kinase inhibitors and anti-FGF antibodies, and more preferred examples are VEGF receptor kinase inhibitors and anti-VEGF antibodies.

(1) VEGF receptor kinase inhibitors

In the present invention, the VEGF receptor kinase inhibitor may include, for example, compounds of Formula (XXIV):

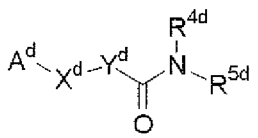

(XXIV)

(i) $A^d$

$A^d$ represents a group represented by the following formula:

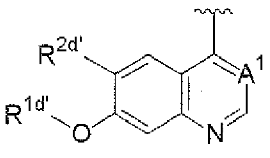

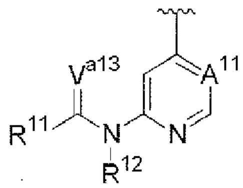

or

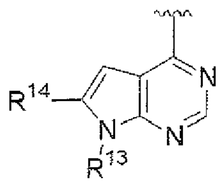

(wherein R1d' represents a group represented by the formula $-V^1-V^2-V^3$ (wherein $V^1$ represents an optionally substituted $C_{1-6}$ alkylene group; $V^2$ represents a single bond, an oxygen atom, a sulfur atom, a carbonyl group, a sulfinyl group, a sulfonyl group, a group represented by the formula $-CONR^{6d}-$, a group represented by the formula $-SO_2NR^{6d}-$, a group represented by the formula $-NR^{6d}SO_2-$, a group represented by the formula $-NR^{6d}CO-$ or a group represented by the formula $-NR^{6d}-$ (wherein $R^{6d}$ represents a hydrogen atom, an optionally substituted $C_{i-6}$ alkyl group or an optionally substituted $C_{3-8}$ cyc10alkyl group); and $V^3$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5- to 10-membered heteroaryl group or an optionally substituted 3- to 10-membered non-aromatic heterocyclic group);

$R^{2d'}$ represents a cyano group, an optionally substituted $C_{l-6}$ alkoxy group, a carboxyl group, an optionally substituted $C_{2-7}$ alkoxycarbonyl group or a group represented by the formula $-CONV^{a11}V^{a12}$ (wherein $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-9}$ cycloalkyl group, an optionally substituted $C_{6-io}$ aryl group, an optionally substituted 5- to 10-membered heteroaryl group or an optionally substituted 3- to 10-membered non-aromatic heterocyclic group; and $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-i}$o aryl group, an optionally substituted 5- to 10-membered heteroaryl group, an

optionally substituted 3- to 10-membered non-aromatic heterocyclic group, a hydroxyl group, an optionally substituted $C_{i}$-$_{6}$ alkoxy group or an optionally substituted $C_{3}$-$_{S}$ cycloalkoxy group);
$A^1$ represents an optionally substituted carbon atom or a nitrogen atom;
$R^{11}$ represents a hydrogen atom, an optionally substituted $C_{1-6}$ alkyl group, an optionally substituted $C_{2-6}$ alkenyl group, an optionally substituted $C_{2}$-$_{6}$ alkynyl group, an optionally substituted $C_{3-8}$ cycloalkyl group, an optionally substituted $C_{6-10}$ aryl group, an optionally substituted 5- to 10-membered heteroaryl group, an optionally substituted 3- to 10-membered non-aromatic heterocyclic group or an optionally substituted mono-$C_{1-6}$ alkylamino group;
R $^{12}$ represents a hydrogen atom or an optionally substituted $C_{1-6}$ alkyl group;
$V^{a}$!3 represents an oxygen atom or a sulfur atom,
All represents an optionally substituted carbon atom or a nitrogen atom;
$R^{13}$ represents a hydrogen atom, an optionally substituted $C_{i}$-$_{6}$ alkyl group or an optionally substituted $C_{3_}$g cycloalkyl group; and
$R1^4$ represents a group represented by the formula a - $V^{a14}$ - $V^{a15}$ (wherein $V^{a14}$ represents a single bond or a carbonyl group; and $V^{a15}$ represents a hydrogen atom, a hydroxyl group, an optionally substituted $C_{1-6}$ alkyl group, an optionally substituted $C_{2}$-$_{6}$ alkenyl group, an optionally substituted $C_{2-6}$ alkynyl group, an optionally substituted $C_{3-8}$ cycloalkyl group, an optionally substituted $C_{6}$-$_{io}$ aryl group, an optionally substituted 5- to 10-membered heteroaryl group, an optionally substituted 3- to 10-membered non-aromatic heterocyclic group, an amino group, an optionally substituted mono-$C_{1-6}$ alkylamino group, an optionally substituted di-$C_{1-6}$ alkylamino group, a formyl group, a carboxyl group or an optionally substituted $C_{2}$-$_{7}$ alkoxycarbonyl group)).

(ii) $X^d$
$X^d$ represents an oxygen atom or a sulfur atom.
(iii) yd
$Y^d$ represents a group represented by the following formula:

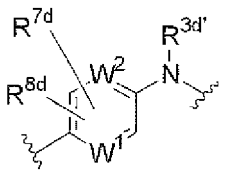
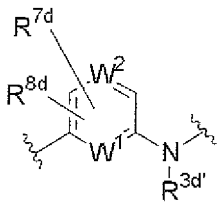
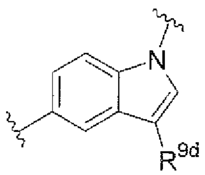
or
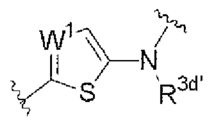

(wherein $R^{3d'}$ represents a hydrogen atom, an optionally substituted $C_{1-6}$ alkyl group, an optionally substituted $C_{2}$-$_{6}$ alkenyl group, an optionally substituted $C_{2}$-$_{6}$ alkynyl group, an optionally substituted $C_{3}$-$_{8}$ cycloalkyl group, an optionally substituted $C_{2}$-$_{7}$ acyl group or an optionally substituted $C_{2}$-$_{7}$ alkoxycarbonyl group;

$R^{7d}$ and $R^{8d}$ each independently represent a hydrogen atom, a halogen atom, a cyano group, a nitro group, an amino group, an optionally substituted $C_{1-6}$ alkyl group, an optionally substituted $C_{3}$-$_{8}$ cycloalkyl group, an optionally substituted $C_{1-6}$ alkoxy group, an optionally substituted $C_{1-6}$ alkylthio group, a formyl group, an optionally substituted $C_{2}$-$_{7}$ acyl group, an optionally substituted $C_{2}$-$_{7}$ alkoxycarbonyl group or a group represented by the formula - CON$V^{d1}V^{d2}$ (wherein $V^{d1}$ and $V^{d2}$ each independently represent a hydrogen atom or an optionally substituted Ci-$_{6}$ alkyl group);
$R^{9d}$ represents a hydrogen atom, a halogen atom or a $C_{l}$-$_{6}$ alkyl group; and
$W^1$ and $W^2$ each independently represent an optionally substituted carbon atom or a nitrogen atom).

(iv) $R^{4d}$
$R^{4d}$ represents a hydrogen atom, an optionally substituted $C_{l}$-$_{6}$ alkyl group, an optionally substituted $C_{2}$-$_{6}$ alkenyl group, an optionally substituted $C_{2-6}$ alkynyl group, an optionally substituted $C_{3-8}$ cycloalkyl group, an optionally substituted $C_{2}$-$_{7}$ acyl group or an optionally substituted $C_{2-7}$ alkoxycarbonyl group.
(v) $R^{5d}$
$R^{5d}$ represents a hydrogen atom, an optionally substituted $C_{1-6}$ alkyl group, an optionally substituted $C_{2-6}$ alkenyl group, an optionally substituted $C_{2}$-$_{6}$ alkynyl group, an optionally substituted $C_{3-8}$ cycloalkyl group, an optionally substituted $C_{6-10}$ aryl group, an optionally substituted 5- to 10-membered heteroaryl group or an optionally substituted 3- to 10-membered non-aromatic heterocyclic group.

The compounds of Formula (XXIV) can be prepared in a known manner, for example as described in International Publication No. WO02/032872, International Publication No. W02004/020434, and/or International Publication No.

W02005/063713.

In the present invention, the VEGS receptor kinase inhibitor may preferably include compounds of Formula (XXV):

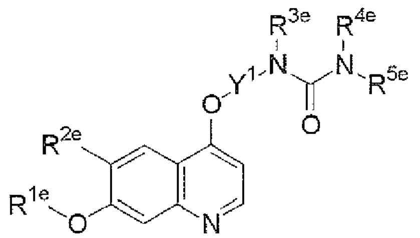

(XXV)

Formula (XXV) is a preferred example of the compounds of Formula (XXIV).

(i) $R^{1e}$

$R^{1e}$ represents a group represented by the formula $-V^{1e}-V^{2e}-V^{3e}$ (wherein Vle represents an optionally substituted $C_{1-6}$ alkylene group; $V^{2e}$ represents a single bond, an oxygen atom, a sulfur atom, a carbonyl group, a sulfinyl group, a sulfonyl group, a group represented by the formula $-CONR^{6e}-$, a group represented by the formula $-SO_2NR^{6e}-$, a group represented by the formula $-NR^{6e}SO_2-$, a group represented by the formula $-NR^{6e}CO-$ or a group represented by the formula $-NR^{6e}-$ (wherein $R^{6e}$ represents a hydrogen atom, an optionally substituted $C_{1-6}$ alkyl group or an optionally substituted $C_{3-8}$ cycloalkyl group); and $V^{3e}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5- to 10-membered heteroaryl group or an optionally substituted 3- to 10-membered non-aromatic heterocyclic group).

Preferred examples of $R^{1e}$ include a $C_{1-6}$ alkyl group. In this case, however, $R^{1e}$ may have one or more substituents selected from a 3- to 10-membered non-aromatic heterocyclic group which may have a $C_{1-6}$ alkyl group, a hydroxyl group, a $C_{1-6}$ alkoxy group, an amino group, a mono-$C_{1-6}$ alkylamino group and a di-$C_{1-6}$ alkylamino group.

More preferred examples ofR1e include a methyl group or a group represented by any of the following formulae:

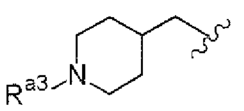

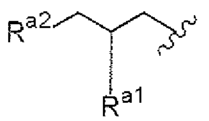

or

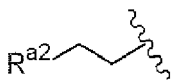

(wherein R a3 represents a methyl group; $R^{a1}$ represents a hydrogen atom or a hydroxyl group; and $R^{a2}$ represents a methoxy group, an ethoxy group, a 1-pyrrolidinyl group, a 1-piperidinyl group, a 4-morpholinyl group, a dimethylamino group or a diethylamino group).

Even more preferred examples of $R^{1e}$ include a methyl group or a 2-methoxyethyl group.

(ii) $R^{2e}$

$R^{ze}$ represents a cyano group, an optionally substituted $C_{l\cdot 6}$ alkoxy group, a carboxyl group, an optionally substituted $Cz_{_7}$ alkoxycarbonyl group or a group represented by the formula $-CONV^{e11}V^{e12}$ (wherein yell represents a hydrogen atom, an optionally substituted $C_{1}$-$_{6}$ alkyl group, an optionally substituted $C_{2\cdot 6}$ alkenyl group, an optionally substituted $C_{2}$-$_{6}$ alkynyl group, an optionally substituted $C_{3}$-$_{8}$ cycloalkyl group, an optionally substituted $C_{6}$-$_{10}$ aryl group, an optionally substituted 5- to 10-membered heteroaryl group or an optionally substituted 3- to 10-membered non-aromatic heterocyclic group; and $^{ye}$l2 represents a hydrogen atom, an optionally substituted $C_{1-6}$ alkyl group, an optionally substituted $C_{2}$-$_{6}$ alkenyl group, an optionally substituted $C_{2-6}$ alkynyl group, an optionally substituted $C_{3-8}$ cycloalkyl group, an optionally substituted $C_{6}$-io aryl group, an optionally substituted 5- to 10-membered heteroaryl group, an optionally substituted 3- to 10-membered non-aromatic heterocyclic group, a hydroxyl group, an optionally substituted $C_{l_6}$ alkoxy group or an optionally substituted $C_{3}$-$_{8}$ cycloalkoxy group).

Preferred examples of $R^{2e}$ include a cyano group or a group represented by the formula $-CONV^{e11}V^{12}$ (wherein $V^{e11}$ and $V^{e12}$ are as defined above).

More preferred examples of $R^{2e}$ include a cyano group or a group represented by the formula $-CONHV^{e16}$ (wherein $V^{e16}$ represents a hydrogen atom, a $C_{1-6}$ alkyl group, a $C_{3}$-$_{s}$ cycloalkyl group, a $C_{1-6}$ alkoxy group or a $C_{3-8}$ cycloalkoxy group, provided that $^{yl6}$ may have one or more substituents selected from a halogen atom, a cyano group,

a hydroxyl group and a $C_{1-6}$ alkoxy group).

Even more preferred examples of $R^{2e}$ include a group represented by the formula - $CONHV^{e17}$ (wherein $V^{e17}$ represents a hydrogen atom, a $C_{l}$-$_{6}$ alkyl group or a $C_{1}$-$_{6}$ alkoxy group).

Most preferred examples of $R^{2e}$ include a group represented by the formula - $_{m}$ $CONHV^{e18}$ (wherein $V^{e18}$ represents a hydrogen atom, a methyl group or a methoxy group).

(iii) $Y^1$

$Y^1$ represents a group represented by the following formula:

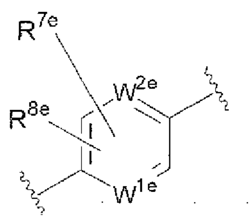

or

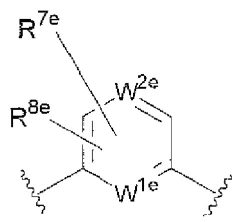

(wherein $R^{7e}$ and $R^{8e}$ each independently represent a hydrogen atom, a halogen atom, a cyano group, a nitro group, an amino group, an optionally substituted $C_{1-6}$ alkyl group, an optionally substituted $C_{3-8}$ cycloalkyl group, an optionally substituted $C_{1-6}$ alkoxy group, a formyl group, an optionally substituted $C_{2-7}$ acyl group, an optionally substituted $C_{2-7}$ alkoxycarbonyl group or a group represented by the formula $-CONV^{e1}V^{e2}$ (wherein Vel and $V^{e2}$ each independently represent a hydrogen atom or an optionally substituted $C_{1-6}$ alkyl group); and

$W^{1e}$ and $W^{2e}$ each independently represent an optionally substituted carbon atom or a nitrogen atom).

Preferred examples of $Y^1$ include a group represented by the following formula:

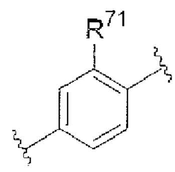

(wherein $R^{71}$ represents a hydrogen atom or a halogen atom).

(iv) $R^{3e}$ and $R^{4e}$

$R^{3e}$ and $R^{4e}$ each independently represent a hydrogen atom, an optionally substituted $C_{1-6}$ alkyl group, an optionally substituted $C_{2-6}$ alkenyl group, an optionally substituted $C_{2}$-$_{s}$ alkynyl group, an optionally substituted $C_{3-8}$ cycloalkyl group, an optionally substituted Z2-7 acyl group or an optionally substituted $C_{2-7}$ alkoxycarbonyl group.

Preferred examples of $R^{3e}$ and $R^{4e}$ include a hydrogen atom.

(v) $R^{5e}$

$R^{5e}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5- to 10-membered heteroaryl group or an optionally substituted 3- to 10-membered non-aromatic heterocyclic group.

Preferred examples of $R^{5e}$ include a hydrogen atom, a $C_{1-6}$ alkyl group, a $C_{3-8}$ cycloalkyl group or a $C_{6-10}$ aryl group (provided that $R^{5e}$ may have one or more substituents selected from a halogen atom and a methanesulfonyl group).

More preferred examples of $R^{5e}$ include a methyl group, an ethyl group or a cyclopropyl group.

Moreover, preferred examples of the compounds of Formula (XXV) may include compounds of Formula (V):

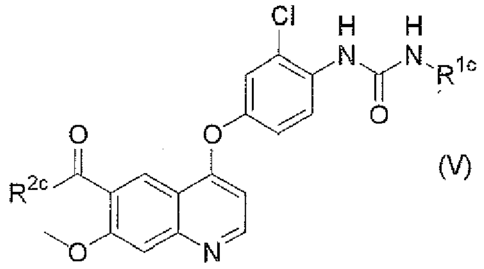

(V)

[wherein $R^{1c}$ represents a hydrogen atom, a methyl group, an ethyl group, a n-propyl group or a cyclopropyl group, and $R^{2c}$ represents $-NH_2$ or $-NHOCH_3$].

$R^{1c}$ may have a substituent such as an alkyl group (e.g., a methyl group, an ethyl group, a propyl group, a butyl group), an alkoxy group (e.g., a methoxy group, an ethoxy group, a propoxy group, a butoxy group), an amino group, a hydroxyl group, a halogen atom or a silyl group.

Preferred examples of the compounds of Formula (XXV) may also include:

N-(4-(6-cyano-7-(2-methoxyethoxy)-4-quinolyl)oxy-2-fluorophenyl)-N'-(4-fluorophenyl)urea,
N-(2-chloro-4-((6-cyano-7-((1-methyl-4-piperidyl)methoxy)-4-quinolyl)oxy)phenyl)-N'-cyclopropylurea,
N-(4-((6-cyano-7-(((2R)-3-(diethylamino)-2-hydroxypropyl)oxy)-4-quinoiyl)oxy)phenyl)-N'-(4-fluorophenyl)urea,
N-(4-((6-cyano-7-(((2R)-2-hydroxy-3-(1-pyrrolidino)propyl)oxy)-4-quinolyl)oxy)phenyl)-N'-(4-fluorophenyl)urea,
4-(3-chloro-4-(cycfopropylaminocarbonyl)aminophenoxy)-7-methoxy-6-quinolinecarboxamide,
4-(3-chloro-4-(cyclopropylaminocarbonyl)atninophenoxy)-7-(2-methoxyethoxy)-6-quinolinecarboxamide,
N6-cyclopropyl-4-(3-chloro-4-(((cyclopropylamino)carbonyl)amino)phenoxy)-7-methoxy-6-quinolinecarboxamide,
N6-(2-metboxyethyl)-4-(3-chloro-4-(((cyclopropylamino)carbonyl)amlno)phenoxy)-7-methoxy-6-quinolinecarboxamide,
N6-(2-fluoroethyl)-4-(3-chloro-4-(((cyclopropylamino)carbonyl)amino)phenoxy)-7-methoxy-6-quinolinecarboxamide,
N6-methoxy-4-(3-chloro-4-(((cyclopropylamino)carbonyl)amino)phenoxy)-7-methoxy-6-quinolinecarboxamide,
N6-methyl-4-(3-chloro-4-(((cyclopropylamino)carbonyl)amino)phenoxy)-7-methoxy-6-quinolinecarboxamide,
N6-ethyl-4-(3-chloro-4-(((cyclopropylarnino)carbonyl)amino)phenoxy)-7-methoxy-6-quinolinecarboxamide,
4-(3-fluoro-4-(cyclopropylaminocarbonyl)aminophenoxy)-7-(2-methoxyethoxy)-6-quinolinecarboxamide,
4-(3-chloro-4-(cydopropylaminocarbonyl)aminophenoxy)-7-(2-hydroxyethoxy)-6-quinolinecarboxamide,
4-(3-chloro-4-(cyclopropylaminocarbonyl)aminophenoxy)-7-((2S)-2,3-dihydroxypropyl)oxy-6-quinolinecarboxamide,
4-(3-chloro-4-(methylaminocarbonyl)aminophenoxy)-7-methoxy-6-quinolinecarboxamide,
4-(3-chloro-4-(ethylaminocarbonyl)aminophenoxy)-7-methoxy-6-quinolinecarboxamide,
N6-methoxy-4-(3-chloro-4-(((ethylamino)carbonyl)amino)phenoxy)-7-methoxy-6-quinolinecarboxarnide,
4-(3-chloro-4-(cydopropylaminocarbonyl)aminophenoxy)-7-(2-ethoxyethoxy)-6-quinolinecarboxamide,
4-(4-((cyc!opropylammo)carbonyl)aminophenoxy)-7-(2-methoxyethoxy)-6-quinolinecarboxamide,
N-(2-fluoro-4-((6-carbamoyl-7-methoxy-4-quinolyl)oxy)phenyl)-N'-cydopropylurea,
N6-(2-hydroxyethyl)-4-(3-chloro-4-(((cydopropylamino)carbonyl)amino)phenoxy)-7-methoxy-6-quinolinecarboxamide,
4-(3-chloro-4-(1-propylaminocarbonyl)aminophenoxy)-7-methoxy-6-quinolinecarboxamide,
4-(3-chloro-4-(cis-2-fluoro-cyclopropylaminocarbonyl)aminophenoxy)-7-methoxy-6-quinolinecarboxamide,
N6-methyl-4-(3-chloro-4-(((cyclopropylamino)carbonyl)amino)phenoxy)-7-(2-methoxyethoxy)-6-quinolinecarboxamide,
N6-methyl-4-(3-chloro-4-(((ethylamino)carbonyl)amino)phenoxy)-7-methoxy-6-quinolinecarboxamide,
4-(3-chloro-4-(cyclopropylaminocarbonyl)aminophenoxy)-7-(2-(4-morpholino)ethoxy)-5-quinolinecarboxamide,
4-(3-chloro-4-(2-fluoroethylaminocarbonyl)aminophenoxy)-7-methoxy-6-quinolinecarboxamide,
N6-((2R)tetrahydro-2-foranylmethyl)-4-(3-chloro-4-(((methylamino)carbonyl)ainino)phenoxy)-7-methoxy-6-quinolinecarboxamide,
4-(3- fluoro-4-(ethylaminocarbonyl)aminophenoxy)-7-methoxy-6-quinolinecarboxamide,
4-(3-chloro-4-(((cydopropylamino)carbonyl)arnino)phenoxy)-7-((2R)-2-hydroxy-3-(1-pyrrolidino)propoxy)-6-quinolinecarboxamide,
N6-methyl-4-(3-chloro-4-(((methylamino)carbonyl)amino)phenoxy)-7-( (2R)-3-diethylamino-2-hydroxypropoxy)-6-quinolinecarboxarmide,

N6-methyl-4-(3-chloro-4-(((ethylamino)carbonyl)amino)phenoxy)-7-((2R)-3-diethylamino-2-hydroxypropoxy)-6-quinolinecarboxamide,
N6-methyl-4-(3-chloro-4-(((methylamino)carbonyl)amino)phenoxy)-7-((2R)-2-hydroxy-3-(1-pyrrolidino)propoxy)-6-quinolinecarboxamide,
N6-methyl-4-(3-chloro-4-(((ethylamino)carbonyl)amino)phenoxy)-7-((2R)-2-hydroxy-3-(1-pyrrolidino)propoxy)-6-quinolinecarboxamide,
N6-methyl-4-(3-chloro-4-(((methylammo)carbonyl)amino)phenoxy)-7-((1-methyl-4-piperidyl)methoxy)-6-quinolinecarboxamide,
N6-methyl-4-(3-chloro-4-(((ethylamino)carbonyl)amino)phenoxy)-7-((1-methyl-4-piperidyl)methoxy)-6-quinolinecarboxamide,
N-(4-(6-cyano-7-(2-methoxyethoxy)-4-quinolyl)oxy-2-fluorophenyl)-N'-cyclopropylurea,
N-(4-(6-cyano-7-(3-(4-morpholino)propoxy)-4-quinolyl)oxyphenyl)-N'-(3-(methylsulfonyl)phenyl)urea,
4-(4-((cydopropylamino)carbonyl)aminophenoxy)-7-methoxy-6-quinolinecarboxamide,
4-(3-fluoro-4-((2-fluoroethylarnino)carbonyl)aminophenoxy)-7-methoxy-6-quinolinecarboxamide,
N6-(2-ethoxyethyl)-4-(3-chloro-4-(((methylamino)carbonyl)amino)phenoxy)-7-methoxy-6-quinolinecarboxamide,
4-(4-(3-ethylureido)-3-fluoro-phenoxy)-7-methoxyquinoline-6-carboxylic acid (2-cyanoethyl)amide, and
N-(4-(6-(2-cyanoethyl)carbamoyl-7-methoxy-4-quinolyl)oxy-2-fluorophenyl)-N'-cyclopropylurea.

More preferred examples of the compounds of Formula (XXV) may include:

4-(3-chloro-4-(cyclopropylaminocarbonyl)aminophcnoxy)-7-methoxy-6-quinolinecarboxamide,
4-(3-chloro-4-(ethylaminocarbonyl)aminophenoxy)-7-methoxy-6-quinolinecarboxamide,
N6-methoxy-4-(3-chloro-4-(((cydopropylamino)carbonyl)amino)phenoxy)-7-methoxy-6-quinolinecarboxamide,
4-(3-chloro-4-(methylaminocarbonyl)aminophenoxy)-7-methoxy-6-quinolinecarboxamide, and
N6-methoxy-4-(3-chloro-4-(((ethylamino)carbonyl)amino)phenoxy)-7-methoxy-6-quinolinecarboxamide,

Even more preferred examples of the compounds of Formula (XXV) may include 4-(3-chloro-4-(cyclopropylaminocarbonyl)aminophenoxy)-7-metboxy-6-quinolinecarboxamide (see Formula (XV)), and the most preferred example may be a methanesulfonate salt of 4-(3-chloro-4-(cyclopropylaminocarbonyl)aminophenoxy)-7-methoxy-6-quinolinecarboxamide.

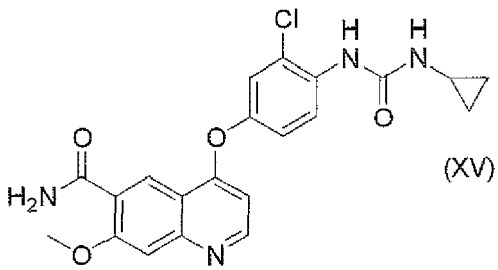

(XV)

The compounds of Formula (XXV) can be prepared in a known manner, for example as described in International Publication No. WO02/032872 and/or International Publication No. WO2005/63713.

In the present invention, the VEGF receptor kinase inhibitor may also preferably include compounds of Formula (XXVI):

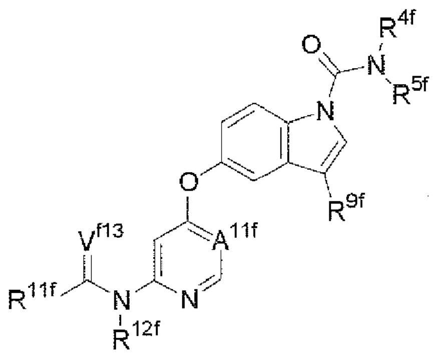

(XXVI)

Formula (XXVI) is a preferred example of the compounds of Formula (XXIV)

(i) $R^{11f}$

$R^{11f}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5- to 10-membered heteroaryl group, an optionally substituted 3- to 10-membered non-aromatic heterocyclic group or an optionally substituted mono-$C_{1-6}$ alkylamino group.

Preferred examples of $R^{11f}$ include an optionally substituted 3- to 10-membered non-aromatic heterocyclic group or an optionally substituted mono-$C_{l-6}$ alkylamino group.

More preferred examples of $R^{11f}$ include any one group selected from those represented by the following formulae:

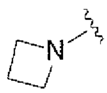 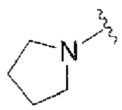 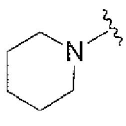 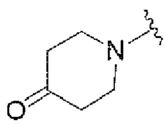 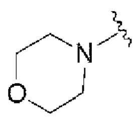

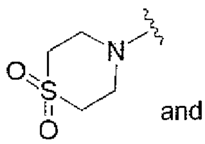 and 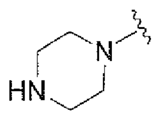

which may have one or more substituents selected from the following substituent group:

[Substituent group]

a hydroxyl group, a $C_{1-6}$ alkyl group, a $C_{3-8}$ cycloalkyl group, and groups represented by the following formulae:

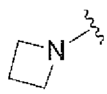 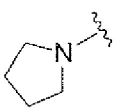 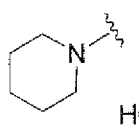 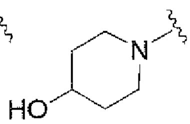 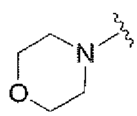 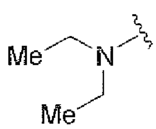

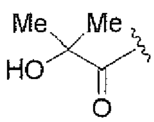 and 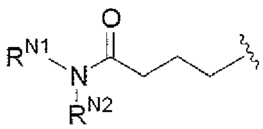

(wherein $R^{N1}$ and $R^{N2}$ each independently represent a hydrogen atom or an optionally substituted $C_{1-6}$ alkyl group; and Me denotes a methyl group).

Even more preferred examples of $R^{11f}$ include any one group selected from those represented by the following formulae;

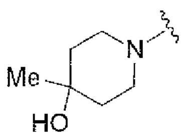 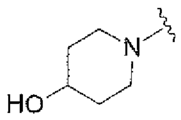 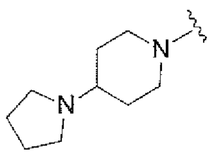 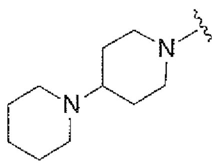 and 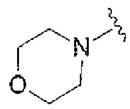

(ii) $R^{12f}$

$R^{12f}$ represents a hydrogen atom or an optionally substituted $C_{1-6}$ alkyl group.
Preferred examples of $R^{12f}$ include a hydrogen atom.
(iii) $V^{f13}$
$V^{f13}$ represents an oxygen atom or a sulfur atom.
Preferred examples of $V^{f13}$ include an oxygen atom.
(iv) $A^{11f}$
$A^{11f}$ represents an optionally substituted carbon atom or a nitrogen atom.
Preferred examples of $A^{11f}$ include a carbon atom.
(v) $R^{4f}$
$R^{4f}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{2-7}$ acyl group or an optionally substituted $C_{2-7}$ alkoxycarbonyl group.

Preferred examples of $R^{4f}$ include a hydrogen atom.
(vi) $R^{5f}$
$R^{5f}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5- to 10-membered heteroaryl group or an optionally substituted 3- to 10-membered non-aromatic heterocyclic group.

Preferred examples of $R^{5f}$ include a $C_{1-6}$ alkyl group or a $C_{3-8}$ cycloalkyl group.

More preferred examples of $R^{5f}$ include a methyl group.
(vii) $R^{9f}$
Razz represents a hydrogen atom, a halogen atom or an optionally substituted $C_{I-6}$ alkyl group.

Preferred examples of $R^{?f}$ include a hydrogen atom.

Moreover, preferred examples of the compounds of Formula (XXVI) may include compounds of Formula (VI):

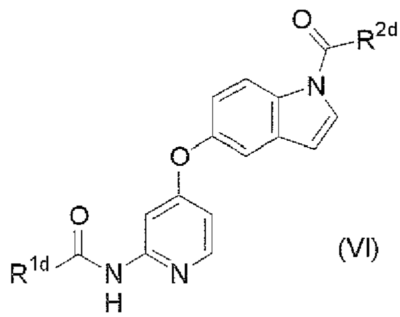

(VI)

In Formula (VI), $R^{ld}$ represents any one group selected $I_T$om the group consisting of those represented by the following formulae:

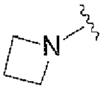 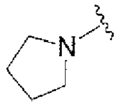 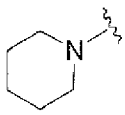 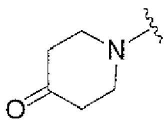 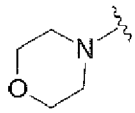

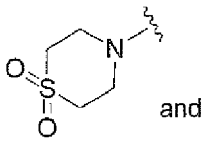 and 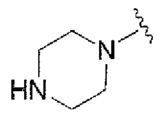

which may have one or more substituents selected from the substituent group a, and $R^{2d}$ represents $-NHR^{3d}$ (wherein $R^{3d}$ represents a methyl group, an ethyl group or a cyclopropyl group), provided that the substituent group ec denotes a group consisting of a hydroxyl group, a C.6 alkyl group, a $C_3$-& cycloalkyl group, and groups represented by the following formulae:

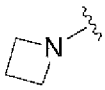 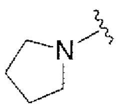 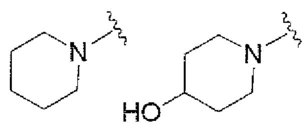 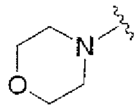 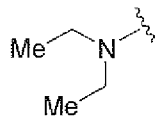

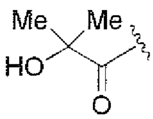 and 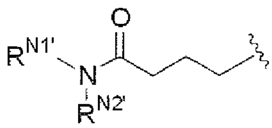

(wherein $R^{N1}$ and $R^{N2}$ each independently represent a hydrogen atom or a $C_{1-6}$ alkyl group).

$R^{3d}$ may have a substituent such as an alkyl group (e.g., a methyl group, an ethyl group, a propyl group, a butyl group), an alkoxy group (e.g., a methoxy group, an ethoxy group, a propoxy group, a butoxy group), an amino group, a hydroxyl group, a halogen atom (e.g., fluorine, chlorine, bromine, iodine) or a silyl group. Examples of substituents introduced into $R^{N1'}$ or $R^{N2'}$ may include the same substituents as mentioned above.

Preferred examples of the compounds of Formula (XXVI) may also include:

5-(2-(((4-hydroxy-4-methylpipeidin-1-yl)carbonyl)amino)pyridin-4-yloxy)-1H-inclole-1-carboxylic acid methylamide,
N1-methyl-5-(2-((4-hydroxypipendino)carbonyl)amino-4-pyridyl)oxy-1H-1-indolecarboxamide,
N1-methyl-5-(2-(((4-(pyrrolidin-1-yl)piperidin-1-yl)carbonyl)amino)pyridin-4-yloxy)-1H-1-indolecarboxamide,
N1-methyl-5-(2-(((4-(piperldin-1-yl)piperidin-1-yl)carbonyl)amino)pyridin-4-yloxy)-1H-1-indolecarboxamide, and
N4-(4-(1-(methylamino)carbonyl-1H-5-indolyl)oxy-2-pyridyl)-4-morpholinecarboxarnide.
The compounds of Formula (XXVI) can be prepared in a known manner, for example as described in International Publication No. WO2004/020434.

In the present invention, the VEGF receptor kinase inhibitor may include, for example:

(1) N-(4-bromo-2-fluorophenyl)-6-methoxy-7-[2-(1H-1,2,3-triazol-1-yl)ethoxy]quinazoline-4-amine (hereinafter also referred to as "ZD4190"; Cancer Research., 60, 970-975, 2000, Journal of Medicinal Chemistry., 42: 5369-5389, 1999),
(2) N-(4-bromo-2-fluorophenyl)-6-methoxy-7-[(1-methylpiperidin-4-yl)methoxy]quinazoline-4-amine (hereinafter also referred to as "ZD6474"; Proc. Am. Assoc. Cancer Res., 42, 583, 2001, Journal of Medicinal Chemistry., 45: 1300-1312, 2002),
(3) 3-[(2,4-dimethylpyrrol-5-yl)methylene]-2-indolinone (hereinafter also referred to as "SU5416"; Cancer Res., 59, 99-106, 1999, Journal of Medicinal Chemistry., 41: 2588-2603, I998, US5792783),
(4) (2)-3-(2,4-dimethyl-5-(2-oxo-1,2-dihydroindol-3-ylidenemethyl)-1H-pyrrol-3-yl)-propionic acid (hereinafter also referred to as "SU6668"; Cancer Res., 60, 4152-4160, 2000, Journal of Medicinal Chemistry., 42: 5120-5130, 1999),
(5) 5-(5-fluoro-2-oxo-1,2-dihydroindol-3-ylidenemethyl)-2,4-dimethyl-1H-pyrrole-3-carboxylic acid (2-diethylaminoethyl)amide (hereinafter also referred to as "SU11248"; Clinical Cancer Research, 9, 327-337, 2003, Journal of

Medicinal Chemistry., 46: 1116-9, 2003),

(6) N,N-dimethylglycine 3-{5,6,7,13-tetrahydro-9-[(1-methylethoxy)methyl]-5-oxo-12H-indeno(2,1-a)pyrrolo(3,4-c) carbazol-12-yl}propyl ester (hereinafter also referred to as "CEP-7055"; Pro. Am. Assoc. Cancer Research, 43, 1080, 2002, Journal of Medicinal Chemistry., 46: 5375-88, 2003),

(7) 3-(4-bromo-2,6-difluoro-benzyloxy)-5-[3-(4-pyrrolidin-1-yl-butyl)-ureido]-isothiazole-4-carboxylic acid amide (hereinafter also referred to as "CP-547,632"; Proc. Am Soc. Clin. Oncology, 21, (Abstract 16), 2002, Cancer Research. 63:7301-9, 2003, WO 99/62890),

(8) N-{2-chloro-4-[(6,7-dimethoxy-4-quinazolinyl)oxy]phenyl}-N'-propylurea (hereinafter also referred to as "KRN633"; Pro. Am. Assoc. Cancer Research, 43, 175, 2002, Molecular Cancer Therapeutics., 3:1639-49, 2004),

(9) 1-(4-chloroanilino)-4-(4-pyridylmethyl)phthalazine (hereinafter also referred to as "PTK787"; Cancer Research, 60, 2179-2189, 2000, Journal of Medicinal Chemistry., 43:2310-23, 2000, WQ98135958) (see Formula (XII) for (1) to (9)),

(10) N-{2-chloro-4-[(6,7-diinethoxy-4-quinolyl)oxy]phenyl}-N'-(S-rr5ethyl-3-isoxazolyl)urea (hereinafter also referred to as "KRN951"; Proceedings of the American Association for Cancer Research, 45, 594, (Abstract 2571), 2004, Proceedings of the American Association for Cancer Research, 45, 595, (Abstract 2575), 2004),

(11) 4-[(4-fluoro-2-methylindol-5-yl)oxy]-6-methoxy-7-[3-(pyrrolidin-1-yl)propoxy]quinazoline (hereinafter also referred to as "AZI32171"; Cancer Research. 65:4389-400, 2005, WOOO/47212), and

(12) 6-[2-(methylcarbamoyl)phenylsulfanyl]-3-E-[2-(pyridin-2-yl)ethenyl]indazole (hereinafter also referred to as "AG013736"; American Journal of Pathology. 165:35-52, 2004) (see Formula (XM) for (10) to (12)).

ZD4190 (Cancer Research., 60, 970-975, 2000, Journal of Medicinal Chemistry., 42: 5369-5389, 1999), ZI36474 (Proc. Am Assoc. Cancer Res., 42, 583, 2001, Journal of Medicinal Chemistry., 45: 1300-1312, 2002), SU5416 (Cancer Res., 59, 99-106, 1999, Journal of Medicinal Chemistry., 41 : 2588-2603, 1998), SU6668 (Cancer Res., 60, 4152-4160, 2000, Journal of IV.ledicinal Chemistry., 42: 5120-5130, 1999), SU11248 (Clinical Cancer Research, 9, 327-337, 2003, Journal of Medicinal Chemistry., 46: 1116-9, 2003), CEP-7055 (Pro. Am. Assoc. Cancer Research, 43, 1080, 2002, Journal of Medicinal Chemistry., 46: 5375-88, 2003), CP-547,632 (Proc. Am. Soc. Clin. Oncology, 21, (Abstract 16), 2002, Cancer Research. 63 ;7301-9, 2003, WO 99/62890), KRN633 (Pro. Am. Assoc- Cancer Research, 43, 175, 2002, Molecular Cancer Therapeutics" 3:1639-49, 2004), PTK787 (ZK222584) (Cancer Research, 60, 2179-2189, 2000, Journal of Medicinal Chemistry., 43:2310-23, 2000), KRN951, AZD2171 (Cancer Research. 65:4389-400, 2005, WOOO/47212) and AG013736 (American Journal of Pathology. 165:35-52, 2004) can be prepared in a known manner, for example as described in the respective documents.

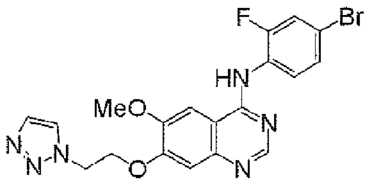

**ZD4190**

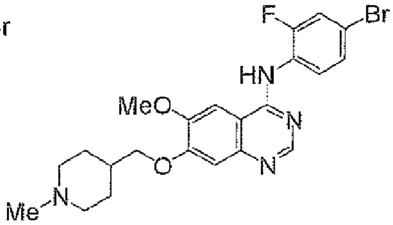

**ZD6474**

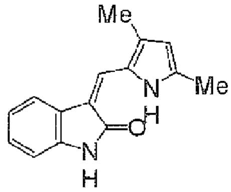

**SU5416**

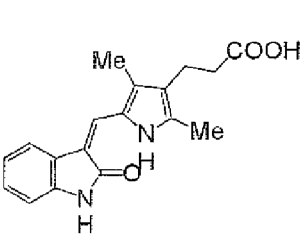

**SU6668**

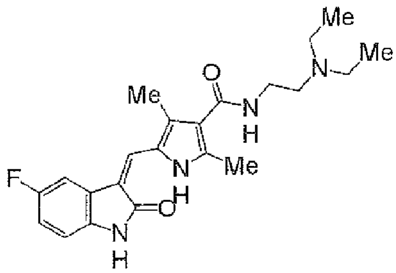

**SU11248**

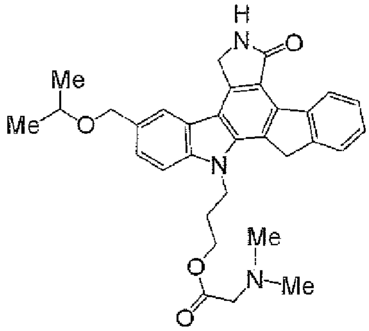

**CEP-7055**

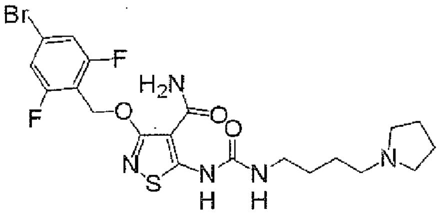

**CP-547,632**

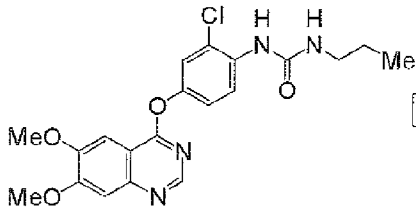

**KRN633**

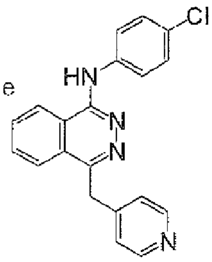

**PTK787**

**(XII)**

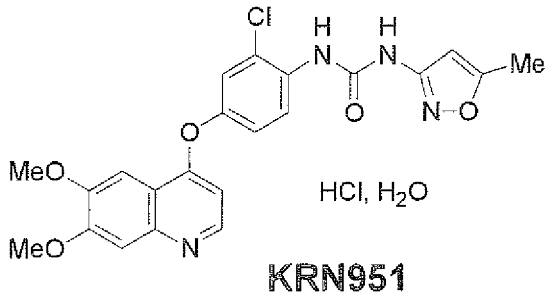

HCl, $H_2O$

**KRN951**

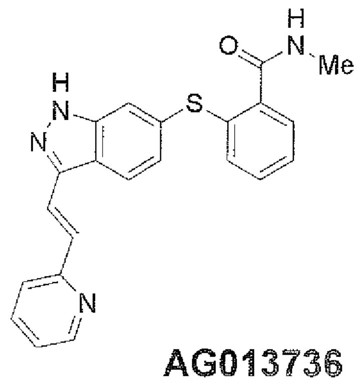

**AG013736**

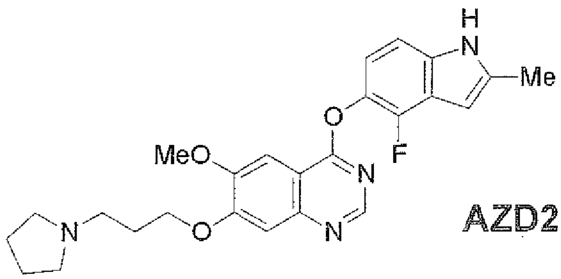

**AZD2171**

**(XIII)**

In the present invention, the VEGF receptor kinase inhibitor may also include:

(13) 5-((Z)-(5-fluoro-2-oxo-1,2-dihydro-3H-indol-3-ylidene)methyl)-N-((2S)-2-hydroxy-3-morpholin-4-ylpropyl)-2,4-dimethyl-1H-pyrrole-3-carboxamide (hereinafter also referred to as "SU14813"; Proceedings of the American Association for Cancer Research, 46, (Abstract 2031), 2005) (see Formula (XVI)):

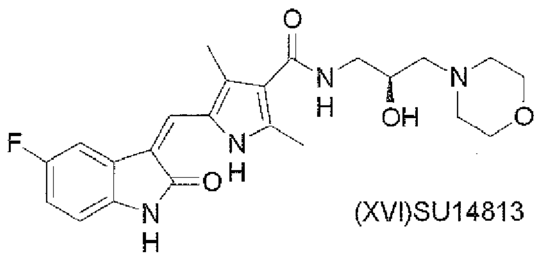

(XVI)SU14813

(14) 3-((quinolin-4-ylmethyl)amino)-N-(4-(trifluoromethoxy)phenyl)thiophene-2-carboxamide (hereinafter also referred to as "OSI930"; Molecular Cancer Therapeutics., 4:1186-1197, 2005) (see Formula (XVH)):

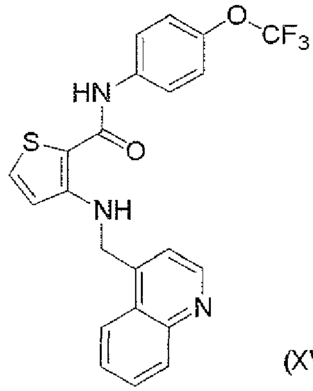

(XVII)OSI930

(15) 6-(2, 6-dichlorophenyl)-8- methyl- 2-phenylamino-8H-pyrido [2, 3-d]pyrimi din- 7 -one (hereinafter also referred to as "TKI-28"; Cancer Biol Ther., 4, 2005) (see Formula (XVIII)):

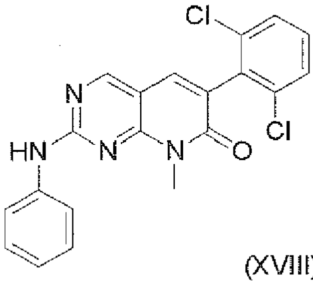

(XVIII)TKI-28

(16) 2-((1,6-dihydro-6-oxo-pyridin-3-ylmethyl)amino)-N-(3-(trifluoromethyl)phenyl)-3-pyridine-carboxamide (hereinafter also referred to as "ABP309"; EORTC-NCI-AACR Symp Mol Targets Cancer Ther., 2, (Abstract 2004) (see Formula (XIX)):

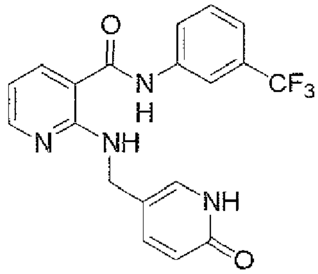

(XIX)ABP309

(17) 4-(4-(4-chloro-phenylamino)-furo[2,3-d]pyridazin-7-yloxymethyl)-pyridine-2-carboxylic acid methylamide, (hereinafter also referred to as "BAY 57-93 52"; International Publication No. WO01/23375) (see Formula (XX)):

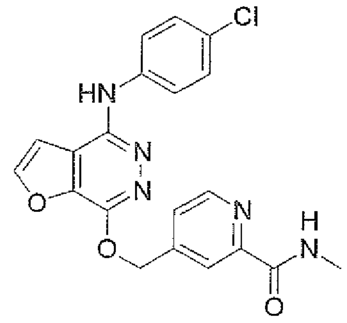

(XX)BAY 57-9352

(18) N-(3 -trifluoromethyl-4-chlorophenyl-N' -(4-(2-methylcarbamoylpyridin-4-yl)oxyphenyl)urea (hereinafter also referred to as "BAY 43-9006"; Cancer Research., 64, 7099-7109, 2004, Organic Process Res Dev, 6, 777-8I, 2002) (see Formula (XXI)):

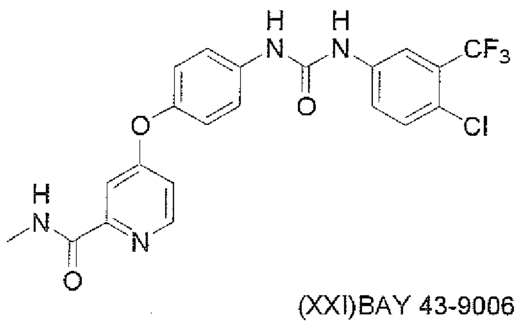

(XXI)BAY 43-9006

(19) 4-amino-5-fluoro-3-(6-(4-methyl-piperazin-I-yl)-1H-benzimidazol-2-yl)-1H-quinolin-2-one (hereinafter also referred to as "CHIR258"; Clinical Cancer Research., 11, 3633-3641, 2005) (see Formula (XXII));

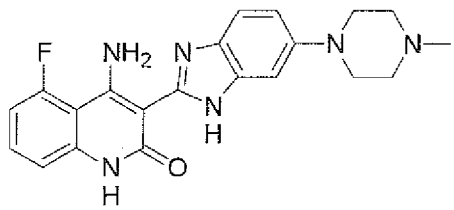

(XXII)CHIR258

(20) 4-( 4-(1-amino-1-methyl-ethyl)-phenyl)-2-( 4-(2-morpholin-4-yl-ethyl)-phenylamino)-pyrimidine-5-carbonitrile (hereinafter also referred to as "JNJ17029259"; Molecular Pharmacology., 66, 635-647, 2004) (see Formula (XXm)):

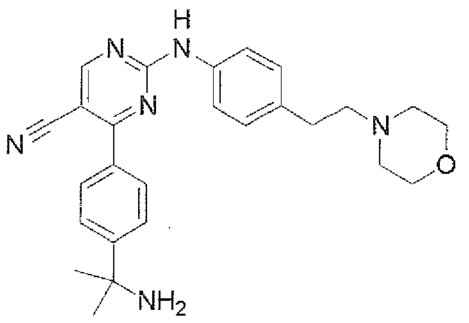

(XXIII)JNJ17029259

(21) [6-[4-[(4-ethylpiperazin-1 -yl)methyl]phenyl]-7H-pyrrolo [2,3 -d]pyrimid:n-4-yl]-((R)-1-phenylethyl)arnine (hereinafter also referred to as "AEE-788"; Cancer Research., 64, 4931-4941, 2004., Cancer Research., 64, 7977-7984, 2004) (see Formula (NXVII)):

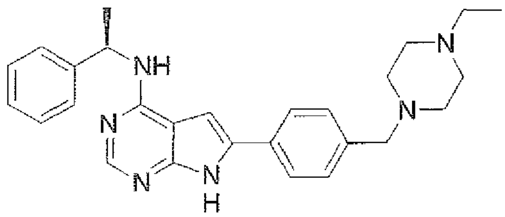

(XXVII)AEE-788

(22) 9-(l-methylethoxy)methyl-12-(3-hydroxypropyl)-6H,7H,13H-indeno[2,l-a]pyrrolo[3,4-c]carbazol-5-one (hereinafter also referred to as "CEP-5214"; Journal of Medicinal Chemistry., 46, 5375-5388, 2003., Cancer Research., 63, 5978-5991, 2003) (see Formula (XXVIII)):

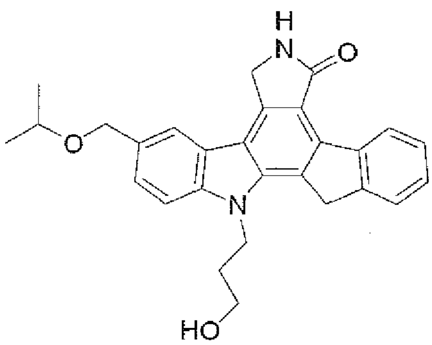

(XXVIII)CEP-5214

(23) N-(2, 4-difluorophenyl)-N' -{ 4-[(6, 7-dimethoxy-4-quinolyl)-oxy ]-2-fluorophenyl} urea (hereinafter also referred to as "KI-8751"; Journal of Medicinal Chemistry., 48, 1359-1366, 2005) (see Formula (XXDo)

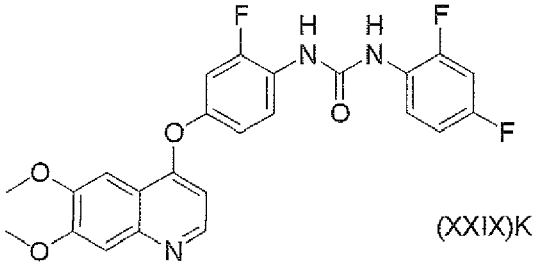

(XXIX)KI-8751

(24) 5-[-N-methyl-N-(4-octadecyloxyphenyl)acetyl]anfno-2-methylthiobenzoic acid (hereinafter also referred to as "VGA-1155"; Anticancer Research., 24, 3009-3017, 2004) (see Formula (XXX):

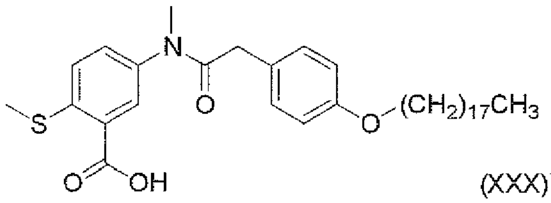

(XXX)VGA-1155

(25) N-[4-(3-amino-1H-indazol-4-yl)phenyl]-N'-(2-fluoro-5-methylphenyl)urea (hereinafter also referred to as "ABT-869"; Proceedings of the American Association for Cancer Research., 46, 1407, (Abstract 5981), 2005) (see Formula (XXXI))-,

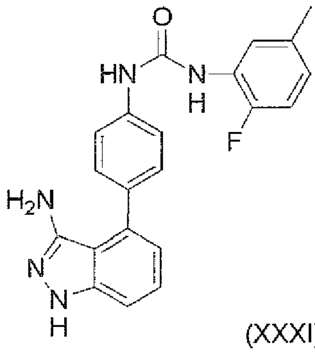

(XXXI)ABT-869

(26) 2-methyl-6-[2-(1-methyl-1H-imidazol-2-yl)-thieno[3,2-b]pyidin-7-yloxy]-benzo[b]thiophene-'3-carboxylic acid methylamide (hereinafter also referred to as "AG-028262"; WO03/06462, US20041009965) (see Formula (XXXTI)):

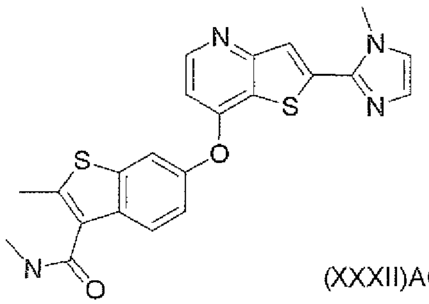

(XXXII)AG-028262

(27) (R)-1-(4-(4-fluoro-2-methyl-1H-indol-5-yloxy)-5-methylpyrrolo[1,2-f][1,2,4]triazin-6-yloxy)propan-2-ol (hereinafter also referred to as "BMS-540215"; Proceedings of the American Association for Cancer Research., 46, (Abstract

3033), 2005) (see Formula (XXXIII)):

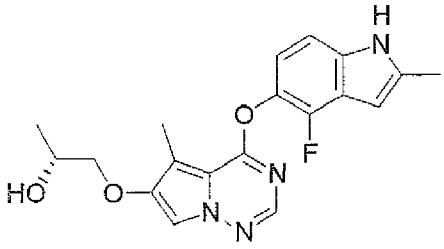

(XXXIII)BMS-540215

and

(28) (S)-((R)-1-(4-(4-fluoro-2-methyl-1H-indol-5-yloxy)-5-methylpyrrolo[1,2-f][1,2,4]triazin-6-yloxy)propan-2-ol) 2-aminopropanonate (hereinafter also referred to as "BMS-582664"; Proceedings of the American Association for Cancer Research., 46, (Abstract 3033), 2005) (see Formula (XXXIV)):

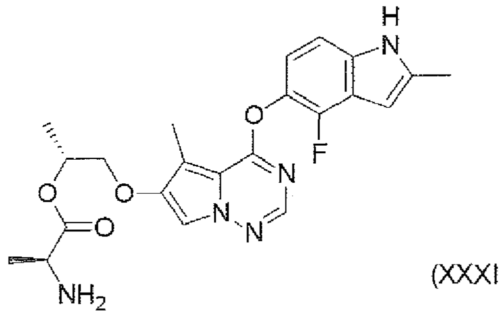

(XXXIV)BMS-582664

SU14813, OSI930, TKI-28, ABP309, BAY 57-9352, BAY 43-9006, CIDR258, JNJ17029259, AEE-788, CEP-5214, KI-8751, VGA-1155, ABT-869, AG-028262, BMS-540215 and BMS-582664 can be prepared in a known manner, for example as described in the respective documents.

In the present invention, the VEGF receptor kinase inhibitor may also include, for example, BIBF1120 fIV00 1/2708 1 ), ZK304709 (Proceedings of the American Association for Cancer Research, 46, (Abstract 5842), 2005), Exel 7647 (EORTC-NCI-AACR Symp Mol Targets Cancer Ther., (Abstract 134), 2004), AMG706 (EORTC-NCI-AACR Symp Mol Targets Cancer Ther., 2, (Abstract 151), 2004), GW-654652 (Blood.,103, 3474-3479, 2004, Proceedings of the American Association for Cancer Research, 44, 9, (Abstract 39), 2003, Proceedings of the American Association for Cancer Research, 44, 9, (Abstract 40), 2003), and GW 786034 (Proc. Am. Soc. Clin. Oncology, (Abstract 3054), 2004). EIBF 1120, ZK304709, Exe17647, AMG706, fJW 654652 and GW-7δ603 can be prepared in a known manner.

In the present invention, the VEGF receptor kinase inhibitor may further include, for example, anti-VEGF receptor antibodies. Anti-VEGF receptor antibodies are those having affinity for the VEGF receptor or a fragment thereof Such anti-VEGF receptor antibodies are preferably neutralizing antibodies that recognize and bind to the VEGF receptor to inhibit the vascular endothelial cell growth activity of VEGF. The anti-$VE_GF$ receptor antibodies may be either polyclonal or monoclonal. Also, there is no particularly limitation on the isotypes of the antibodies. The anti-VEGF receptor antibodies may also be either antibody fragments or single chain antibodies (see the section about anti-VEGF antibodies and anti-FGF antibodies described later).

The anti-VEGF receptor antibodies may preferably include 2C3 antibody (US6524583, US6676941), IMC-1121b (US6811779), IMC-18F1 (Proceedings of the American Association for Cancer Research, 45, 694, (Abstract 3005), 2004), fMC-1C1I (US5747651), and IMC-2C6 (Proceedings of the American Association for Cancer Research, 44, 1479, (Abstract 6454), 2003). These 2C3 antibody, IMC-1121b, IMC-18F1, IMC-1C11 I and IMC-2C6 can be prepared in a known manner, for example as described in the respective documents.

(2) FGF receptor kinase inhibitors

In the present invention, preferred FGF receptor kinase inhibitors are 1-[2-amino-6-(3,5-dimethoxyphenyl)-pyrido(2,3-d)pyrimidin-7-yl]-3-tert-butylurea (hereinafter also referred to as "PD166866"; J. M. C., 40, 2296-2303, 1997) and 1-tert-butyl-3-[2-(3-dimethylamino)propylamino-6-(3,5-dimethoxyphenyl)-pyrido(2,3-d)pyrimidin-7-yl]urea (hereinafter also referred to as "PD173074"; EMBO J., 17, 5896-5904, 1998).

PD166866 (J. M. C., 40, 2296-2303, 1997) and PD173074 (EMBO J., 17, 5596-5904, 1998) can be prepared in a known manner.

Compounds serving as VEGF receptor kinase inhibitors and compounds serving as FGF receptor kinase inhibitors may form pharmacologically acceptable salts with acids or bases. The above receptor kinase inhibitors of the present invention also encompass these pharmacologically acceptable salts. Examples of salts with acids include inorganic acid salts (e.g., hydrochloride salt, hydrobromide salt, sulfate salt, phosphate salt), as well as salts with organic acids such as formic acid, acetic acid, lactic acid, succinic acid, fumaric acid, maleic acid, citric acid, tartaric acid, benzoic acid, methanesulfonic acid, benzenesulfonic acid, p-toluenesulfonic acid and trifluoroacetic acid. Likewise, examples of salts with bases include alkali metal salts (e.g., sodium salt, potassium salt), alkaline earth metal salts (e.g., calcium salt, magnesium salt), salts with organic bases such as trimethylamine, triethylamine, pyridine, picoline, dicyclohexylamine, N,N'-dibenzylethylenediamine, arginine and lysine (organic amine salts), as well as ammonium salts.

Alternatively, compounds serving as VEGF receptor kinase inhibitors and compounds serving as FGF receptor kinase inhibitors may be in anhydride form or may form solvates such as hydrates. Solvates may be either hydrates or anhydrates, preferably hydrates. Solvents available for use include water, alcohols (e.g., methanol, ethanol, n-propanol) and dimethylformamide.

In a case where these compounds are present in solvate form and/or have optical isomers, the VEGF receptor kinase inhibitors and FGF receptor kinase inhibitors according to the present invention also encompass their solvates and/or optical isomers. The above receptor kinase inhibitors according to the present invention further encompass VEGF receptor kinase inhibitors and FGF receptor kinase inhibitors that are metabolized in vivo by oxidation, reduction, hydrolysis, conjugation, etc. Furthermore, the above receptor kinase inhibitors according to the present invention encompass compounds that produce VEGF receptor kinase inhibitors and FGF receptor kinase inhibitors when metabolized *in vivo* by oxidation, reduction, hydrolysis, etc.

(3) Anti-VEGF antibodies and anti-FGF antibodies

In the present invention, the anti-VEGF antibody is preferably a neutralizing antibody that recognizes and binds to VEGF to inhibit the vascular endothelial cell growth activity of VEGF.

Likewise, in the present invention, the anti-FGF antibody is preferably a neutralizing antibody that recognizes and binds to FGF to inhibit the fibroblast growth activity of FGF.

In the present invention, the anti-VEGF antibodies and anti-FGF antibodies may be either polyclonal or monoclonal. Also, there is no particularly limitation on the isotypes of these antibodies; the antibodies may have any isotype, for example, IgG ($IgG_1$, $IgG_2$, $IgG_3$, $IgG_4$), IgM, IgA ($IgA_1$, $IgA_2$), IgD or IgE.

Polyclonal and monoclonal antibodies can be prepared in a manner well known to those skilled in the art (Antibodies: A Laboratory Manual, E. Harlow and D. Lane, ed., Cold Spring Harbor Laboratory (Cold Spring Harbor, NY, 1988)).

Polyclonal antibodies can be obtained, for example, by administering an antigen to a mammal (e.g., mouse, rabbit, rat), collecting the blood from the mammal, and separating and purifying antibody molecules from the collected blood. Procedures for immunization are known to those skilled in the art and can be accomplished, e.g., by one or more administrations of an antigen. Although an antigen (including a part or all of VEGF or FGF) may be used by being dissolved in an appropriate buffer, for example, containing a commonly-used adjuvant such as complete Freund's adjuvant or aluminum hydroxide, the use of an adjuvant may be eliminated depending on the route of administration or conditions, etc.

After 1 to 2 months from the last immunization, the blood may be collected from the mammal and then separated and purified in a routine manner, e.g., by centrifugation, precipitation with ammonium sulfate or polyethylene glycol, or various chromatographic techniques to obtain polyclonal antibodies in the form of a polyclonal antiserum.

For monoclonal antibody production, for example, the hybridoma method may be used. As in the case of polyclonal antibody production, a mammal is first immunized. When the appropriate number of days have passed after the immunization, a part of the blood is preferably sampled and measured for its antibody titer in a known manner, e.g., by the ELISA method.

The spleen is then isolated from the sensitized and immunized animal to obtain B cells. The B cells are then fused with myeloma cells in a routine manner to create antibody-producing hybridomas. There is no particular limitation on the type of myeloma cells to be used and it is possible to use known types of myeloma cells. For cell fusion, any

method known in the art, such as the Sendai virus method, the polyethylene glycol method or the protoplast method, may be selected and used. The resulting hybridomas are cultured in a routine manner in HAT medium (i.e., a medium containing hypoxanthine, aminopterin and thymidine) for an appropriate period of time to effect hybridoma selection. After screening to select a desired antibody-producing hybridoma, the hybridoma is cloned.

For screening, it is possible to use any method known for antibody detection, such as the ELISA method or the radioimmunoassay method. For cloning, any method known in the art may be used, including the limiting dilution method and the FACS method. The resulting hybridoma is cultured in an appropriate culture medium or is administered to a mammal (e.g., mouse peritoneal cavity) with which the hybridoma is compatible. From the culture solution or peritoneal fluid thus obtained, a desired monoclonal antibody can be isolated and purified by salting-out, ion exchange chromatography gel filtration, affinity chromatography, etc.

Fragments and V region single chain antibodies of the above antibodies may also be used in the present invention. Such antibody fragments mean partial regions of the above polyclonal or monoclonal antibodies. Specific examples include $F(ab')_2$, Fab', Fab, Fv (variable fragment of antibody), sFv, dsFv (disulphide stabilized Fv) or dAb (single domain antibody). Moreover, the antibodies used in the present invention may also be humanized antibodies or human antibodies. Human antibodies can be created in the same manner as used for standard monoclonal antibody production, except for using a mammal whose immune system is replaced by the human immune system.

3. Pharmaceutical compositions, kits, method for cancer prevention/treatment, and method for angiogenesis inhibition

The present invention relates to pharmaceutical compositions, kits, a method for cancer prevention/treatment, and a method for angiogenesis inhibition, which are characterized in that a sulfonamide-including compound is combined with an angiogenesis inhibitor.

In the present invention, the sulfonamide-including compound is as described in "1. Sulfonamide-including compounds" and, for example, may be at least one compound selected from (1) a compound of Formula (I), preferably LY186641 or LY295501, (2) a compound of Formula (II), preferably LY-ASAP, (3) LY573636 (Formula (III))(4) CQS (Formula (IV)), and (5) Formula (XIV), preferably E7070 or E7820. The sulfonamide-including compound is more preferably at least one compound selected from LY295501 and LY573636, and particularly preferably a sodium salt of LY573636.

Alternatively, in the present invention, the sulfonamide-including compound is preferably E7070 or E7820.

In the present invention, the angiogenesis inhibitor is as described in "2. Angiogenesis inhibitors" and, for example, may be (a) a VEGF receptor kinase inhibitor, (b) an anti-VEGF antibody, (c) a FGF receptor kinase inhibitor or (d) an anti-FGF antibody.

In the present invention, the VEGF receptor kinase inhibitor (a) may be, for example, a compound represented by at least one selected from Formula (XXIV), Formula (XII), Formula (XIII), Formulae (XVI) to (XXIII) and Formulae (XXVII) to (XXXIV). In the present invention, the VEGF receptor kinase inhibitor (a) is preferably a compound of Formula (XXV) or (XXVI), more preferably a compound of Formula (V) or (VI), even more preferably 4-(3-chloro-4-(cyclopropylaminocarbonyl)aminophenoxy)-7-methoxy-6-quinolinecarboxamide falling within the scope of Formula (V), and particularly preferably a methanesulfonate salt of 4-(3-chloro-4-(cyclopropylaminocarbonyl)aminophenoxy)-7-methoxy-6-quinolinecarboxamide. Alternatively, in the present invention, the VEGF receptor kinase inhibitor (a) is preferably a compound represented by at least one selected from Formula (XII), Formula (XIII), Formulae (XVI) to (XXIII) and Formulae (XXVII) to (XXXIV), and more preferably at least one compound selected from KRN951 (Formula (XIII)), AZD2171 (Formula (XIII)), AG013736 (Formula (XIII)), SU14813 (Formula (XVI)), OSI930 (Formula (XVII)), TKI-28 (Formula (XVIII)), ABP309 (Formula (XIX)), BAY 57-9352 (Formula (XX)), BAY 43-9006 (Formula (XXI)), CHIR258 (Formula (XXII)), JNJ17029259 (Formula (XXIII)), AEE-788 (Formula (XXVII), CEP-5214 (Formula (XXVIII)), KI-8751 (Formula (XXIX)), VGA-1155 (Formula (XXX)), ABT-869 (Formula (XXXI)), AG-028262 (Formula (XXXII)), BMS-540215 (Formula (XXXIII)) and BMS-582664 (Formula (XXXIV)). Alternatively, in the present invention, the VEGF receptor kinase inhibitor (a) may be, for example, an anti-VEGF receptor antibody.

In the present invention, the FGF receptor kinase inhibitor (c) is preferably PD166866 or PD173074.

In the present invention, the above compounds and angiogenesis inhibitors also encompass their pharmacologically acceptable salts or solvates (e.g., hydrates) thereof

In the present invention, the sulfonamide compound and the angiogenesis inhibitor may be used in any combination of the above compounds and substances. Such a combination is not limited in any way and may include:

(i) a combination of a compound of Formula (I), a compound of Formula (II), LY573636, CQS or E7070 with an angiogenesis inhibitor;
(ii) a combination of a compound of Formula (XIV) with a VEGF receptor kinase inhibitor; and
(iii) a combination of a compound of Formula (XIV) with an anti-VEGF antibody.

The pharmaceutical composition of the present invention comprises a sulfonamide-including compound in combination with an angiogenesis inhibitor. The pharmaceutical composition of the present invention is useful as a pharmaceutical composition for cancer treatment and as a pharmaceutical composition for angiogenesis inhibition.

Alternatively, the pharmaceutical composition of the present invention is also provided as another embodiment, i.e., a pharmaceutical composition comprising a sulfonamide-including compound, which is administered together with an angiogenesis inhibitor to a patient. The sulfonamide-including compound and the angiogenesis inhibitor may be administered simultaneously or separately. The term "simultaneously" means that these components are administered at the same timing in a single administration schedule. In this case, it is not necessary to use completely the same hour and minute for administration. The term "separately" means that these components are administered at different timings in a single administration schedule.

In the present invention, the phrase "in combination with" means a combination of compounds for combined use, and encompasses both modes in which separate compounds are administered in combination and as a mixture.

Likewise, the kit of the present invention comprises a formulation comprising a sulfonamide-including compound and a formulation comprising an angiogenesis inhibitor. The formulations contained in the kit of the present invention may be of any dosage form as long as they contain a sulfonamide-including compound or an angiogenesis inhibitor. The kit of the present invention is useful as a kit for angiogenesis inhibition or as a kit for cancer treatment.

The pharmaceutical composition and/or kit of the present invention may further be combined with one or more additional anti-cancer agents. Such additional anti-cancer agents are not limited in any way as long as they are formulations having an anti-cancer effect. Such additional anti-cancer agents include, for example, irinotecan hydrochloride (CPT-11), oxaliplatin, 5-fluorouracil (5-FU), docetaxel (Taxotere®), gemcitabine hydrochloride (Gemzar®), calcium folinate (leucovorin), gefitinib (Iressa®), erlotinib (Tarceva®), and cetuximab (Erbitux®). The above additional anti-cancer agents are particularly preferably irinotecan hydrochloride, oxaliplatin, 5-fluorouracil, calcium folinate, gefitinib, erlotinib and cetuximab in a case where the cancer type targeted by cancer therapeutic agent is colon cancer. Likewise, the above additional anti-cancer agents are particularly preferably gemcitabine hydrochloride, gefitinib, erlotinib and cetuximab for pancreas cancer, and gefitinib, erlotinib and cetuximab for renal cancer.

The pharmaceutical composition and/or kit of the present invention can be used as an angiogenesis inhibitory agent or as a cancer therapeutic agent.

In the present invention, the term "cancer therapeutic agent" is used to mean an anti-tumor agent, a cancer prognosis improver, a cancer recurrence inhibitor, a cancer metastasis inhibitor, etc.

The effect of cancer treatment may be confirmed by findings such as roentgenogram or CT analysis or histopathological diagnosis of biopsy specimens, or alternatively, by tumor marker levels.

The pharmaceutical composition and/or kit of the present invention may be administered to mammals (e.g., human, rat, rabbit, sheep, pig, cattle, cat, dog, monkey).

The cancer type targeted by cancer therapeutic agent is not limited in any way, and examples include at least one selected from the group consisting of brain tumor, cervical cancer, esophageal cancer, tongue cancer, lung cancer, breast cancer, pancreas cancer, gastric cancer, large intestine cancer, small intestine cancer, duodenal cancer, colon cancer, rectal cancer, bladder cancer, renal cancer, liver cancer, prostate cancer, uterine cancer, ovarian cancer, thyroid gland cancer, gallbladder cancer, pharyngeal cancer, sarcoma (e.g., osteosarcoma, myosarcoma, fibrosarcoma) and melanoma.

In the case of using the pharmaceutical composition and/or kit of the present invention, administration may be conducted orally or parenterally.

In the case of using the pharmaceutical composition and/or kit of the present invention, the dose of the sulfonamide-including compound will vary depending on the severity of symptoms, the age, sex, body weight and differences in susceptibility of individual patients, the method of administration, the duration of administration, the interval of administration, the nature, pharmacy and type of individual pharmaceutical formulations, as well as the type of active ingredient, etc. Without being particularly limited, the daily dose for adults (body weight: 60 Kg) is usually 10 to 6000 mg, preferably 50 to 4000 mg, and more preferably 100 to 3000 mg, which may usually be given once to three times a day.

In the case of using the pharmaceutical composition and/or kit of the present invention, without being particularly limited, the daily adult dose of the VEGF receptor kinase inhibitor is usually 10 to 6000 mg, preferably 50 to 4000 mg, and more preferably 50 to 2000 mg, which may usually be given once to three times a day.

Likewise, in the case of using the pharmaceutical composition and/or kit of the present invention, without being particularly limited, the daily adult dose of the FGF receptor kinase inhibitor is usually 10 to 6000 mg, preferably 50 to 4000 mg, and more preferably 50 to 2000 mg, which may usually be given once to three times a day.

In the case of using the pharmaceutical composition and/or kit of the present invention, without being particularly limited, the dose of the anti-VEGF antibody is usually 1 to 6000 mg, preferably 10 to 2000 mg, and more preferably 10 to 1000 mg, which may usually be given once to three times a day or a week.

In the case of using the pharmaceutical composition and/or kit of the present invention, without being particularly limited, the dose of the anti-FGF antibody is usually 1 to 6000 mg, preferably 10 to 2000 mg, and more preferably 10 to

1000 mg, which may usually be given once to three times a day or a week.

The amount of the sulfonamide-including compound to be used is not limited in any way and will vary depending on individual combinations with the VEGF receptor kinase inhibitor, anti-VEGF antibody, FGF receptor kinase inhibitor or anti-FGF antibody. For example, it is about a 0.01- to 100-fold excess (by weight), more preferably about a 0.1- to 10-fold excess (by weight), of the VEGF receptor kinase inhibitor, anti-VEGF antibody, FGF receptor kinase inhibitor or anti-FGF antibody.

The pharmaceutical composition of the present invention may be formulated into various dosage forms, for example, oral solid formulations or parenteral formulations including injections, suppositories, ointments and cataplasm.

Likewise, the sulfonamide-including compound and the angiogenesis inhibitor contained in the kit of the present invention may each be formulated into various dosage forms, for example, oral solid formulations or parenteral formulations including injections, suppositories, ointments and cataplasm.

To prepare oral solid formulations, these active ingredients may be supplemented with excipients and, if necessary, with binders, disintegrating agents, lubricants, coloring agents, flavoring agents and the like, followed by formulation in a routine manner into tablets, coated tablets, granules, fine granules, powders, capsules, etc. It is also possible to prepare oral non-solid formulations such as syrups, when needed.

Examples of excipients include lactose, corn starch, sucrose, glucose, sorbit, crystalline cellulose, and silicon dioxide. Examples of binders include polyvinyl alcohol, ethylcellulose, methylcellulose, gum arabic, hydroxypropylcellulose, and hydroxypropylmethylcellulose. Examples of lubricants include magnesium stearate, talc, and silica. Examples of coloring agents include those permitted for use in pharmaceutical preparations. Examples of flavoring agents include cocoa powder, menthol, aromatic acid, peppermint oil, borneol, and cinnamon powder. Of course, tablets and granules may further be coated appropriately with sugar coating, gelatin coating or other coatings, if necessary.

To prepare injections, these active ingredients may be supplemented, if necessary, with pH adjustors, buffers, suspending agents, solvent aids, stabilizing agents, isotonizing agents, preservatives and the like, followed by formulation in a routine manner into intravenous injections, subcutaneous injections, intramuscular injections, or intravenous drip infusions. In this case, it is also possible to prepare lyophilized formulations in a routine manner, if necessary. Examples of suspending agents may include methylcellulose, Polysorbate 80, hydroxyethylcellulose, gum arabic, powdered tragacanth, sodium carboxymethylcellulose, and polyoxyethylenesorbitan monolaurate.

Examples of solvent aids may include polyoxyethylene hydrogenated castor oil, Polysorbate 80, nicotinamide, polyoxyethylenesorbitan monolaurate, Macrogol, and an ethyl ester of castor oil fatty acid.

Likewise, examples of stabilizing agents may include sodium sulfite, and sodium metasulfite, Examples of preservatives may include methyl parahydroxybenzoate, ethyl parahydroxybenzoate, sorbic acid, phenol, cresol, and chlorocresol.

In the kit of the present invention, the formulation comprising a sulfonamide-including compound and the formulation comprising an angiogenesis inhibitor may be mixed together or may be included in separate containers and packaged in a single package. These formulations may be administered either simultaneously or sequentially.

The kit of the present invention can be used as a kit for angiogenesis inhibition or as a kit for cancer treatment and further comprises a packaging container, an instruction manual, an package insert and the like, in addition to the formulation comprising a sulfonamide-including compound and the formulation comprising an angiogenesis inhibitor. These packaging container, instruction manual and package insert may describe a combination for combined use of the formulations or may describe a dosage regimen for administration of separate formulations in combination or as a mixture, etc. Such a dosage regimen may be described by reference to the above description.

In another embodiment, the kit of the present invention may comprise (a) at least one selected from the group consisting of a packaging container, an instruction manual and an package insert, each of which describes the combined use of a sulfonamide-including compound and an angiogenesis inhibitor, and (b) a pharmaceutical composition comprising the sulfonamide-including compound. The kit is useful as a kit for cancer treatment or as a kit for angiogenesis inhibition. The above pharmaceutical composition comprising the sulfonamide-including compound is useful as a pharmaceutical composition for cancer treatment and as a pharmaceutical composition for angiogenesis inhibition. These packaging container, instruction manual and package insert may describe the combined use of the sulfonamide-including compound and the angiogenesis inhibitor or may describe a dosage regimen for administration of separate substances in combination or as a mixture, etc. Such a dosage regimen may be determined by reference to the above description about the pharmaceutical composition/kit.

Moreover, the present invention also includes the use of a sulfonamide-including compound for the manufacture of a pharmaceutical composition in combination with an angiogenesis inhibitor.

In the use of the present invention, the above pharmaceutical composition is useful as a pharmaceutical composition for cancer treatment and as a pharmaceutical composition for angiogenesis inhibition.

Moreover, the present invention also includes a method for preventing or treating cancer or a method for inhibiting angiogenesis, which comprises simultaneously or separately administering a sulfonamide-including compound and an angiogenesis inhibitor to a patient. In the method of the present invention for preventing or treating cancer, there

is no particular limitation on the route and method of administration of the sulfonamide-including compound and the angiogenesis inhibitor. With respect to the route and method of administration, reference may be made to the above description about the pharmaceutical composition of the present invention.

The present invention will be further described by way of the following illustrative examples, which are not intended to limit the scope of the invention.

Example 1 DNA microarray analysis

1) Cell culturing, compound treatment and RNA extraction

With the aim of studying compound-induced changes in gene expression by DNA microarray analysis, the human colon cancer cell line HCT116 (American Type Culture Collection, Manassas, VA, U.S.A.) and the human leukemia cell line MOLT-4 (American Type Culture Collection, Manassas, VA, U.S.A.) were cultured in RPMI-1640 medium supplemented with 10% fetal bovine serum, 100 units/ml penicillin and 100 μg/ml streptomycin. Hereafter, culturing and compound treatment were carried out in an incubator adjusted to 5% $CO_2$ and 37°C. HCT116 cells and MOLT-4 cells were seeded at a density of 2.0 x $10^6$ cells into cell culture dishes of 10 cm diameter and cultured for 24 hours, followed by compound treatment as shown below.

With respect to HCT116 cells, the following 12 compounds were evaluated: E7820 (0.8 μM), E7070 (0.8 μM), LY295501 (30 μM),CQS (8 μM),adriamycin (0.2 μM), daunomycin (0.2 μM), ICRF154 (80 μM), ICRF159 (80 μM), kenpaullone (10 μM), alsterpullone (10 μM), trichostatin A (0.1 μM) and rapamycin (80 μM). On the other hand, with respect to MOLT-4 cells, E7070 (0.8 μM)was evaluated. These compounds are all known compounds, among which adriamycin and daunomycin are known as DNA topoisomerase II inhibitors of DNA intercalation type, ICRF154 and ICRF159 are known as DNA topoisomerase II inhibitors of catalytic type, kenpaullone and alsterpullone are known as cyclin-dependent kinases (CDKs) inhibitors, trichostatin A is known as a histone deacetylase inhibitor, and rapamycin is known as a mTOR/FRAP inhibitor. The treatment concentration of each compound was set to a concentration three to five times higher than the 50% growth inhibitory concentration of each compound against HCT116 cells (based on 3-day cell growth inhibition assays using WST-8). After treatment for 24 hours at the set concentration in parentheses following the name of each compound, the cells were collected. Moreover, the cells cultured for 24 hours in the absence of these compounds were also collected.

Extraction of total RNA from the collected cells was performed using a TRIZOL reagent (Invitrogen Corporation) according to the instructions attached thereto.

2) Gene expression analysis by DNA microarrays

The resulting RNA was dissolved in 100 μl of diethyl pyrocarbonate (DEPC)-treated sterilized water and further purified using an RNeasy column (QIAGEN), followed by synthesis of double-stranded cDNA using a SuperScript Choice System (Invitrogen Corporation) and a T7-d$(T)_{24}$ primer.

First, 10 μg of RNA was supplemented with 5 μMT7-d$(T)_{24}$primer, 1x First strand buffer, 10 mM DTT, 500 μM dNTP mix and 20 units/μl SuperScript II Reverse Transcriptase, and then reacted at 42°C for 1 hour to synthesize single-stranded DNA. Subsequently, 1x Second strand buffer, 200 μM dNTP mix, 67 U/ml DNA ligase, 270 U/ml DNA polymerase I and 13 U/ml RNase H were added and reacted at 16°C for 2 hours to synthesize double-stranded cDNA. Further, 67 U/ml T4 DNA polymerase I was added and reacted at 16°C for 5 minutes, followed by addition of 10μ1of 0.5 MEDTA to stop the reaction.

The resulting cDNA was purified by phenol/chloroform and labeled with biotinylated UTP and CTP using an RNA Transcript Labeling Kit (Enzo Diagnostics) according to the instructions attached thereto. The reaction product was purified on an RNeasy column and then heated in 200 mM trisacetic acid pH 8.1, 150 mM magnesium acetate, 50 mM potassium acetate at 94°C for 35 minutes to fragment cRNA.

The fragmented cRNA was hybridized to a GeneChip (Affymetrix) Human Focus array in 100 mM MES, 1 M sodium salt, 20 mM EDTA, 0.01% Tween 20 at 45°C for 16 hours. After hybridization, the GeneChip was washed and stained according to the protocol Midi_euk2 attached to an Affymetrix fluidics station. The staining was accomplished by using streptavidin-phycoerythrin and a biotinylated anti-streptavidin goat antibody. The stained GeneChip was scanned with an HP argon ion laser confocal microscope (Hewlett Packard) and measured for fluorescence intensity. The measurement was performed with an excitation wavelength of 488 nm and an emission wavelength of 570 nm.

Quantitative data analyses were all conducted by using GeneChip software (Affymetrix) and Gene Spring (Silicongenetics). In the case of using GeneChip software to evaluate compound-induced changes in gene expression, if there is a 2-fold or more difference in the quantified value of RNA between two groups, i.e., compound-treated and untreated groups, it is determined that the expression of a corresponding gene has significantly "increased" or "decreased." In the case of using Gene Spring to evaluate the similarity of changes in gene expression induced by each

compound, hierarchical clustering analysis was performed based on changes in the expression of all genes mounted on the Human Focus Arrays.

Figure 1 shows the results of hierarchical clustering analysis performed on HCT116 cells.

As a result of the analysis, adriamycin and daunomycin, ICRF154 and ICRF159, as well as Kenpaullone and alsterpullone, each pair of which shared the same mechanism of action, were found to cause genetic changes similar to each other (Figure 1). It was therefore confirmed that compounds having the same mechanism of action mutually caused similar genetic changes.

E7070, E7820, LY295501 and CQS were found to cause similar genetic changes (Figure 1). Thus, this analysis strongly suggested that E7070, E7820, LY295501 and CQS appeared to have the same or similar mechanism of action and hence produced the same or similar genetic changes and effects.

Example 2 DNA microarray analysis

In this example, HCT 116 cells were used to study changes in gene expression induced by treatment with the following 12 compounds: E7820 (0.16 μM), E7070 (0.26 μM), LY186641 (59 μM), LY295501 (24 μM), LY573636 (9.6 μM), CQS (4.0 μM), MST16 (100 μM), etoposide (3.6 μM), ethoxzolamide (410 μM), capsaicin (280 μM), trichostatin A (0.16 μM) and kenpaullone (7.1 μM)

These compounds are all known compounds, among which MST16 is known as a DNA topoisomerase II inhibitor of catalytic type, etoposide is known as a DNA topoisomerase II inhibitor inducing cleavable complex formation, ethoxzolamide is known as a carbonic anhydrase inhibitor, capsaicin is known as a tumor-specific plasma membrane NADH oxidase inhibitor, trichostatin A is known as a histone deacetylase inhibitor, and kenpaullone is known as a cyclin-dependent kinases (CDKs) inhibitor.

The treatment concentration of each compound was set to a concentration twice higher than the 50% growth inhibitory concentration of each compound against HCT116 cells (based on 3-day cell growth inhibition assays using MTT). After treatment for 24 hours at the set concentration in parentheses following the name of each compound, the cells were collected. Moreover, the cells cultured for 24 hours in the absence of these compounds were also collected.

This example was performed in duplicate for each sample (for experimental convenience, individual samples were subnumbered Control-1, Control-2, E7820-1, E7820-2, etc., such that they could be distinguished from each other). The subsequent procedures were exactly the same as shown in Example 1. Then, a GeneChip (Affymetrix) system (Human Focus array) was used to analyze changes in gene expression induced by each compound.

The 26 cel files obtained in this example for 13 samples (Control + 12 compounds) in duplicate were normalized at the probe level by applying the RMA (robust multi-array average) method (Biostatistics (2003), 4, 249-264), followed by calculating the log value of signal intensity at the gene level. Subsequently, the log value of signal intensity in the untreated group (Control-1) was subtracted from the log value of signal intensity in each compound-treated group for each gene to obtain the log signal ratio of each compound-treated group to Control-1. The cosine correlation coefficient was then calculated and defined as a correlation coefficient between experiments (Figure 2). Based on this correlation coefficient, hierarchical clustering analysis was performed by the UPGMA (Unweighted Pair Group Method with Arithmetic mean) method (Figure 3). The same calculation was also performed on Control-2 (Figures 4 and 5). The software programs used were R 2.0.1 (http://www.r-project.org/) and affy package 1.5.8 (http://www.bioconductor.org).

In Figures 2 to 5, "LY1" denotes LY186641, "LY2" denotes LY295501, "LY5" denotes LY573636, "CAI" denotes ethoxzolamide, "Cap" denotes capsaicin, "MST" denotes MST16, "Etop" denotes etoposide, "TSA" denotes trichostatin A, and "Kenp" denotes kenpaullone. In Figures 3 and 5, "de hclust (*, "average")" is a command for statistical analysis and means that the average of duplicate experimental data was used for clustering analysis in R.

As a result of the analysis, E7070, E7820, LY186641, LY295501, LY573636 and CQS were found to have a very high similarity in genetic changes caused in HCT116 cells and to have different profiles than any other compounds (MST16, etoposide, ethoxzolamide, capsaicin, trichostatin A, kenpaullone) (Figures 2 to 5). Thus, this analysis strongly suggested that E7070, E7820, LY186641, LY295501, LY573636 and CQS appeared to have the same or similar mechanism of action and hence produced the same or similar genetic changes and effects.

Example 3 Cancer cell line panel experiment

Panels of 36 human cancer cell lines were used to study the correlation of antiproliferative activity among E7820, E7070, CQS, LY186641 and LY295501. The cancer cell lines used were the following 36 lines: DLD-1, HCT15, HCT116, HT29, SW480, SW620 and WiDr (human colon cancer cell lines), A427, A549, LX-1, NCI-H460, NCI-H522, PC-9 and PC-10 (human lung cancer cell lines), GT3TKB, HGC27, MKN1, MKN7, MKN28 and MKN74 (human stomach cancer cell lines), AsPC-1, KP-1, KP-4, MiaPaCall, PANC-1 and SUIT-2 (human pancreas cancer cell lines), BSY-1, HBC5, MCF-7, MAD-MB-231, MDA-MB-435 and MDA-MB-468 (human breast cancer cell lines), as well as CCRF-CEM, HL60, K562 and MOLT-4 (human leukemia cell lines). All cells were cultured using RPMI-1640 medium supplemented

with 10% fetal bovine serum, 100 units/ml penicillin and 100 μg/ml streptomycin under 5% $CO_2$ conditions at 37°C (Table 1)

Table 1

**36 human cancer cell lines tested in 3-day MTT assays**

| Colon | Stomach | Breast |
|---|---|---|
| DLD-1 (1250/well, 16.8 h) | GT3TKB (2000/well), 21.1 h) | BSY-1 (2000/well, 46.1 h) |
| HCT15 (1500/well, 14.5 h) | HGC27 (1500/well, 14.6 h) | HBC5 (2000/well, 31.8 h) |
| HCT116 (1250/well, 13.4 h) | MKN1 (4000/well, 35.9 h) | MCF-7 (3000/well, 29.5 h) |
| HT29 (2500/well, 19.8 h) | MKN7 (3000/well, 37.4 h) | MDA-MR231 (2000/well, 21.6 h) |
| SW480 (3000/well), 19.5 h) | MKN28 (2000/well, 22.7 h) | MDA-MB-435 (3000/well, 24.4 h) |
| SW620 (2500/well, 17.3 h) | MKN74 (4000/well, 24.8 h) | MDA-MB-468 (3000/well, 34.2 h) |
| WiDr (2000/well, 18.9 h) | | |
| | Pancreas | Leukemia |
| Lung | AsPC-1 (2500/well, 28.4 h) | CCRF-CEM (1500/well, 27.2 h) |
| A427 (2500/well, 32.4 h) | KP-1 (2000/well, 24.8 h) | HL60 (1500/well, 29.5 h) |
| A549 (1250/well, 18.9 h) | KP-4 (2000/well, 16.7 h) | K562 (1500/well, 20.6 h) |
| LX-1 (2000/well, 17.2 h) | MiaPaCall (2500/well, 19.1 h) | MOLT-4 (1500/well, 22.3 h) |
| NCI-H460 (1000/well, 13.6 h) | PANC-1 (2500/well, 27.9 h) | |
| NCI-H522 (4000/well, 80.4 h) | SUIT-2 (2000/well, 15.6 h) | |
| PC-9 (2000/well, 23.7 h) | | |
| PC-10 (2000/well, 24.0 h) | | |

**Cell line (initial cell number, doubling time)**

Table 1 shows the type, initial cell number and doubling time of human cancer cell lines in human cancer cell line panels.

Cells of these cell lines were seeded in 96-well microplates (flat-bottomed) (50 μl/well) at the cell numbers indicated in Table 1. After 24 hours, a 3-fold dilution series of each compound was added (50 μl/well). After 72 hours, WST-8 (10 μl/well) was further added and the absorbance at 450 nm was measured. The least squares method was used to determine the 50% growth inhibitory concentration of each compound against all 36 cancer cell lines, followed by comparing the pattern of growth inhibition between compounds As indexes of correlation, Pearson's correlation coefficients were used (Paull, K. D. et al. Display and analysis of patterns of differential activity of drugs against human tumor cell lines: development of mean graph and COMPARE algorithm. J. Natl. Cancer Inst. 1989, 81, 1088-1092; Monks, A. et al. Feasibility of a high-flux anti-cancer drug screen using a diverse panel of cultured human tumor cell lines. J. Natl. Cancer Inst. 1991, 83, 757-766).

As a result, E7070, E7820, LY186641, LY295501 and CQS were found to show high correlation coefficients in antiproliferative activity against each cancer cell line (Table 2). Thus, this analysis strongly suggested that E7070, E7820, LY186641, LY295501 and CQS appeared to have the same or similar mechanism of action and hence produced the same or similar genetic changes and effects.

Table 2

| | E7070 | E7820 | CQS | LY186641 | LY295501 |
|---|---|---|---|---|---|
| E7070 | 1.00 | 0.98 | 0.97 | 0.93 | 0.80 |
| E7820 | 0.98 | 1.00 | 0.96 | 0.95 | 0.82 |
| CQS | 0.97 | 0.96 | 1.00 | 0.92 | 0.82 |
| LY186641 | 0.93 | 0.95 | 0.92 | 1.00 | 0.81 |
| LY295501 | 0.80 | 0.82 | 0.82 | 0.81 | 1.00 |

Table 2 shows correlation coefficients between compounds (E7070, E7820, CQS, LY186641 and LY295501) in human cancer cell line panels.

Example 4 Cross resistance in E7070-resistant sub-clonal cell lines

E7070-resistant sub-clonal cell lines were used to evaluate the antiproliferative activity of E7820, LY186641, LY295501, LY-ASAP and CQS. HCT116-C9 was a sub-clone separated from the human colon cancer cell line HCT116 (American Type Culture Collection, Manassas, VA, U.S A.), while HCT116-C9-C1 and HCT116-C9-C4 were E7070-resistant sub-clonal cell lines obtained by culturing this HCT116-C9 in the presence of gradually increasing concentrations of E7070 (Molecular Cancer Therapeutics, 2002, 1, 275-286)

These 3 cell lines HCT116-C9, HCT116-C9-C1 and HCT116-C9-C4 were each seeded at 3000 cells/well in 96-well microplates (flat-bottomed) (50 μl/well). After 24 hours, a 3-fold dilution series of each compound was added (50 μl/well). After 72 hours, the cell growth inhibitory activity was evaluated by the MTT method (Mossmann T., J. Immunol. Methods, 1983, 65, 55-63). The least squares method was used to determine the 50% growth inhibitory concentration of each compound against each cancer cell line.

As a result, the antiproliferative activity of E7070 against HCT116-C9 (C9) was IC50 0.127 μM, whereas the activity against HCT116-C9-C1 (C9C1) and HCT116-C9-C4 (C9C4) was IC50 31.9 μM and 26.9 μMespectively. It was therefore confirmed that the antiproliferative activity of E7070 against C9C1 and C9C4 was greatly reduced (Figure 6). Likewise, the antiproliferative activity of E7820, CQS, LY186641, LY295501 and LY-ASAP against HCT116-C9 was IC50 - 0.080 μM.73 μM, 33.6 μM, 10.9 μMnd 1.63 μM, respectively, whereas the activity against HCT116-C9-C1 and HCT116-C9-C4 was as follows: IC50 = 51.2 μM,34 μM,34 μM, 111 μM and 113 μM, respectively, for HCT116-C9-C1 and IC50 = 52.8 μM, 517 μM, 138 μM, 110 μM and 90.3 μM,respectively, for HCT116-C9-C4. Thus, with respect to the antiproliferative activity of E7820, CQS, LY186641, LY295501 and LY-ASAP, a greater reduction in the activity was observed in C9C1 and C9C4 than in C9 (Figure 6). Thus, this strongly suggested that E7070, E7820, LY186641, LY295501, LY ASAP and CQS appeared to have the same or similar mechanism of action and hence produced the same or similar genetic changes and effects.

Example 5 Cross resistance in E7070-resistant sub-clonal cell lines

Exactly the same procedure as used in Example 4 was repeated to evaluate the antiproliferative activity of both LY573636 and E7070 using E7070-resistant sub-clonal cell lines.

As a result, it was confirmed again that the antiproliferative activity of E7070 was more greatly reduced in HCT116-C9-C1 and HCT116-C9-C4 (IC50 = 32.7 μMnd 28.0 μM, respectively) than in HCT116-C9 (IC50 = 0.127 μM (Figure 7). Likewise, with respect to the antiproliferative activity of LY573636, a greater reduction in the activity was observed in HCT116-C9-C1 and HCT116-C9-C4 (IC50 = 264 μM and 240 μM, respectively) than in HCT116-C9 (IC50 = 5.11 μM) (Figure 7). Thus, this strongly suggested that LY573636 appeared to have the same or similar mechanism of action as E7070 and hence produced the same or similar genetic changes and effects.

These results (Examples 1 to 5) indicated that E7070, E7820, LY186641, LY295501, LY-ASAP, LY573636 or CQS or combinations thereof produced the same or similar genetic changes as well as the same or similar actions and effects.

Moreover, E7820 has been found to show excellent angiogenesis inhibitory activity and anti-tumor activity when used in combination with an angiogenesis inhibitory agent (see WO03/074045).

It was therefore indicated that a sulfonamide-including compound, preferably E7070, LY186641, LY295501, LY-ASAP, LY573636 or CQS or combinations thereof, showed excellent angiogenesis inhibitory activity and anti-tumor activity when used in combination with an angiogenesis inhibitor.

Example 6 Combined use of E7820 and 4-(3-chloro-4-(cyclopropylaminocarbonyl)-aminophenoxy)-7-methoxy-6-quinolinecarboxamide in subcutaneous transplantation model (in vivo) of human renal cancer cell line 786-O

Human renal cancer cell line 786-O (purchased from ATCC) was cultured in RPMII640 (containing 10% FBS) to about 80% confluency in a 5% carbon dioxide incubator, and the cells were collected by trypsin-EDTA. The cells were diluted with 50% Matrigel-containing phosphate buffer to prepare a 1 x $10^8$ cells/mL suspension, and the resulting cell suspension was subcutaneously transplanted in 0.1 mL volumes to nude mice at the side of their body. Starting from 7 days after the transplantation, E7820 (100 mg/kg, given twice a day for 2 weeks) and 4-(3-chloro-4-(cyclopropylamino-carbonyl)aminophenoxy)-7-methoxy-6-quinolinecarboxamide (100 mg/kg, given once a day for 2 weeks) were administered alone or in combination by the oral route. Tumor long and short axes were measured with a digimatic caliper (Mitsutoyo) and used to calculate both tumor volume and relative tumor volume according to the following equations.

$$\text{Tumor volume TV} = \text{tumor long axis (mm)} \times \text{tumor short axis}^2\ (\text{mm}^2)/2$$

Relative tumor volume RTV = tumor volume at the day of measurement/tumor volume at the first day of administration

If the co-treated group shows a statistically significant interaction as analyzed by two-way ANOVA, E7820 and 4-(3-chloro-4-(cyclopropylaminocarbonyl)aminophenoxy)-7-methoxy-6-quinolinecarboxamide are determined to have a synergistic effect on each other.

As a result, E7820 was found to produce a synergistic effect when used in combination with 4-(3-chloro-4-(cyclopropylaminocarbonyl)aminophenoxy)-7-methoxy-6-quinolinecarboxamide, and their combined use showed an excellent anti-tumor effect when compared to the effect of E7820 or 4-(3-chloro-4-(cyclopropylaminocarbonyl)aminophenoxy)-7-methoxy-6-quinolinecarboxamide alone (Table 3 and Figure 8).

Table 3

| Analyte administered | Relative tumor volume at Day 22 Mean ± SD | Two-way ANOVA |
|---|---|---|
| Control (untreated) | 1.61 ± 0.23 | |
| E7820 100 mg/kg | 0.80±013 | |
| Compound A 100 mg/kg | 0.59±012 | |
| E7820 100 mg/kg + Compound A 100 mg/kg | 0.16 ± 0.02 | p<0.01 Synergistic effect |

Table 3 shows the anti-tumor effect produced by either one or a combination of E7820 and 4-(3-chloro-4-( cyclopropylaminocarbonyl)aminophenoxy)-7 -methoxy-6-quinolinecarboxamide (in Table 3 and Figure 8, Compound A denotes 4-(3-chloro-4-(cyclopropylaminocarbonyl)-aminophenoxy)-7-methoxy-6-quinotinecarboxamide) in the transplantation model *(in vivo)* of renal cancer cell line 786-O. The first day of administration was defined as Day 1.

In view of the foregoing, a combination of E7820 and 4-(3-chloro-4-(cyclopropylaminocarbonyl)aminophenoxy)-7-methoxy-6-quinolinecarboxamide provides pharmaceutical compositions and kits showing excellent anti-tumor activity; the pharmaceutical compositions and kits of the present invention can be used for cancer treatment.

INDUSTRIAL APPLICABILITY

The present invention provides pharmaceutical compositions and kits showing excellent angiogenesis inhibitory activity and/or anti-tumor activity.

More specifically, as a result of combining sulfonamide-including compounds, preferably E7070, LY186641, LY295501, LY-ASAP, LY573636 or CQS or combinations thereof, with angiogenesis inhibitors, the present invention provides pharmaceutical compositions and kits showing excellent angiogenesis inhibitory activity and/or anti-tumor activity. The pharmaceutical compositions and kits of the present invention are useful for cancer treatment or angiogenesis inhibition. Likewise, when combining sulfonamide-including compounds, preferably E7820, with VEGF receptor kinase inhibitors, the present invention provides pharmaceutical compositions and kits showing excellent angiogenesis inhibitory activity and/or anti-tumor activity. The pharmaceutical compositions and kits of the present invention are useful for cancer treatment or angiogenesis inhibition.

## Claims

**1.** A pharmaceutical composition comprising a sulfonamide-including compound in combination with a VEGF receptor kinase inhibitor, wherein the sulfonamide-including compound is N-(3 -cyano-4-methyl- H-indol-7-yl)-3-cyanobenzenesulfonamide, or a pharmacologically acceptable salt thereof or a solvate thereof.

**2.** The pharmaceutical composition according to claim 1, wherein the VEGF receptor kinase inhibitor is at least one compound selected from the group consisting of:

(10) N-{2-chloro-4-[(6,7-dimethoxy-4-quinolyl)oxy]phenyl}-N'-(5-methyl-3-isoxazolyl)urea,
(11) 4-[(4-fluoro-2-methylindol-5-yl)oxy]-6-methoxy-7-[3-(pyrrolidin-1-yl)propoxy] quinazoline,
(12) 6-[2-(methylcarbamoyl)phenylsulfanyl]-3-E-[2-(pyridin-2-yl)ethenyl]indazole,
(13) 5-((Z)-(5-fluoro-2-oxo-1,2-dihydro-3H-indol-3-ylidene)methyl)-N-((2S)-2-hydroxy-3-morpholin-4-ylpropyl)-2,4-dimethyl-1 H-pyrrole-3-carboxamide,
(14) 3-((quinolin-4-ylmethyl)amino)-N-(4-(trifluoromethoxy)phenyl)thiophene-2-carboxamide,

(15) 6-(2,6-dichlorophenyl)-8-methyl-2-phenylamino-8H-pyrido[2,3-d]pyrimidin-7-one,
(16) 2-((1,6-dihydro-6-oxo-pyridin-3-ylmethyl)amino)-N-(3-(trifluoromethyl) phenyl)-3-pyridine-carboxamide,
(17) 4-(4-(4-chloro-phenylamino)-furo[2,3-d]pyridazin-7-yloxymethyl)-pyridine-2-carboxylic acid methylamide,
(18) N-(3-trifluoromethyl-4-chlorophenyl)-N'-(4-(2-methylcarbamoylpyridin-4-yl) oxyphenyl)urea,
(19) 4-amino-5-fluoro-3-(6-(4-methyl-piperazin-1-yl)-1H-benzimidazol-2-yl)-1 H-quinolin-2-one,
(20) 4-(4-(1-amino-1-methyl-ethyl)-phenyl)-2-(4-(2-morpholin-4-yl-ethyl)-phenylamino)-pyrimidine-5-carbonitrile,
(21) [6-[4-[(4-ethylpiperazin-1-yl)methyl]phenyl]-7H-pyrrolo[2,3-d]pyrimidin-4-yl]-((R)-1-phenylethyl)amine,
(22) 9-(1-methylethoxy)methyl-12-(3-hydroxypropyl)-6H,7H,13H-indeno[2,1-a] pyrrolo [3,4-c]carbazol-5 -one,
(23) N-(2,4-difluorophenyl)-N'-{4-[(6,7-dimethoxy-4-quinolyl)-oxy]-2-fluorophenyl} urea,
(24) 5-[N-methyl-N-(4-octadecyloxyphenyl)acetyl]amino-2-methylthiobenzoic acid,
(25) N-[4-(3-amino-1 H-indazol-4-yl)phenyl]-N' -(2-fluoro-5-methylphenyl)urea,
(26) 2-methyl-6-[2-(1-methyl-1H-imidazol-2-yl)-thieno[3,2-b]pyridin-7-yloxy]-benzo[b]thiophene-3-carboxylic acid methylamide,
(27) (R)-1-(4-(4-fluoro-2-methyl-1H-indol-5-yloxy)-5-methylpyrrolo[1,2-f][1,2,4] triazin-6-yloxy)propan-2-ol, and
(28) (S)-((R)-I-(4-(4-fluoro-2-methyl-1H-indol-5-yloxy)-5-methylpyrrolo[1,2-f] [1,2,4]triazin-6-yloxy)propan-2-ol) 2-aminopropanonate,
or a pharmacologically acceptable salt thereof or a solvate thereof.

**3.** The pharmaceutical composition according to claim 1, wherein the VEGF receptor kinase inhibitor is a methanesulfonate salt of 4-(3-chloro-4-(cyclopropylamino-carbonyl)aminophenoxy)-7-methoxy-6-quinolinecarboxamide.

**4.** The pharmaceutical composition according to any one of claims 1 to 3, which is a pharmaceutical composition for cancer treatment.

**5.** A pharmaceutical composition comprising a sulfonamide-including compound in combination with a VEGF receptor kinase inhibitor for simultaneous or separate use in the treatment of cancer or in the inhibition of angiogenesis, wherein the sulfonamide-including compound is as defined in claim 1, and wherein the VEGF receptor kinase inhibitor is as defined in claim 2 or 3.

**6.** A sulfonamide-including compound for use in treating cancer or inhibiting angiogenesis, wherein the sulfonamide-including compound is as defined in claim 1, and wherein the sulfonamide-including compound is for administration simultaneously or separately with a VEGF receptor kinase inhibitor as described in claim 2 or 3.

**7.** A VEGF receptor kinase inhibitor for use in treating cancer or inhibiting angiogenesis, wherein the VEGF receptor kinase inhibitor is as described in claim 2 or 3, and wherein the VEGF receptor kinase inhibitor is for administration simultaneously or separately with a sulfonamide-including compound as described in claim 1.

**8.** Use of a sulfonamide-including compound in the manufacture of a medicament for use in the treatment of cancer or in the inhibition of angiogenesis, wherein the sulfonamide-including compound is as described in claim 1, and wherein the sulfonamide-including compound is formulated for administration simultaneously or separately with a VEGF receptor kinase inhibitor as described in claim 2 or 3.

**9.** Use of a VEGF receptor kinase inhibitor in the manufacture of a medicament for use in the treatment of cancer or in the inhibition of angiogenesis, wherein the VEGF receptor kinase inhibitor is as described in claim 2 or 3, and wherein the VEGF receptor kinase inhibitor is formulated for administration simultaneously or separately with a sulfonamide-including compound as described in claim 1.

**10.** A kit comprising:

(a) at least one selected from the group consisting of a packaging container, an instruction manual and an package insert, each of which describes the combined use of a sulfonamide-including compound and a VEGF receptor kinase inhibitor; and
(b) a pharmaceutical composition comprising the sulfonamide-including compound,
wherein the sulfonamide-including compound is as defined in claim 1 and wherein the VEGF receptor kinase inhibitor is as defined in claim 2 or 3.

**11.** A kit comprising a set of a pharmaceutical composition comprising a sulfonamide-including compound and a pharmaceutical composition comprising a VEGF receptor kinase inhibitor,
wherein the sulfonamide-including compound is as defined in claim 1, and wherein the VEGF receptor kinase inhibitor is as defined in claim 2 or 3.

**12.** A kit according to claim 10 or 11 for use in the treatment of cancer or in the inhibition of angiogenesis.

FIG. 1

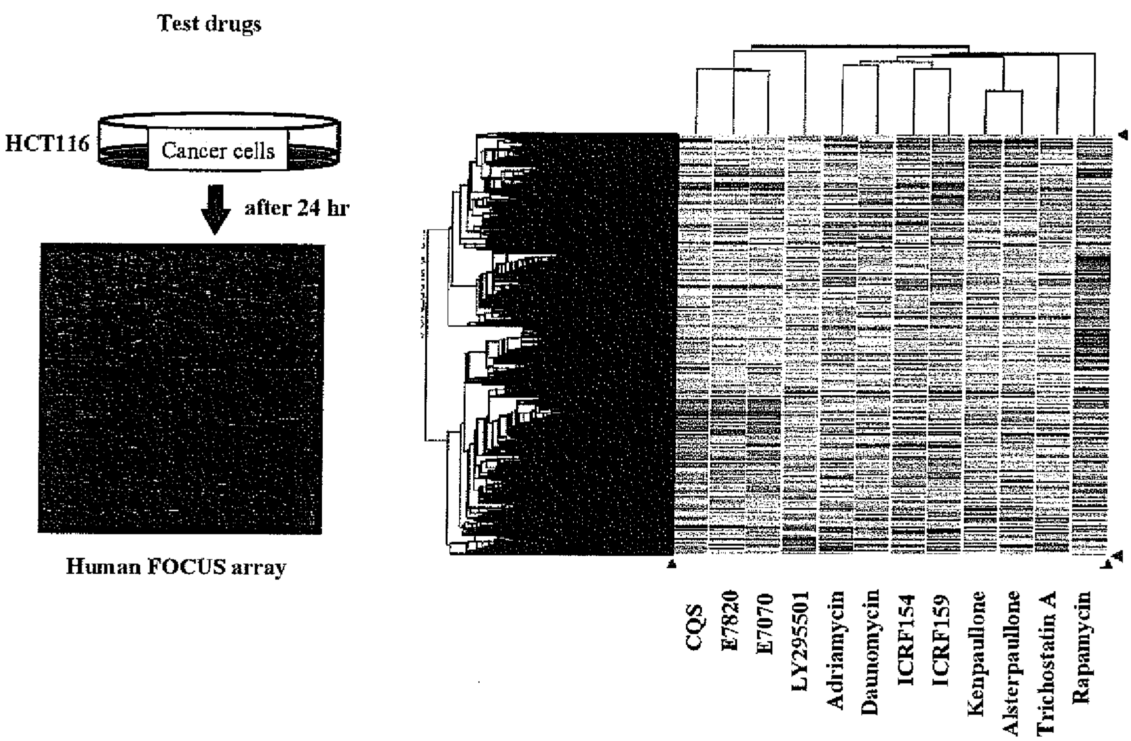
Test drugs
HCT116
Cancer cells
after 24 hr
Human FOCUS array
CQS
E7820
E7070
LY295501
Adriamycin
Daunomycin
ICRF154
ICRF159
Kenpaullone
Alsterpaullone
Trichostatin A
Rapamycin

FIG. 2

| | E7070.1 | E7070.2 | E7820.1 | E7820.2 | CQS.1 | CQS.2 | LY1.1 | LY1.2 | LY2.1 | LY2.2 | LY5.1 | LY5.2 | CAI.1 | CAI.2 | Cap.1 | Cap.2 | MST.1 | MST.2 | Etop.1 | Etop.2 | TSA.1 | TSA.2 | Kenp.1 | Kenp.2 |
|---|---|---|---|---|---|---|---|---|---|---|---|---|---|---|---|---|---|---|---|---|---|---|---|---|
| E7070-1 | *1.00* | 0.95 | 0.87 | 0.87 | 0.87 | 0.87 | 0.83 | 0.83 | 0.86 | 0.86 | 0.88 | 0.88 | 0.16 | 0.17 | 0.11 | 0.11 | 0.35 | 0.35 | 0.16 | 0.17 | 0.31 | 0.31 | 0.32 | 0.32 |
| E7070-2 | 0.95 | *1.00* | 0.87 | 0.86 | 0.87 | 0.87 | 0.83 | 0.83 | 0.86 | 0.85 | 0.88 | 0.88 | 0.17 | 0.17 | 0.11 | 0.11 | 0.36 | 0.36 | 0.16 | 0.17 | 0.32 | 0.32 | 0.32 | 0.33 |
| E7820-1 | 0.87 | 0.87 | *1.00* | 0.96 | 0.90 | 0.90 | 0.88 | 0.88 | 0.83 | 0.82 | 0.88 | 0.88 | 0.09 | 0.09 | 0.07 | 0.07 | 0.26 | 0.26 | 0.08 | 0.08 | 0.24 | 0.24 | 0.20 | 0.21 |
| E7820-2 | 0.87 | 0.86 | 0.96 | *1.00* | 0.90 | 0.90 | 0.88 | 0.88 | 0.83 | 0.82 | 0.88 | 0.88 | 0.09 | 0.09 | 0.07 | 0.07 | 0.27 | 0.26 | 0.08 | 0.09 | 0.24 | 0.25 | 0.21 | 0.21 |
| CQS-1 | 0.87 | 0.87 | 0.90 | 0.90 | *1.00* | 0.96 | 0.87 | 0.87 | 0.84 | 0.83 | 0.90 | 0.90 | 0.13 | 0.14 | 0.10 | 0.10 | 0.37 | 0.36 | 0.16 | 0.16 | 0.32 | 0.32 | 0.29 | 0.29 |
| CQS-2 | 0.87 | 0.87 | 0.90 | 0.90 | 0.96 | *1.00* | 0.87 | 0.87 | 0.84 | 0.83 | 0.90 | 0.90 | 0.14 | 0.14 | 0.10 | 0.10 | 0.37 | 0.37 | 0.16 | 0.16 | 0.32 | 0.33 | 0.29 | 0.29 |
| LY1-1 | 0.83 | 0.83 | 0.88 | 0.88 | 0.87 | 0.87 | *1.00* | 0.97 | 0.82 | 0.81 | 0.89 | 0.89 | 0.25 | 0.25 | 0.24 | 0.24 | 0.30 | 0.30 | 0.12 | 0.13 | 0.25 | 0.26 | 0.25 | 0.25 |
| LY1-2 | 0.83 | 0.83 | 0.88 | 0.88 | 0.87 | 0.87 | 0.97 | *1.00* | 0.82 | 0.81 | 0.89 | 0.88 | 0.25 | 0.25 | 0.24 | 0.25 | 0.30 | 0.30 | 0.12 | 0.13 | 0.25 | 0.26 | 0.25 | 0.26 |
| LY2-1 | 0.86 | 0.86 | 0.83 | 0.83 | 0.84 | 0.84 | 0.82 | 0.82 | *1.00* | 0.94 | 0.85 | 0.85 | 0.20 | 0.20 | 0.14 | 0.15 | 0.35 | 0.35 | 0.17 | 0.18 | 0.34 | 0.34 | 0.32 | 0.32 |
| LY2-2 | 0.86 | 0.85 | 0.82 | 0.82 | 0.83 | 0.83 | 0.81 | 0.81 | 0.94 | *1.00* | 0.84 | 0.84 | 0.19 | 0.19 | 0.15 | 0.15 | 0.35 | 0.35 | 0.18 | 0.18 | 0.33 | 0.34 | 0.31 | 0.32 |
| LY5-1 | 0.88 | 0.88 | 0.88 | 0.88 | 0.90 | 0.90 | 0.89 | 0.89 | 0.85 | 0.84 | *1.00* | 0.97 | 0.20 | 0.20 | 0.15 | 0.15 | 0.39 | 0.39 | 0.21 | 0.22 | 0.34 | 0.34 | 0.31 | 0.32 |
| LY5-2 | 0.88 | 0.88 | 0.88 | 0.88 | 0.90 | 0.90 | 0.89 | 0.88 | 0.85 | 0.84 | 0.97 | *1.00* | 0.20 | 0.20 | 0.15 | 0.15 | 0.39 | 0.39 | 0.21 | 0.22 | 0.34 | 0.34 | 0.31 | 0.32 |
| CAI-1 | 0.16 | 0.17 | 0.09 | 0.09 | 0.13 | 0.14 | 0.25 | 0.25 | 0.20 | 0.19 | 0.20 | 0.20 | *1.00* | 0.98 | 0.75 | 0.75 | 0.38 | 0.38 | 0.34 | 0.34 | 0.25 | 0.25 | 0.25 | 0.25 |
| CAI-2 | 0.17 | 0.17 | 0.09 | 0.09 | 0.14 | 0.14 | 0.25 | 0.25 | 0.20 | 0.19 | 0.20 | 0.20 | 0.98 | *1.00* | 0.75 | 0.75 | 0.38 | 0.38 | 0.34 | 0.33 | 0.25 | 0.25 | 0.26 | 0.26 |
| Cap-1 | 0.11 | 0.11 | 0.07 | 0.07 | 0.10 | 0.10 | 0.24 | 0.24 | 0.14 | 0.15 | 0.15 | 0.15 | 0.75 | 0.75 | *1.00* | 0.99 | 0.35 | 0.34 | 0.31 | 0.31 | 0.19 | 0.19 | 0.14 | 0.14 |
| Cap-2 | 0.11 | 0.11 | 0.07 | 0.07 | 0.10 | 0.10 | 0.24 | 0.25 | 0.15 | 0.15 | 0.15 | 0.15 | 0.75 | 0.75 | 0.99 | *1.00* | 0.34 | 0.34 | 0.31 | 0.31 | 0.18 | 0.18 | 0.14 | 0.14 |
| MST-1 | 0.35 | 0.36 | 0.26 | 0.27 | 0.37 | 0.37 | 0.30 | 0.30 | 0.35 | 0.35 | 0.39 | 0.39 | 0.38 | 0.38 | 0.35 | 0.34 | *1.00* | 0.94 | 0.62 | 0.62 | 0.57 | 0.57 | 0.39 | 0.39 |
| MST-2 | 0.35 | 0.36 | 0.26 | 0.26 | 0.36 | 0.37 | 0.30 | 0.30 | 0.35 | 0.35 | 0.39 | 0.39 | 0.38 | 0.38 | 0.34 | 0.34 | 0.94 | *1.00* | 0.62 | 0.63 | 0.57 | 0.57 | 0.39 | 0.39 |
| Etop-1 | 0.16 | 0.16 | 0.08 | 0.08 | 0.16 | 0.16 | 0.12 | 0.12 | 0.17 | 0.18 | 0.21 | 0.21 | 0.34 | 0.34 | 0.31 | 0.31 | 0.62 | 0.62 | *1.00* | 0.96 | 0.45 | 0.45 | 0.24 | 0.24 |
| Etop-2 | 0.17 | 0.17 | 0.08 | 0.09 | 0.16 | 0.16 | 0.13 | 0.13 | 0.18 | 0.18 | 0.22 | 0.22 | 0.34 | 0.33 | 0.31 | 0.31 | 0.62 | 0.63 | 0.96 | *1.00* | 0.45 | 0.45 | 0.24 | 0.24 |
| TSA-1 | 0.31 | 0.32 | 0.24 | 0.24 | 0.32 | 0.32 | 0.25 | 0.25 | 0.34 | 0.33 | 0.34 | 0.34 | 0.25 | 0.25 | 0.19 | 0.18 | 0.57 | 0.57 | 0.45 | 0.45 | *1.00* | 0.93 | 0.34 | 0.34 |
| TSA-2 | 0.31 | 0.32 | 0.24 | 0.25 | 0.32 | 0.33 | 0.26 | 0.26 | 0.34 | 0.34 | 0.34 | 0.34 | 0.25 | 0.25 | 0.19 | 0.18 | 0.57 | 0.57 | 0.45 | 0.45 | 0.93 | *1.00* | 0.34 | 0.34 |
| Kenp-1 | 0.32 | 0.32 | 0.20 | 0.21 | 0.29 | 0.29 | 0.25 | 0.25 | 0.32 | 0.31 | 0.31 | 0.31 | 0.25 | 0.26 | 0.14 | 0.14 | 0.39 | 0.39 | 0.24 | 0.24 | 0.34 | 0.34 | *1.00* | 0.96 |
| Kenp-2 | 0.32 | 0.33 | 0.21 | 0.21 | 0.29 | 0.29 | 0.25 | 0.26 | 0.32 | 0.32 | 0.32 | 0.32 | 0.25 | 0.26 | 0.14 | 0.14 | 0.39 | 0.39 | 0.24 | 0.24 | 0.34 | 0.34 | 0.96 | *1.00* |

0.7<X<=1.0 (thick solid box)

0.4<X<=0.7 (dashed box)

0<X<=0.4 (thin solid box)

FIG. 3

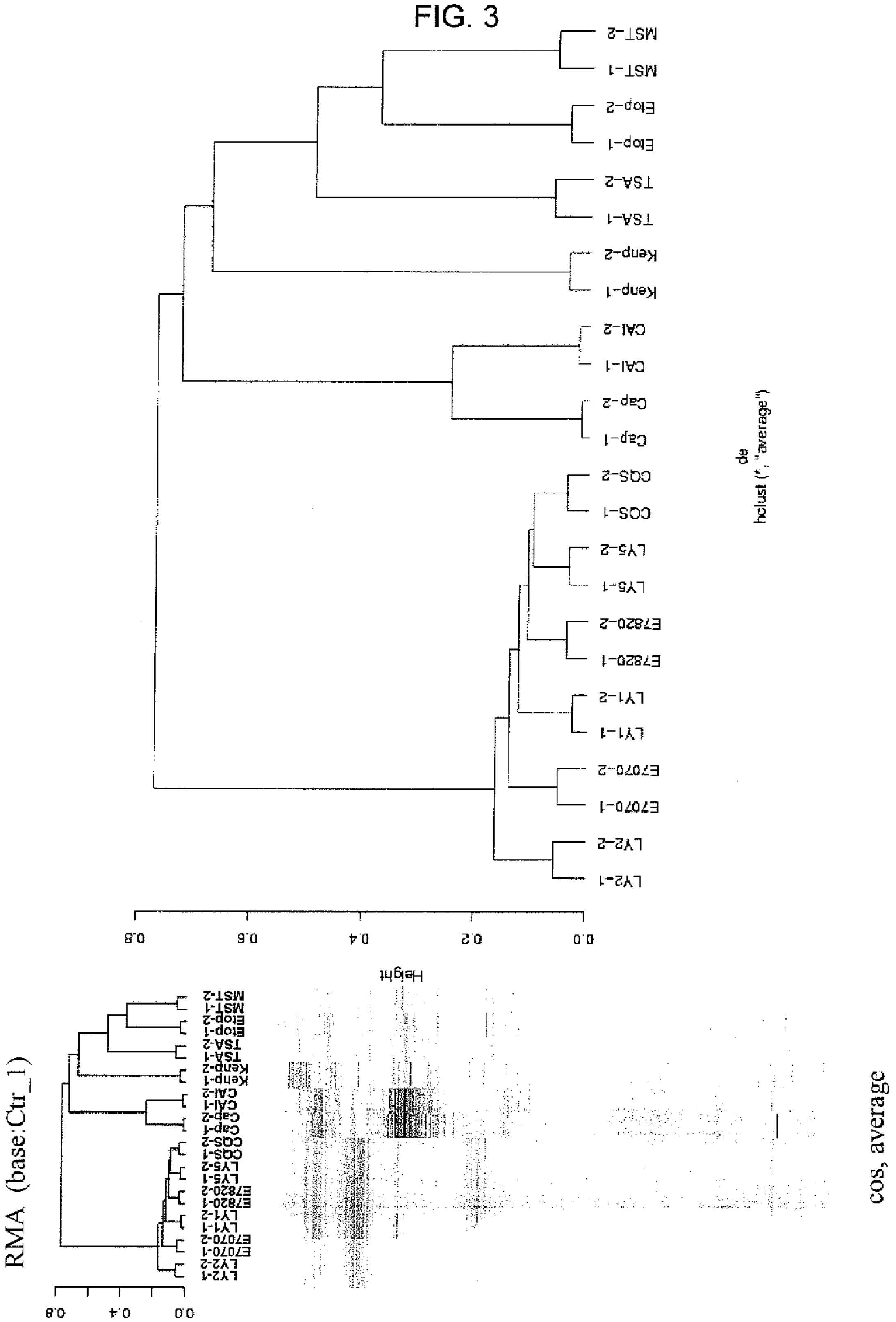
MST-2
MST-1
Etop-2
Etop-1
TSA-2
TSA-1
Kenp-2
Kenp-1
CAI-2
CAI-1
Cap-2
Cap-1
COS-2
COS-1
LY5-2
LY5-1
E7820-2
E7820-1
LY1-2
LY1-1
E7070-2
E7070-1
LY2-2
LY2-1
0.8
0.6
0.4
0.2
0.0
Height
de
hclust (*, "average")
RMA (base:Ctr_1)
cos, average

FIG. 4

| | E7070.1 | E7070.2 | E7820.1 | E7820.2 | CQS.1 | CQS.2 | LY1.1 | LY1.2 | LY2.1 | LY2.2 | LY5.1 | LY5.2 | CAI.1 | CAI.2 | Cap.1 | Cap.2 | MST.1 | MST.2 | Etop.1 | Etop.2 | TSA.1 | TSA.2 | Kenp.1 | Kenp.2 |
|---|---|---|---|---|---|---|---|---|---|---|---|---|---|---|---|---|---|---|---|---|---|---|---|---|
| E7070-1 | *1.00* | 0.94 | 0.87 | 0.87 | 0.86 | 0.86 | 0.81 | 0.81 | 0.84 | 0.83 | 0.86 | 0.86 | 0.04 | 0.04 | 0.03 | 0.04 | 0.24 | 0.23 | 0.06 | 0.06 | 0.22 | 0.22 | 0.19 | 0.19 |
| E7070-2 | 0.94 | *1.00* | 0.86 | 0.86 | 0.85 | 0.85 | 0.81 | 0.81 | 0.84 | 0.83 | 0.86 | 0.86 | 0.04 | 0.04 | 0.03 | 0.03 | 0.24 | 0.23 | 0.05 | 0.06 | 0.22 | 0.22 | 0.19 | 0.20 |
| E7820-1 | 0.87 | 0.86 | *1.00* | 0.96 | 0.90 | 0.90 | 0.86 | 0.86 | 0.81 | 0.80 | 0.87 | 0.87 | 0.00 | 0.00 | 0.02 | 0.03 | 0.22 | 0.22 | 0.03 | 0.04 | 0.21 | 0.22 | 0.12 | 0.12 |
| E7820-2 | 0.87 | 0.86 | 0.96 | *1.00* | 0.90 | 0.90 | 0.85 | 0.85 | 0.81 | 0.80 | 0.87 | 0.67 | 0.00 | 0.00 | 0.02 | 0.03 | 0.22 | 0.21 | 0.03 | 0.04 | 0.21 | 0.22 | 0.12 | 0.13 |
| CQS-1 | 0.86 | 0.85 | 0.90 | 0.90 | *1.00* | 0.96 | 0.85 | 0.85 | 0.82 | 0.81 | 0.89 | 0.88 | 0.02 | 0.02 | 0.03 | 0.03 | 0.28 | 0.27 | 0.07 | 0.07 | 0.25 | 0.25 | 0.17 | 0.18 |
| CQS-2 | 0.86 | 0.85 | 0.90 | 0.90 | 0.96 | *1.00* | 0.86 | 0.85 | 0.82 | 0.81 | 0.89 | 0.89 | 0.02 | 0.02 | 0.03 | 0.04 | 0.28 | 0.27 | 0.07 | 0.07 | 0.25 | 0.25 | 0.17 | 0.18 |
| LY1-1 | 0.81 | 0.81 | 0.86 | 0.85 | 0.85 | 0.86 | *1.00* | 0.97 | 0.80 | 0.79 | 0.88 | 0.88 | 0.17 | 0.17 | 0.19 | 0.19 | 0.20 | 0.20 | 0.04 | 0.04 | 0.17 | 0.17 | 0.15 | 0.16 |
| LY1-2 | 0.81 | 0.81 | 0.86 | 0.85 | 0.85 | 0.85 | 0.97 | *1.00* | 0.80 | 0.79 | 0.87 | 0.87 | 0.17 | 0.17 | 0.19 | 0.20 | 0.20 | 0.20 | 0.03 | 0.04 | 0.17 | 0.17 | 0.15 | 0.16 |
| LY2-1 | 0.84 | 0.84 | 0.81 | 0.81 | 0.82 | 0.82 | 0.80 | 0.80 | *1.00* | 0.93 | 0.83 | 0.83 | 0.09 | 0.09 | 0.07 | 0.08 | 0.23 | 0.23 | 0.07 | 0.08 | 0.24 | 0.24 | 0.20 | 0.20 |
| LY2-2 | 0.83 | 0.83 | 0.80 | 0.80 | 0.81 | 0.81 | 0.79 | 0.79 | 0.93 | *1.00* | 0.82 | 0.82 | 0.08 | 0.08 | 0.07 | 0.08 | 0.23 | 0.22 | 0.07 | 0.07 | 0.23 | 0.24 | 0.19 | 0.20 |
| LY5-1 | 0.86 | 0.86 | 0.87 | 0.87 | 0.89 | 0.89 | 0.88 | 0.87 | 0.83 | 0.82 | *1.00* | 0.96 | 0.09 | 0.09 | 0.08 | 0.08 | 0.28 | 0.28 | 0.11 | 0.12 | 0.24 | 0.24 | 0.19 | 0.20 |
| LY5-2 | 0.86 | 0.86 | 0.87 | 0.87 | 0.88 | 0.89 | 0.88 | 0.87 | 0.83 | 0.82 | 0.96 | *1.00* | 0.09 | 0.09 | 0.07 | 0.08 | 0.28 | 0.28 | 0.11 | 0.12 | 0.24 | 0.24 | 0.19 | 0.20 |
| CAI-1 | 0.04 | 0.04 | 0.00 | 0.00 | 0.02 | 0.02 | 0.17 | 0.17 | 0.09 | 0.08 | 0.09 | 0.09 | *1.00* | 0.98 | 0.74 | 0.74 | 0.26 | 0.26 | 0.25 | 0.25 | 0.13 | 0.13 | 0.14 | 0.14 |
| CAI-2 | 0.04 | 0.04 | 0.00 | 0.00 | 0.02 | 0.02 | 0.17 | 0.17 | 0.09 | 0.08 | 0.09 | 0.09 | 0.98 | *1.00* | 0.74 | 0.74 | 0.26 | 0.26 | 0.25 | 0.25 | 0.13 | 0.13 | 0.15 | 0.15 |
| Cap-1 | 0.03 | 0.03 | 0.02 | 0.02 | 0.03 | 0.03 | 0.19 | 0.19 | 0.07 | 0.07 | 0.08 | 0.07 | 0.74 | 0.74 | *1.00* | 0.99 | 0.28 | 0.28 | 0.26 | 0.26 | 0.12 | 0.12 | 0.06 | 0.06 |
| Cap-2 | 0.04 | 0.03 | 0.03 | 0.03 | 0.03 | 0.04 | 0.19 | 0.20 | 0.08 | 0.08 | 0.08 | 0.08 | 0.74 | 0.74 | 0.99 | *1.00* | 0.28 | 0.28 | 0.26 | 0.25 | 0.11 | 0.12 | 0.06 | 0.06 |
| MST-1 | 0.24 | 0.24 | 0.22 | 0.22 | 0.28 | 0.28 | 0.20 | 0.20 | 0.23 | 0.23 | 0.28 | 0.28 | 0.26 | 0.26 | 0.28 | 0.28 | *1.00* | 0.92 | 0.57 | 0.57 | 0.50 | 0.50 | 0.24 | 0.25 |
| MST-2 | 0.23 | 0.23 | 0.22 | 0.21 | 0.27 | 0.27 | 0.20 | 0.20 | 0.23 | 0.22 | 0.28 | 0.28 | 0.26 | 0.26 | 0.28 | 0.28 | 0.92 | *1.00* | 0.57 | 0.57 | 0.50 | 0.50 | 0.24 | 0.25 |
| Etop-1 | 0.06 | 0.05 | 0.03 | 0.03 | 0.07 | 0.07 | 0.04 | 0.03 | 0.07 | 0.07 | 0.11 | 0.11 | 0.25 | 0.25 | 0.26 | 0.26 | 0.57 | 0.57 | *1.00* | 0.96 | 0.39 | 0.39 | 0.12 | 0.13 |
| Etop-2 | 0.06 | 0.06 | 0.04 | 0.04 | 0.07 | 0.07 | 0.04 | 0.04 | 0.08 | 0.07 | 0.12 | 0.12 | 0.25 | 0.25 | 0.26 | 0.25 | 0.57 | 0.57 | 0.96 | *1.00* | 0.39 | 0.39 | 0.12 | 0.13 |
| TSA-1 | 0.22 | 0.22 | 0.21 | 0.21 | 0.25 | 0.25 | 0.17 | 0.17 | 0.24 | 0.23 | 0.24 | 0.24 | 0.13 | 0.13 | 0.12 | 0.11 | 0.50 | 0.50 | 0.39 | 0.39 | *1.00* | 0.93 | 0.21 | 0.22 |
| TSA-2 | 0.22 | 0.22 | 0.22 | 0.22 | 0.25 | 0.25 | 0.17 | 0.17 | 0.24 | 0.24 | 0.24 | 0.24 | 0.13 | 0.13 | 0.12 | 0.12 | 0.50 | 0.50 | 0.39 | 0.39 | 0.93 | *1.00* | 0.22 | 0.22 |
| Kenp-1 | 0.19 | 0.19 | 0.12 | 0.12 | 0.17 | 0.17 | 0.15 | 0.15 | 0.20 | 0.19 | 0.19 | 0.19 | 0.14 | 0.15 | 0.06 | 0.06 | 0.24 | 0.24 | 0.12 | 0.12 | 0.21 | 0.22 | *1.00* | 0.95 |
| Kenp-2 | 0.19 | 0.20 | 0.12 | 0.13 | 0.18 | 0.18 | 0.16 | 0.16 | 0.20 | 0.20 | 0.20 | 0.20 | 0.14 | 0.15 | 0.06 | 0.06 | 0.25 | 0.25 | 0.13 | 0.13 | 0.22 | 0.22 | 0.95 | *1.00* |

0.7<X<=1.0 (solid bold box)

0.4<X<=0.7 (dashed box)

0<X<=0.4 (thin box)

FIG. 5

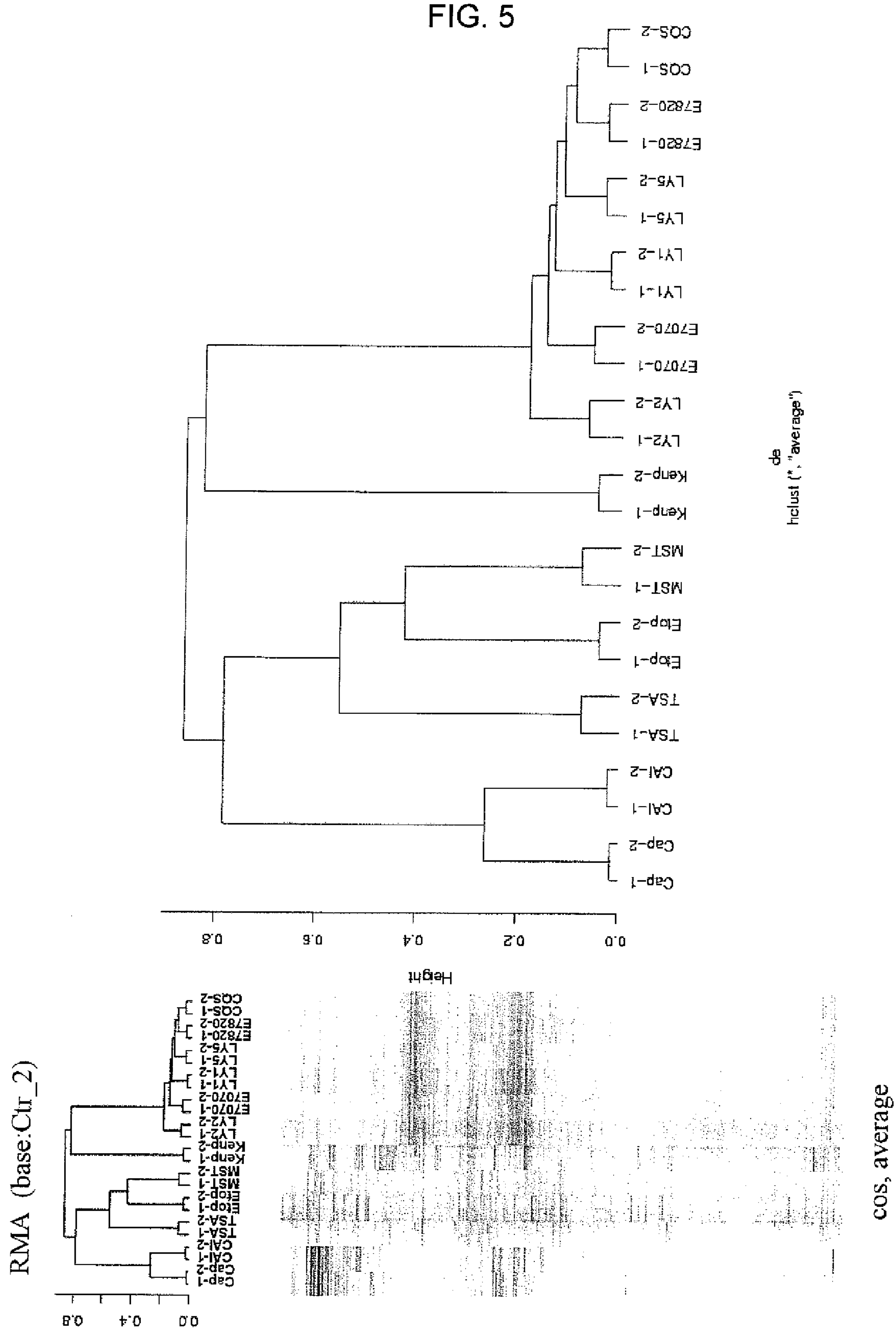
COS-2
COS-1
E7820-2
E7820-1
LY5-2
LY5-1
LY1-2
LY1-1
E7070-2
E7070-1
LY2-2
LY2-1
Kenp-2
Kenp-1
MST-2
MST-1
Etop-2
Etop-1
TSA-2
TSA-1
CAI-2
CAI-1
Cap-2
Cap-1
0.0
0.2
0.4
0.6
0.8
Height
de
hclust (*, "average")
RMA (base:Ctr_2)
cos, average

FIG. 6

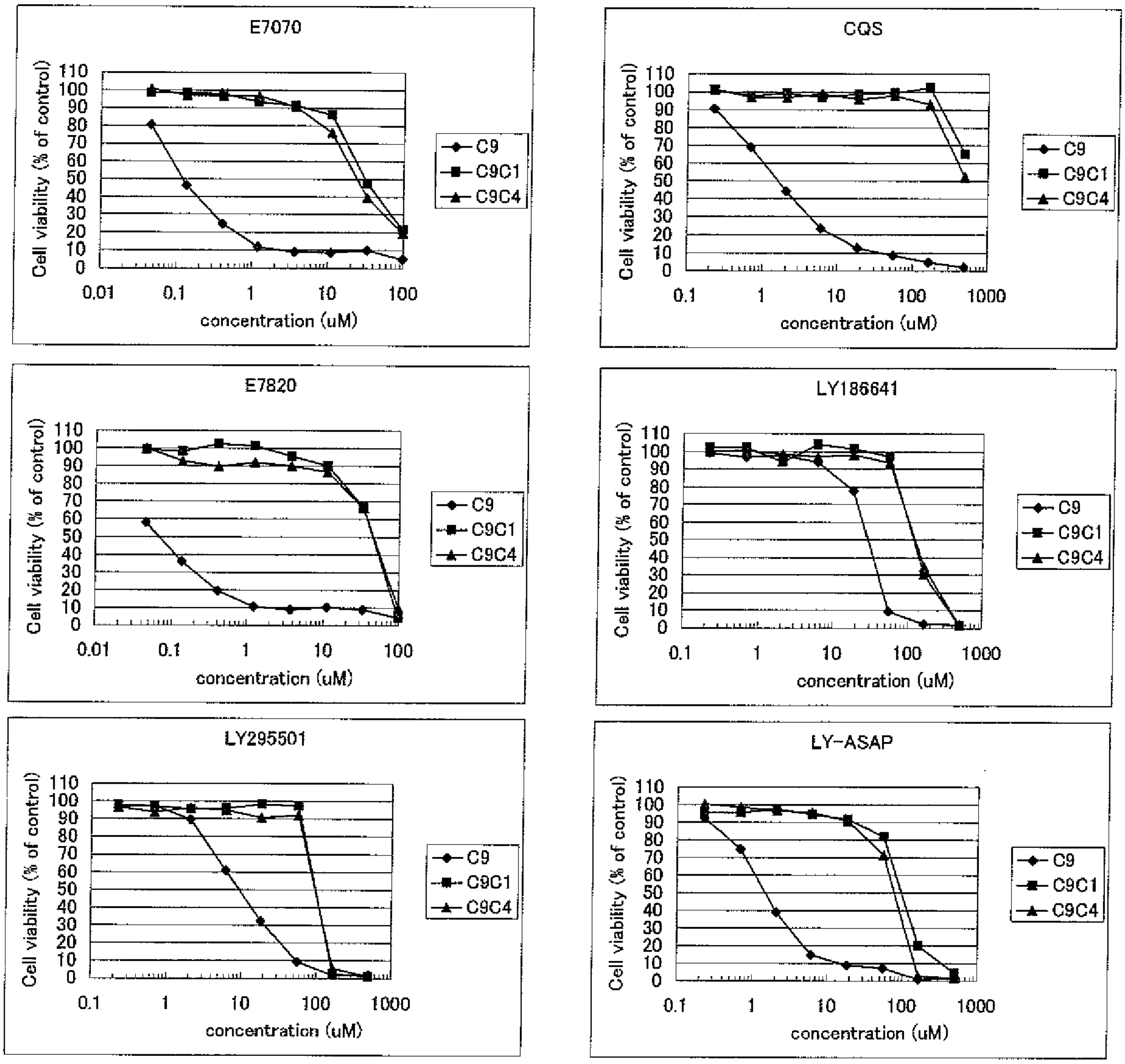
E7070
CQS
E7820
LY186641
LY295501
LY-ASAP
Cell viability (% of control)
concentration (uM)
C9
C9C1
C9C4
0.01
0.1
1
10
100
1000
0
10
20
30
40
50
60
70
80
90
100
110

FIG. 7

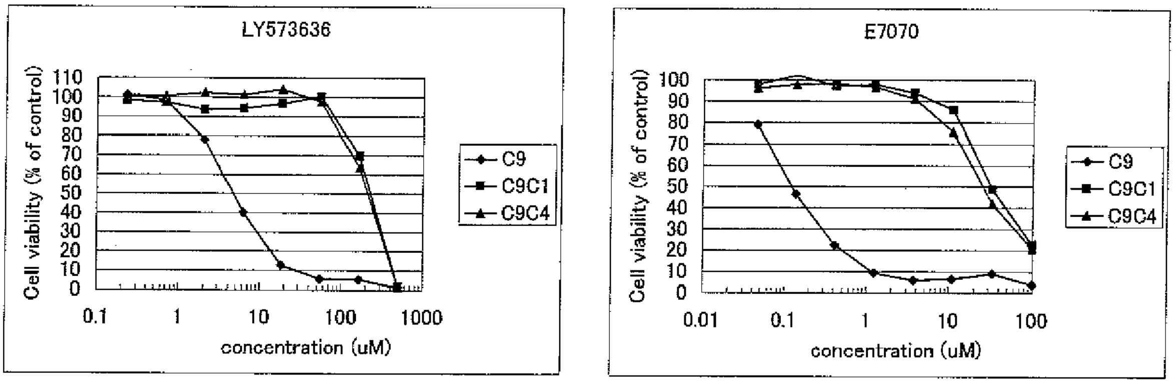
LY573636
Cell viability (% of control)
110
100
90
80
70
60
50
40
30
20
10
0
0.1
1
10
100
1000
concentration (uM)
C9
C9C1
C9C4
E7070
Cell viability (% of control)
100
90
80
70
60
50
40
30
20
10
0
0.01
0.1
1
10
100
concentration (uM)
C9
C9C1
C9C4

FIG. 8

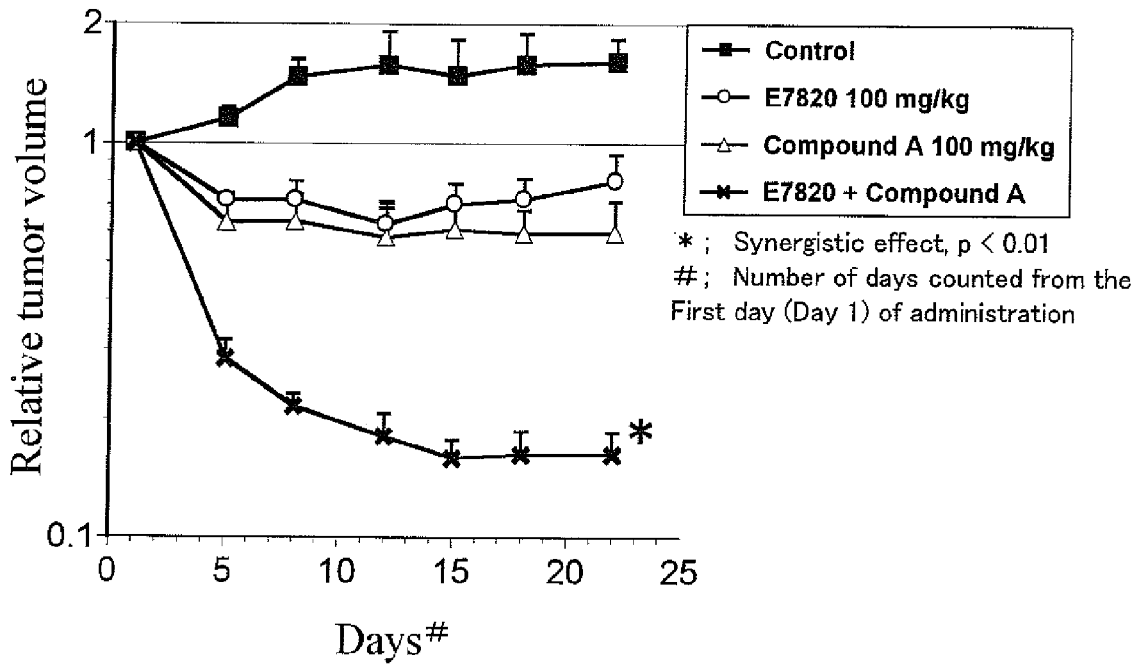
Relative tumor volume
2
1
0.1
0
5
10
15
20
25
Days#
Control
E7820 100 mg/kg
Compound A 100 mg/kg
E7820 + Compound A
*
*; Synergistic effect, p < 0.01
#; Number of days counted from the First day (Day 1) of administration

Europäisches Patentamt
European Patent Office
Office européen des brevets

# EUROPEAN SEARCH REPORT

Application Number
EP 11 16 9343

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 03/074045 A1 (EISAI CO LTD [JP]; WAKABAYASHI TOSHIAKI [JP]; ONO NAOTO [JP]; SEMBA TA) 12 September 2003 (2003-09-12)<br>* figure 1; examples 1-4; tables 1-4 *<br>* claims 1-50 *<br>----- | 1,4,6,8<br><br><br>1-12 | INV.<br>A61K31/18<br>A61K31/381<br>A61K31/404<br>A61K31/4439<br>A61K31/47<br>A61K31/4709<br>A61K31/498<br>A61K31/635<br>A61K31/64<br>A61K39/395<br>A61P9/00<br>A61P35/00<br>A61K45/06 |
| X<br><br>Y | ONO NAOTO ET AL: "Angiogenesis inhibitor, E7820; enhancement of anti-angiogenic activity through the combination with receptor kinase inhibitors of VEGF or bFGF",<br>PROCEEDINGS OF THE ANNUAL MEETING OF THE AMERICAN ASSOCIATION FOR CANCER RESEARCH, AMERICAN ASSOCIATION FOR CANCER RESEARCH, US,<br>vol. 43, 1 March 2002 (2002-03-01), page 182, XP001538359,<br>ISSN: 0197-016X<br>* the whole document *<br>----- | 1,4,6,8<br><br>1-12 | |
| X<br><br>Y | MARME D: "The impact of anti-angiogenic agents on cancer therapy",<br>JOURNAL OF CANCER RESEARCH AND CLINICAL ONCOLOGY 200311 DE LNKD- DOI:10.1007/S00432-003-0488-9,<br>vol. 129, no. 11, November 2003 (2003-11), pages 607-620, XP000002656444,<br>ISSN: 0171-5216<br>* tables 1-4 *<br>----- | 7,9<br><br>1,2,4-12 | TECHNICAL FIELDS SEARCHED (IPC)<br>A61K |
| X,P<br><br>Y,P | WO 2005/063713 A1 (EISAI CO LTD [JP]; MATSUSHIMA TOMOHIRO [JP]; NAKAMURA TAIJU [JP]; YOSH) 14 July 2005 (2005-07-14)<br>* claims 1-7, 37-50 *<br>----- | 7,9<br><br>1,3-12 | |

The present search report has been drawn up for all claims

| Place of search | Date of completion of the search | Examiner |
|---|---|---|
| The Hague | 9 August 2011 | Herdemann, Matthias |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 16 9343

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2011

| Patent document cited in search report | Publication date | Patent family member(s) | Publication date |
|---|---|---|---|
| WO 03074045 A1 | 12-09-2003 | AU 2003211594 A1 | 16-09-2003 |
| | | EP 1481678 A1 | 01-12-2004 |
| | | JP 4542783 B2 | 15-09-2010 |
| | | US 2009269352 A1 | 29-10-2009 |
| | | US 2005119303 A1 | 02-06-2005 |
| WO 2005063713 A1 | 14-07-2005 | AU 2004309217 A1 | 14-07-2005 |
| | | BR PI0418200 A | 17-04-2007 |
| | | CA 2543650 A1 | 14-07-2005 |
| | | CN 1890220 A | 03-01-2007 |
| | | CN 101337931 A | 07-01-2009 |
| | | CN 101337932 A | 07-01-2009 |
| | | CN 101337933 A | 07-01-2009 |
| | | EP 1698623 A1 | 06-09-2006 |
| | | HK 1102027 A1 | 18-06-2010 |
| | | IL 175933 A | 30-12-2010 |
| | | JP 4648835 B2 | 09-03-2011 |
| | | JP 2010280713 A | 16-12-2010 |
| | | KR 20060113759 A | 02-11-2006 |
| | | KR 20070107185 A | 06-11-2007 |
| | | KR 20080028511 A | 31-03-2008 |
| | | MX PA06007256 A | 23-08-2006 |
| | | NZ 547518 A | 26-06-2009 |
| | | RU 2328489 C2 | 10-07-2008 |
| | | US 2007078159 A1 | 05-04-2007 |
| | | ZA 200605226 A | 25-04-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7165708 A
- WO 0050395 A
- EP 0222475 A
- WO 02098848 A
- WO 03074045 A
- EP 0222475 A1
- EP 0555036 A2
- WO 021038848 A
- WO 9507276 A
- WO 02032872 A
- WO 2004020434 A
- WO 2005063713 A
- WO 200563713 A
- US 5792783 A
- WO 9962890 A
- WO 98135958 A
- WO OO47212 A
- WO 0123375 A
- WO 0306462 A
- US 20041009965 A
- US 6524583 B
- US 6676941 B
- US 6811779 B
- US 5747651 A


### Non-patent literature cited in the description


- **SCHENA M ; SHALON D ; DAVIS RW ; BROWN PO.** *Science,* 1995, vol. 270, 467-70
- **LOCKHART, D.J. ; DONG, H. ; BYRNE, M.C. ; FOLLETTIE, M.T ; GALLO, M.V ; CHEE, M.S. ; MITTMANN, M. ; WANG C. ; KOBAYASHI, M. ; HORTON, H.** *Nature Biotechnology,* 1996, vol. 14, 1675-1680
- **RHEE CH ; RUAN S ; CHEN S ; CHENCHIK A ; LEVIN VA ; YUNG AW ; FULLER GN ; ZHANG W.** *Oncol Rep,* 1999, vol. 6, 393-401
- **ZIMMERMANN J ; ERDMANN D ; LALANDE I ; GROSSENBACHER R ; NOORANI M ; FURST P.** *Oncogene,* 2000, vol. 19, 2913-20
- **KUDOH K ; RAMANNA M ; RAVATN R ; ELKAHL-OUN AG ; BITTNER ML ; MELTZER PS ; TRENT JM ; DALTON WS ; CHIN KV.** *Cancer Res,* 2000, 4161-6
- **ROSS DT ; SCHERF U ; EISEN MB ; PEROU CM ; REES C ; SPELLMAN P ; IYER V ; JEFFREY S ; VAN DE RIJN M ; WALTHAM M.** *Nat Genet,* 2000, vol. 24, 227-3 5
- **SCHERF U ; ROSS DT ; WALTHAM M ; SMITH LH ; LEE JK ; TANABE L ; KOHN KW ; REINHOLD WC ; MYERS TG ; ANDREWS DT.** *Nat Genet,* 2000, vol. 24, 236-44
- *J. Am. Chem. Soc.,* 1947, vol. 71, 6-10
- *Cancer Research,* 1995, vol. 55, 529b-5301
- *Cancer Research,* 1991, vol. 51, 6180-4
- *J. Clinical Investigation,* vol. 111, 1287-95
- *Clinical Cancer Research,* 2000, vol. 6, 3056-61
- *International J. Cancer,* 1998, vol. 78, 361-5
- *Annuals ofIV.Y Acad. Science,* 1999, 84-6
- *International J. Pancreatol,* 1997, vol. 21, 1-12
- *Science,* 1998, vol. 282, 1324-1327
- *Cancer Research,* 2000, vol. 60, 970-975
- *Journal of Medicinal Chemistry,* 1999, vol. 42, 5369-5389
- *Proc. Am. Assoc. Cancer Res.,* 2001, vol. 42, 583
- *Journal of Medicinal Chemistry,* 2002, vol. 45, 1300-1312
- *Cancer Res.,* 1999, vol. 59, 99-106
- *Journal of Medicinal Chemistry,* 1998, vol. 41, 2588-2603
- *Cancer Res.,* 2000, vol. 60, 4152-4160
- *Journal of Medicinal Chemistry,* 1999, vol. 42, 5120-5130
- *Clinical Cancer Research,* 2003, vol. 9, 327-337
- *Journal of Medicinal Chemistry,* 2003, vol. 46, 1116-9
- *Pro. Am. Assoc. Cancer Research,* 2002, vol. 43, 1080
- *Journal of Medicinal Chemistry,* 2003, vol. 46, 5375-88
- *Proc. Am Soc. Clin. Oncology,* 2002, vol. 21
- *Cancer Research,* 2003, vol. 63, 7301-9
- *Pro. Am. Assoc. Cancer Research,* 2002, vol. 43, 175
- *Molecular Cancer Therapeutics,* 2004, vol. 3, 1639-49
- *Cancer Research,* 2000, vol. 60, 2179-2189

- *Journal of Medicinal Chemistry,* 2000, vol. 43, 2310-23
- *Proceedings of the American Association for Cancer Research,* 2004, vol. 45, 594
- *Proceedings of the American Association for Cancer Research,* 2004, vol. 45, 595
- *Cancer Research,* 2005, vol. 65, 4389-400
- *American Journal of Pathology,* 2004, vol. 165, 35-52
- *Proc. Am Assoc. Cancer Res.,* 2001, vol. 42, 583
- *Journal of IV.ledicinal Chemistry,* 1999, vol. 42, 5120-5130
- *Proc. Am. Soc. Clin. Oncology,* 2002, vol. 21
- *Pro. Am. Assoc- Cancer Research,* 2002, vol. 43, 175
- *Proceedings of the American Association for Cancer Research,* 2005, vol. 46
- *Molecular Cancer Therapeutics,* 2005, vol. 4, 1186-1197
- *Cancer Biol Ther.,* 2005, vol. 4
- *EORTC-NCI-AACR Symp Mol Targets Cancer Ther,* 2004, vol. 2
- *Cancer Research,* 2004, vol. 64, 7099-7109
- *Organic Process Res Dev,* 2002, vol. 6, 777-8l
- *Clinical Cancer Research,* 2005, vol. 11, 3633-3641
- *Molecular Pharmacology,* 2004, vol. 66, 635-647
- *Cancer Research,* 2004, vol. 64, 4931-4941
- *Cancer Research,* 2004, vol. 64, 7977-7984
- *Journal of Medicinal Chemistry,* 2003, vol. 46, 5375-5388
- *Cancer Research,* 2003, vol. 63, 5978-5991
- *Journal of Medicinal Chemistry,* 2005, vol. 48, 1359-1366
- *Anticancer Research,* 2004, vol. 24, 3009-3017
- *Proceedings of the American Association for Cancer Research,* 2005, vol. 46, 1407
- *EORTC-NCI-AACR Symp Mol Targets Cancer Ther,* 2004
- *Blood.,* 2004, vol. 103, 3474-3479
- *Proceedings of the American Association for Cancer Research,* 2003, vol. 44, 9
- *Proc. Am. Soc. Clin. Oncology,* 2004
- *Proceedings of the American Association for Cancer Research,* 2004, vol. 45, 694
- *Proceedings of the American Association for Cancer Research,* 2003, vol. 44, 1479
- *J. M. C.,* 1997, vol. 40, 2296-2303
- *EMBO J.,* 1998, vol. 17, 5896-5904
- *EMBO J.,* 1998, vol. 17, 5596-5904
- Antibodies: A Laboratory Manual. Cold Spring Harbor Laboratory, 1988
- *Biostatistics,* 2003, vol. 4, 249-264
- **PAULL, K. D. et al.** Display and analysis of patterns of differential activity of drugs against human tumor cell lines: development of mean graph and COMPARE algorithm. *J. Natl. Cancer Inst.,* 1989, vol. 81, 1088-1092
- **MONKS, A. et al.** Feasibility of a high-flux anti-cancer drug screen using a diverse panel of cultured human tumor cell lines. *J. Natl. Cancer Inst.,* 1991, vol. 83, 757-766
- *Molecular Cancer Therapeutics,* 2002, vol. 1, 275-286
- **MOSSMANN T.** *J. Immunol. Methods,* 1983, vol. 65, 55-63